# EUROPEAN PATENT SPECIFICATION

(11) **EP 4 472 650 B1**
(45) Date of publication and mention of the grant of the patent: **12.08.2026**
(21) Application number: 23702159.7
(22) Date of filing: 31.01.2023
(51) Int. Cl.: A61K 36/185, A61K 31/045, A61K 31/352, A61P 29/00

(54) **COMPOSITION COMPRISING DELTA-9-TETRAHYDROCANNABINOL AND TERPENES**
ZUSAMMENSETZUNG MIT DELTA-9-TETRAHYDROCANNABINOL UND TERPENEN
COMPOSITION COMPRENANT DU DELTA-9-TÉTRAHYDROCANNABINOL ET DES TERPÈNES

(30) Priority: 31.01.2022 EP 22154377; 31.01.2022 EP 22154378; 31.01.2022 EP 22154379; 31.01.2022 EP 22154380; 31.01.2022 EP 22154381; 31.01.2022 EP 22154382; 31.01.2022 EP 22154383; 31.01.2022 EP 22154384
(43) Date of publication of application: 11.12.2024
(73) Proprietor: Vertanical GmbH, 82166 Gräfelfing (DE)
(72) Inventor: BAASCH, Bastian, 86919 Utting am Ammersee (DE); FISCHER, Clemens, 80802 München (DE)
(74) Representative: Vossius & Partner Patentanwälte Rechtsanwälte mbB
(86) International application number: PCT/EP2023/052333
(87) International publication number: WO 2023/144420

(56) References cited:
- WO-A1-2020/006598
- WO-A1-2020/006599
- US-A1- 2018 352 848
- US-A1- 2021 204 591

## Description

The present disclosure relates to a pharmaceutical formulation comprising a composition comprising delta-9-tetrahydrocannabinol (THC), alpha-bisabolol, guaiol and beta-caryophyllene, and at least one terpene selected from the group consisting of linalool, alpha-humulene, nerolidol, caryophyllene oxide, alpha-pinene, camphene, beta-pinene, beta-myrcene, limonene, eucalyptol, ocimene, gamma-terpinene, terpinolene, alpha-terpinene, para-cymene, isopulegol and geraniol. The pharmaceutical formulation according to the present invention may be used in medicine, specifically in the treatment and/or prevention of chronic cancer pain, somatic pain, visceral pain, central neuropathic pain, peripheral neuropathic pain or complex pain syndromes. The invention is set out in the appended set of claims.

The main active substances rendering *Cannabis* relevant as an herbal medicine are delta-9-tetrahydrocannabinol (THC) and cannabidiol (CBD) which are present in the *Cannabis* plant predominantly as carboxylated 9-tetrahydrocannabinolic acid (THCA) and cannabidiolic acid (CBDA). Both belong to the substance class of cannabinoids, comprising amongst the two aforementioned cannabinoids of a variety of chemically and pharmaceutically related individual cannabinoid substances. Whereas THC is psychoactive and CBD is not, nearly all of the cannabinoids influence the central nervous system. Said activity of cannabinoids has led to a recent renaissance of *Cannabis* research, because controlled studies with either THC and/or CBD as single drug substance or using *Cannabis* plant extracts have shown positive results in the treatment of neuropathic pain, fibromyalgia, rheumatoid arthritis, and mixed chronic pain (Bridgeman M.B. and Abazia D.T., P&T 2017, 42(3), pp. 180-188). Moreover, plant-derived *Cannabis* medicinal extracts were found to be superior in relieving pain in patients with multiple sclerosis, spinal cord injury, brachial plexus damage and limp amputation due to neurofibromatosis. It was found that both THC and CBD alone as well as in a 1:1 ratio were effective (Wade, D.T. et al., Clin. Rehabil. 2003, 17(1), pp. 21-29). Similar results were obtained using a whole plant extract while interestingly, a benefit of the whole plant extract over THC alone was observed in multiple sclerosis patients for treating pain and spasms (Zajicek J et al., Lancet. 2003, 362(9395), pp. 1517-1526). In consequence, further research was dedicated to the complex interaction of the several cannabinoids and other substances in whole plant extracts of *Cannabis.* Within that study of Zajicek J et al., it was argued that the further constituents of the extract may synergistically contribute to such activity of cannabinoids.

Because the pain relief effect of *Cannabis* was found to be induced not only by its antinociceptive but also by an anti-inflammatory activity (Comelli F. et al., Phytotherapy Research 2008, 22(8), pp. 1017-1024), both the anti-inflammatory activity as well as the complex interplay between the main cannabinoids (such as THC and CBD) and other constituents of *Cannabis* extracts were further investigated. This resulted in the finding, that THC is deemed to be the main anti-inflammatory active substance at higher doses, while CBD has anti-inflammatory activity only at lower dosages, but cytotoxic activity at higher doses. Also in cell culture models using HCT-116 and CaCO2 cells it was found that *Cannabis* extracts are more active in reducing inflammation than the single cannabinoid alone. The *Cannabis* extracts were fractionated, but there was no dedicated finding on which individual compounds in the fractions actually contributed to the main activities attributed to either CBD as produced from CBDA (referred in this description to CBD(A)) and/or THC as produced from THCA (referred in this description to THC(A)) (Nallathambi R. et al., Cannabis and Cannabinoid Research 2017, 2(1), pp. 167-182).

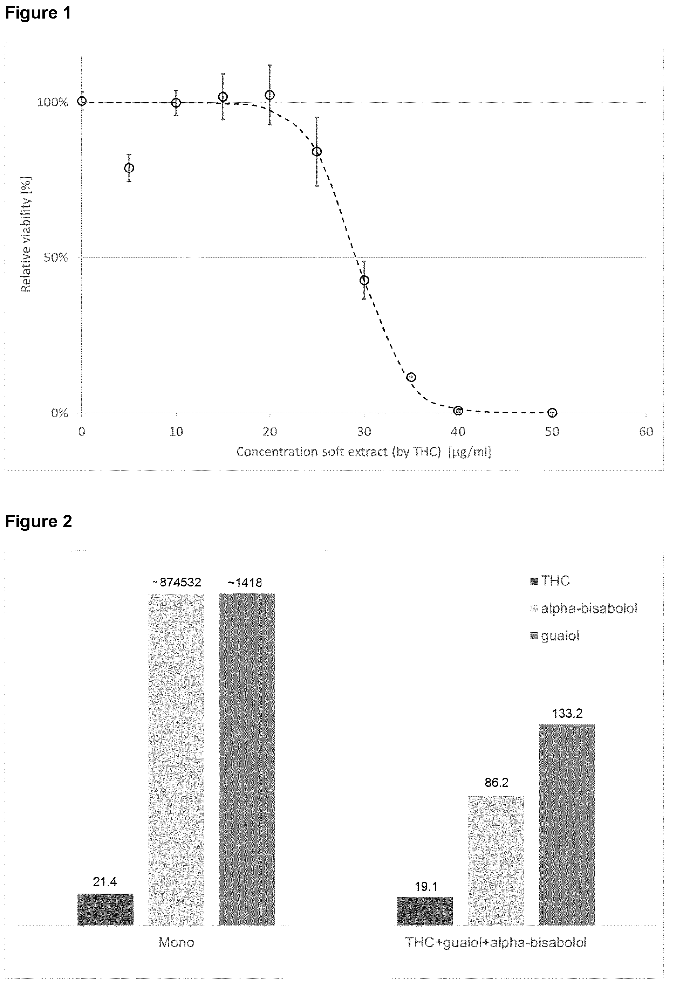

Today, there is no doubt that these cytotoxic and anti-inflammatory activities are substantially derived not only from the main constituents THC(A) and/or CBD(A). It was shown that *Cannabis* plants produce more than 600 different, in parts biologically active, secondary metabolites (Andre CM. et al., Front. Plant Sci. 2016, 7, 19) such as terpenes and terpenoids, sterols, triglycerides, alkanes, squalenes, tocopherols, and others. The mix of these secondary metabolites varies depending on several factors, including the specific *Cannabis* variety, the parts of the *Cannabis* plant to be extracted, the method of extraction and the processing of the extract. Specifically, there was a cytotoxic and anti-inflammatory contribution shown from terpenes, which are, however, deemed to be far less potent than CBD (Gallily R. et al., Cannabis and Cannabinoid Research 2018, 3(1), pp. 282-290). A study of Namdar et al. demonstrated a significant correlation between certain phytocannabinoids and sets of terpenes, such that a *Cannabis* plant that preferably comprises CBD(A) can be discriminated from a variety that comprises primarily THC(A) on the basis of the associated patterns of terpenes (Namdar D. et al., Molecules 2019, 24(3031), pp. 1-17). The study furthermore showed that THC activity is enhanced only by its co-related terpenes beta-thujene, alpha-pinene, camphene, beta-pinene, alpha-phellandrene, alpha-terpinene, beta-phellandrene, isosativene and alpha-guaiene, while other terpenes (e.g. associated with CBD(A)) inhibit its biological activity. CBD(A) was found to be associated with, amongst others, the terpenes alpha-bisabolol and guaiol (Figure 1). The authors of Namdar et al. conclude that terpenes act as promotors and instigators of therapeutic phytocannabinoid activity. Such enhanced biological activity attributable to secondary metabolites - mainly terpenes - produced by *Cannabis* plants is termed "inter-entourage effect" of phytocannabinoids. Therefore, certain cytotoxic and potentially anti-inflammatory or antinociceptive/antihyperalgesic effects mainly derived from CBD(A) and/or THC(A) may be each influenced by a specific set of terpenes associated with such main cannabinoid.

However, the remaining problem today is still the lack of knowledge about the interplay between the alleged main constituents CBD(A) and/or THC(A) and the complex pattern of terpenes making up the full mixture of an herbal medicine derived from *Cannabis.* Thus, there is an urgent need for better understanding the interplay between cannabinoids and individual terpenes as well as a pattern of terpenes and for determining a potentially superior ratio of those pharmaceutically precious substances.

The present invention meets this demand by providing a pharmaceutical formulation comprising delta-9-tetrahydrocannabinol (THC), alpha-bisabolol, guaiol and beta-caryophyllene, and at least one terpene selected from the group consisting of linalool, alpha-humulene, nerolidol, caryophyllene oxide, alpha-pinene, camphene, beta-pinene, beta-myrcene, limonene, eucalyptol, ocimene, gamma-terpinene, terpinolene, alpha-terpinene, para-cymene, isopulegol and geraniol. The combination of THC and the terpenes surprisingly and unexpectedly increase the cytotoxic effect of the terpenes. Notably, the activity of terpenes which are not correlated to THC is increased. As shown in the appended Examples, it was found in cancer cell culture models subjected to cytotoxicity assay that the IC50 value of the exemplary terpenes alpha-bisabolol and guaiol is decreased when the cells are co-cultured with THC in a specific THC/alpha-bisabolol and THC/guaiol ratio. Further, it was shown that that anti-inflammatory activity is enhanced when THC and the exemplary terpenes alpha-bisabolol and guaiol are administered in a specific THC/alpha-bisabolol and THC/guaiol ratio in a combinatory treatment when compared to a treatment with either THC, alpha-bisabolol or guaiol alone. Accordingly, the present invention is based on a surprising and unexpected effect since alpha-bisabolol and guaiol are known in the art to be associated with CBD as main cannabinoid rather than THC. Although the prior art describes various compositions comprising THC and terpenes (WO 2020/006599, WO 2020/006598, US 2021/204591, and US 2018/352848), none of the prior art documents describes the specific constitution and the advantageous THC/terpene ratio disclosed in the present invention. This is further, because terpenes are suggested in the art to execute an enhancing effect on cannabinoids by means of an "inter-entourage effect" rather than vice versa. The *Cannabis* plant extract of the present invention comprises THC and the terpenes alpha-bisabolol and guaiol in the ratio as tested individually in the Examples (see Table 3). Thus, it is believed that a pharmaceutical formulation of the invention which has the same individual effective components and THC/terpene ratio as the *Cannabis* plant extract disclosed in the present Examples (Table 3), are particularly effective in providing cytotoxic activity and anti-inflammatory activity. Further, the Examples provide evidence that the pharmaceutical formulation of the invention have a superior technical effect of providing improved short- and long-term stability of cannabinoids such as THC by comprising a carrier oil such as sesame oil (Example 3).

The present invention is as defined in the claims.

The present invention relates to a pharmaceutical formulation that comprises a composition described herein in the following, wherein the composition is a *Cannabis* plant extract. Specifically, the composition comprises delta-9-tetrahydrocannabinol (THC), alpha-bisabolol, guaiol and beta-caryophyllene, and at least one terpene selected from the group consisting of linalool, alpha-humulene, nerolidol, caryophyllene oxide, alpha-pinene, camphene, beta-pinene, beta-myrcene, limonene, eucalyptol, ocimene, gamma-terpinene, terpinolene, alpha-terpinene, para-cymene, isopulegol and geraniol. The composition may comprise any amount of THC, any amount of alpha-bisabolol, any amount of guaiol, any amount of beta-caryophyllene, and any amount of at least one terpene selected from the group consisting of linalool, alpha-humulene, nerolidol, caryophyllene oxide, alpha-pinene, camphene, beta-pinene, beta-myrcene, limonene, eucalyptol, ocimene, gamma-terpinene, terpinolene, alpha-terpinene, para-cymene, isopulegol and geraniol in any suitable combination of contents, provided that the components are present in the composition described herein. As such, the composition described herein is considered to comprise a mixture of THC, alpha-bisabolol, guaiol and beta-caryophyllene, and at least one terpene selected from the group consisting of linalool, alpha-humulene, nerolidol, caryophyllene oxide, alpha-pinene, camphene, beta-pinene, beta-myrcene, limonene, eucalyptol, ocimene, gamma-terpinene, terpinolene, alpha-terpinene, para-cymene, isopulegol and geraniol.

The composition as described herein may comprise any suitable ratio of THC, alpha-bisabolol, guaiol and beta-caryophyllene, and at least one terpene selected from the group consisting of linalool, alpha-humulene, nerolidol, caryophyllene oxide, alpha-pinene, camphene, beta-pinene, beta-myrcene, limonene, eucalyptol, ocimene, gamma-terpinene, terpinolene, alpha-terpinene, para-cymene, isopulegol and geraniol.

Suitable ratios present in the composition as described herein include THC/alpha-bisabolol ratios of between about 50:1, 60:1, 70:1, 80:1, 90:1, 100:1, 110:1, 120:1, 130:1, 140:1, 150:1, 160:1, 170:1, 180:1, 190:1, 200:1, 210:1, 220:1, 230:1, 240:1, 250:1, 260:1, 270:1, 280:1, 290:1, 300:1, 310:1, 320:1, 330:1, 340:1, 350:1, 360:1, 370:1, 380:1, 390:1, 400:1, 410:1, 420:1, 430:1, 440:1, 450:1, 460:1, 470:1, 480:1, 490:1 and 500:1; for example between 75:1, 80:1, 90:1, 100:1, 110:1, 120:1, 130:1, 140:1, 150:1, 160:1, 170:1, 180:1, 190:1, 200:1, 210:1, 220:1, 230:1, 240:1, 250:1, 260:1, 270:1, 280:1, 290:1, 300:1, 310:1, 320:1, 330:1, 340:1, 350:1, 360:1, 370:1, 380:1, 390:1, 400:1, 410:1, 420:1, 430:1, 440:1 and 450:1; or between 100:1, 110:1, 120:1, 130:1, 140:1, 150:1, 160:1, 170:1, 180:1, 190:1, 200:1, 210:1, 220:1, 230:1, 240:1, 250:1, 260:1, 270:1, 280:1, 290:1, 300:1, 310:1, 320:1, 330:1, 340:1, 350:1, 360:1, 370:1, 380:1, 390:1, 400:1, 410:1, 420:1, 430:1, 440:1, 450:1, 460:1, 470:1, 480:1, 490:1 and 500:1; or between 75:1, 80:1, 90:1, 100:1, 110:1, 120:1, 130:1, 140:1, 150:1, 160:1, 170:1, 180:1, 190:1, 200:1, 210:1, 220:1, 230:1, 240:1, 250:1, 260:1, 270:1, 280:1, 290:1, 300:1, 310:1, 320:1, 330:1, 340:1, 350:1, 360:1, 370:1, 380:1, 390:1 and 400:1; or between 150:1, 160:1, 170:1, 180:1, 190:1, 200:1, 210:1, 220:1, 230:1, 240:1, 250:1, 260:1, 270:1, 280:1, 290:1, 300:1, 310:1, 320:1, 330:1, 340:1, 350:1, 360:1, 370:1, 380:1, 390:1 and 400:1; or between 150:1, 160:1, 170:1, 180:1, 190:1, 200:1, 210:1, 220:1, 230:1, 240:1, 250:1, 260:1, 270:1, 280:1, 290:1, 300:1, 310:1, 320:1, 330:1, 340:1 and 350:1; or alternatively, between 160:1, 170:1 180:1, 190:1, 200:1, 210:1, 220:1, 230:1, 240:1, 250:1, 260:1, 270:1, 280:1, 290:1 and 295:1. In the context of the present disclosure, suitable ratios present in the composition as described herein include THC/alpha-bisabolol ratios of more than 50:1, 60:1, 70:1, 80:1, 90:1, 100:1, 110:1, 120:1, 130:1, 140:1, 150:1, 160:1, 170:1, 180:1, 185:1, 190:1, 200:1, 210:1, 220:1, 230:1, 240:1, 250:1, 260:1, 270:1, 280:1, 290:1, 300:1, 310:1, 320:1, 330:1, 340:1, 350:1, 360:1, 370:1, 380:1, 390:1, 400:1, 410:1, 420:1, 430:1, 440:1, 450:1, 460:1, 470:1, 480:1, 490:1, or 500:1. It is preferred that THC and alpha-bisabolol are present in the composition in a ratio of more than 160:1, in a ratio of between 100:1 and 450:1, even more preferably in a ratio of between 160:1 and 295:1.

Suitable ratios present in the composition as described herein include THC/guaiol ratios of between about 10:1, 20:1, 30:1, 40:1, 50:1, 60:1, 70:1, 80:1, 90:1, 100:1, 110:1, 120:1, 130:1, 140:1, 150:1, 160:1, 170:1, 180:1, 190:1, 200:1, 210:1, 220:1, 230:1, 240:1, 250:1, 260:1, 270:1, 280:1, 290:1, 300:1, 310:1, 320:1, 330:1, 340:1, 350:1, 360:1, 370:1, 380:1, 390:1, 400:1, 410:1, 420:1, 430:1, 440:1, 450:1, 460:1, 470:1, 480:1, 490:1 and 500:1; for example between 30:1, 40:1, 50:1, 60:1, 70:1, 80:1, 90:1, 100:1, 110:1, 120:1, 130:1, 140:1, 150:1, 160:1, 170:1, 180:1, 190:1, 200:1, 210:1, 220:1, 230:1, 240:1, 250:1, 260:1, 270:1, 280:1, 290:1, 300:1, 310:1, 320:1, 330:1, 340:1, 350:1, 360:1, 370:1, 380:1, 390:1, 400:1, 410:1, 420:1, 430:1, 440:1 and 450:1; or between 50:1, 60:1, 70:1, 80:1, 90:1, 100:1, 110:1, 120:1, 130:1, 140:1, 150:1, 160:1, 170:1, 180:1, 190:1, 200:1, 210:1, 220:1, 230:1, 240:1, 250:1, 260:1, 270:1, 280:1, 290:1, 300:1, 310:1, 320:1, 330:1, 340:1, 350:1, 360:1, 370:1, 380:1, 390:1 and 400:1; or between 50:1, 60:1, 70:1, 80:1, 90:1, 100:1, 110:1, 120:1, 130:1, 140:1, 150:1, 160:1, 170:1, 180:1, 190:1, 200:1, 210:1, 220:1, 230:1, 240:1 and 250:1; or, alternatively, between 105:1, 110:1, 115:1, 120:1, 125:1, 130:1, 135:1, 140:1, 145:1, 150:1, 155:1, 160:1, 165:1, 170:1, 175:1, 180:1, 185:1, 190:1, 195:1 and 200:1. Suitable ratios present in the composition as described herein include THC/guaiol ratios of more than 10:1, 20:1, 30:1, 40:1, 50:1, 60:1, 70:1, 80:1, 90:1, 100:1, 105:1, 110:1, 120:1, 130:1, 140:1, 150:1, 160:1, 170:1, 180:1, 190:1, 200:1, 210:1, 220:1, 230:1, 240:1, 250:1, 260:1, 270:1, 280:1, 290:1, 300:1, 310:1, 320:1, 330:1, 340:1, 350:1, 360:1, 370:1, 380:1, 390:1, 400:1, 410:1, 420:1, 430:1, 440:1, 450:1, 460:1, 470:1, 480:1, 490:1, or 500:1. It is preferred that THC and guaiol are present in the composition described herein in a ratio of more than 105:1, in a ratio of between 50:1 and 250:1, even more preferably in a ratio of between 105:1 and 200:1.

Suitable ratios present in the composition as described herein include THC/beta-caryophyllene ratios of between about 10:1, 20:1, 30:1, 40:1, 50:1, 60:1, 70:1, 80:1, 90:1, 100:1, 110:1, 120:1, 130:1, 140:1, 150:1, 160:1, 170:1, 180:1, 190:1, 200:1, 210:1, 220:1, 230:1, 240:1, 250:1, 260:1, 270:1, 280:1, 290:1, 300:1, 310:1, 320:1, 330:1, 340:1, 350:1, 360:1, 370:1, 380:1, 390:1, 400:1, 410:1, 420:1, 430:1, 440:1, 450:1, 460:1, 470:1, 480:1, 490:1 and 500:1; for example between 30:1, 40:1, 50:1, 60:1, 70:1, 80:1, 90:1, 100:1, 110:1, 120:1, 130:1, 140:1, 150:1, 160:1, 170:1, 180:1, 190:1, 200:1, 210:1, 220:1, 230:1, 240:1, 250:1, 260:1, 270:1, 280:1, 290:1, 300:1, 310:1, 320:1, 330:1, 340:1, 350:1, 360:1, 370:1, 380:1, 390:1, 400:1, 410:1, 420:1, 430:1, 440:1 and 450:1; or between 50:1, 60:1, 70:1, 80:1, 90:1, 100:1, 110:1, 120:1, 130:1, 140:1, 150:1, 160:1, 170:1, 180:1, 190:1, 200:1, 210:1, 220:1, 230:1, 240:1, 250:1, 260:1, 270:1, 280:1, 290:1, 300:1, 310:1, 320:1, 330:1, 340:1, 350:1, 360:1, 370:1, 380:1, 390:1 and 400:1; or between 50:1, 60:1, 70:1, 80:1, 90:1, 100:1, 110:1, 120:1, 130:1, 140:1, 150:1, 160:1, 170:1, 180:1, 190:1, 200:1, 210:1, 220:1, 230:1, 240:1 and 250:1; or alternatively between 105:1, 110:1, 115:1, 120:1, 125:1, 130:1, 135:1, 140:1, 145:1, 150:1, 155:1, 160:1, 165:1, 170:1, 175:1, 180:1, 185:1, 190:1, 195:1 and 200:1. In the context of the present disclosure, suitable ratios present in the composition as described herein include THC/beta-caryophyllene ratios of more than 10:1, 20:1, 30:1, 40:1, 50:1, 60:1, 70:1, 80:1, 90:1, 100:1, 105:1, 110:1, 120:1, 130:1, 140:1, 150:1, 160:1, 170:1, 180:1, 190:1, 200:1, 210:1, 220:1, 230:1, 240:1, 250:1, 260:1, 270:1, 280:1, 290:1, 300:1, 310:1, 320:1, 330:1, 340:1, 350:1, 360:1, 370:1, 380:1, 390:1, 400:1, 410:1, 420:1, 430:1, 440:1, 450:1, 460:1, 470:1, 480:1, 490:1 or 500:1. It is preferred that THC and beta-caryophyllene are present in the composition described herein in a ratio of more than 105:1, in a ratio of between 50:1 and 250:1, even more preferably in a ratio of between 105:1 and 200:1.

Suitable ratios present in the composition as described herein include a THC/linalool ratio of between 450:1 and 1400:1, a THC/alpha-humulene ratio of between 300:1 and 725:1, a THC/nerolidol ratio of between 200:1 and 600:1, a THC/caryophyllene oxide ratio of between 1000:1 and 2150:1, a THC/alpha-pinene ratio of between 5850:1 and 16000:1, a THC/camphene ratio of between 7300:1 and 132950:1, a THC/beta-pinene ratio of between 6475:1 and 23100:1, a THC/beta-myrcene ratio of between 1950:1 and 6300:1, a THC/limonene ratio of between 1650:1 and 5050:1, a THC/eucalyptol ratio of between 5050:1 and 38650:1, a THC/ocimene ratio of between 2500:1 and 44700:1, a THC/gamma-terpinene ratio of between 4700:1 and 35750:1, a THC/terpinolene ratio of between 5450:1 and 129850:1, a THC/alpha-terpinene ratio of between 5150:1 and 58550:1, a THC/para-cymene ratio of between 4700:1 and 56700:1, a THC/isopulegol ratio of between 4000:1 and 8000:1, and a THC/geraniol ratio of between 1300:1 and 18250:1. Preferred ratios present in the composition as described herein include a THC/linalool ratio of between 500:1 and 1330:1, a THC/alpha-humulene ratio of between 355:1 and 665:1, a THC/nerolidol ratio of between 285:1 and 535:1, a THC/caryophyllene oxide ratio of between 1090:1 and 2080:1, a THC/alpha-pinene ratio of between 6000:1 and 15750:1, a THC/camphene ratio of between 7300:1 and 132950:1, a THC/beta-pinene ratio of between 6545:1 and 23025:1, a THC/beta-myrcene ratio of between 2025:1 and 6210:1, a THC/limonene ratio of between 1700:1 and 4970:1, a THC/eucalyptol ratio of between 5140:1 and 38580:1, a THC/ocimene ratio of between 2570:1 and 44610:1, a THC/gamma-terpinene ratio of between 4800:1 and 35685:1, a THC/terpinolene ratio of 5535:1 and 129965:1, a THC/alpha-terpinene ratio of between 5210:1 and 58480:1, a THC/para-cymene ratio of between 4800:1 and 56625:1, a THC/isopulegol ratio of between 4060:1 and 7915:1 and a THC/geraniol ratio of between 1375:1 and 18150:1.

Accordingly, it is preferred that THC and alpha-bisabolol are present in the composition in a ratio of between 100:1 and 450:1, THC and guaiol are present in the composition described herein in a ratio of between 50:1 and 250:1, THC and beta-caryophyllene are present in the composition in a ratio of between 50:1 and 250:1, and the composition further comprises at least one terpene selected from the group consisting of linalool in a THC/linalool ratio of between 450:1 and 1400:1, alpha-humulene in a THC/alpha-humulene ratio of between 300:1 and 725:1, nerolidol in a THC/nerolidol ratio of between 200:1 and 600:1, caryophyllene oxide in a THC/caryophyllene oxide ratio of between 1000:1 and 2150:1, alpha-pinene in a THC/alpha-pinene ratio of between 5850:1 and 16000:1, camphene in a THC/camphene ratio of between 7300:1 and 132950:1, beta-pinene in a THC/beta-pinene ratio of between 6475:1 and 23100:1, beta-myrcene in a THC/beta-myrcene ratio of between 1950:1 and 6300:1, limonene in a THC/limonene ratio of between 1650:1 and 5050:1, eucalyptol in a THC/eucalyptol ratio of between 5050:1 and 38650:1, ocimene in a THC/ocimene ratio of between 2500:1 and 44700:1, gamma-terpinene a THC/gamma-terpinene ratio of between 4700:1 and 35750:1, terpinolene in a THC/terpinolene ratio of between 5450:1 and 129850:1, alpha-terpinene in a THC/alpha-terpinene ratio of between 5150:1 and 58550:1, para-cymene in a THC/para-cymene ratio of between 4700:1 and 56700:1, isopulegol in a THC/isopulegol ratio of between 4000:1 and 8000:1 and geraniol in a THC/geraniol ratio of between 1300:1 and 18250:1. In the present disclosure, it is even more preferred that THC and alpha-bisabolol are present in the composition in a ratio of between 160:1 and 295:1, THC and guaiol are present in the composition in a ratio of between 105:1 and 200:1, and THC and beta-caryophyllene are present in the composition in a ratio of between 105:1 and 200:1, and the composition further comprises at least one terpene selected from the group consisting of linalool in a THC/linalool ratio of between 500:1 and 1330:1, alpha-humulene in a THC/alpha-humulene ratio of between 355:1 and 665:1, nerolidol in a THC/nerolidol ratio of between 285:1 and 535:1, caryophyllene oxide in a THC/caryophyllene oxide ratio of between 1090:1 and 2080:1, alpha-pinene in a THC/alpha-pinene ratio of between 6000:1 and 15750:1, camphene in a THC/camphene ratio of between 7300:1 and 132950:1, beta-pinene in a THC/beta-pinene ratio of between 6545:1 and 23025:1, beta-myrcene in a THC/beta-myrcene ratio of between 2025:1 and 6210:1, limonene in a THC/limonene ratio of between 1700:1 and 4970:1, eucalyptol in a THC/eucalyptol ratio of between 5140:1 and 38580:1, ocimene in a THC/ocimene ratio of between 2570:1 and 44610:1, gamma-terpinene in a THC/gamma-terpinene ratio of between 4800:1 and 35685:1, terpinolene in a THC/terpinolene ratio of 5535:1 and 129965:1, alpha-terpinene in a THC/alpha-terpinene ratio of between 5210:1 and 58480:1, para-cymene in a THC/para-cymene ratio of between 4800:1 and 56625:1, isopulegol in a THC/isopulegol ratio of between 4060:1 and 7915:1, and geraniol in a THC/geraniol ratio of between 1375:1 and 18150:1.

The term "at least one terpene selected from the group consisting of" means that at least 1, 2, 3, 4, 5, 6, 7, 8, 9, 10, 11, 12, 13, 14, 15, 16, or 17 additional terpene(s) selected from the group consisting of linalool, alpha-humulene, nerolidol, caryophyllene oxide, alpha-pinene, camphene, beta-pinene, beta-myrcene, limonene, eucalyptol, ocimene, gamma-terpinene, terpinolene, alpha-terpinene, para-cymene, isopulegol and geraniol is(are) present in the composition described herein, i.e. the composition which comprises THC, alpha-bisabolol, guaiol and beta-caryophyllene. Exemplarily, in the context of the present disclosure, the composition comprises preferably at least 1, 2, 3, 4, or 5 terpene(s) selected from the group consisting of linalool, alpha-humulene, nerolidol, caryophyllene oxide and limonene.

The composition may comprise at least about 2.5%, 3%, 4%, 5%, 6%, 7%, 8%, 9%, 10%, 15% or 20% THC by weight of the composition; for example between about 2.5%, 3%, 4%, 5%, 6%, 7%, 8%, 9% and 10% THC by weight of the composition; or between 2.5%, 3%, 4%, 5%, 6%, 7% and 8%; or between 3%, 4%, 5%, 6% and 7%; or between 3%, 4%, 5% and 6%; or between 4%, 5% and 6% THC by weight of the composition. The skilled person is aware that the above values given in % by weight may also be provided in parts per million (ppm), e.g. mg/kg or mg/l. It is preferred that the composition comprises THC in an amount of between about 2.5% and 10%, of at least about 4%, or preferably of between about 4% and 6% by weight of the composition. The composition comprises THC most preferably in an amount of 5% by weight of the composition. As a matter of example, an amount of 5% THC by weight of the composition would convert to 50,000 ppm.

The composition may also comprise at least about 0.001%, 0.002%, 0.003%, 0.004%, 0.005%, 0.006%, 0.007%, 0.008%, 0.009%, 0.01%, 0.011%, 0.012%, 0.013%, 0.014%, 0.015%, 0.016%, 0.017%, 0.018%, 0.019%, 0.02%, 0.021%, 0.022%, 0.023%, 0.024%, 0.025%, 0.026%, 0.027%, 0.028%, 0.029%, 0.030%, 0.031%, 0.032%, 0.033%, 0.034%, 0.035%, 0.036%, 0.037%, 0.038%, 0.039% or 0.04% alpha-bisabolol by weight of the composition; for example between about 0.005%, 0.008%, 0.01%, 0.015%, 0.02%, 0.025%, 0.03%, 0.035%, 0.04%, 0.045%, 0.05%, 0.055%, 0.06% and 0.065%; or between 0.001%, 0.002%, 0.003%, 0.004%, 0.005%, 0.006%, 0.007%, 0.008%, 0.009%, 0.01%, 0.011%, 0.012%, 0.013%, 0.014%, 0.015%, 0.016%, 0.017%, 0.018%, 0.019%, 0.02%, 0.021%, 0.022%, 0.023%, 0.024%, 0.025%, 0.026%, 0.027%, 0.028%, 0.029%, 0.030%, 0.031%, 0.032%, 0.033%, 0.034%, 0.035%, 0.036%, 0.037%, 0.038%, 0.039% and 0.04%; or, alternatively, between 0.005%, 0.006%, 0.007%, 0.008%, 0.009%, 0.01%, 0.011%, 0.012%, 0.013%, 0.014%, 0.015%, 0.016%, 0.017%, 0.018%, 0.019%, 0.02%, 0.021%, 0.022%, 0.023%, 0.024%, 0.025%, 0.026%, 0.027%, 0.028%, 0.029% and 0.03%; or between 0.011%, 0.012%, 0.013%, 0.014%, 0.015%, 0.016%, 0.017%, 0.018%, 0.019%, 0.02%, 0.021%, 0.022%, 0.023%, 0.024%, 0.025%, 0.026%, 0.027%, 0.028%, 0.029% and 0.03%; or alternatively between 0.014%, 0.015%, 0.016%, 0.017%, 0.018%, 0.019%, 0.02%, 0.021%, 0.022%, 0.023%, 0.024%, 0.025%, 0.026% and 0.027% alpha-bisabolol by weight of the composition. The composition may comprise alpha-bisabolol in an amount of between 0.008% and 0.065%, preferably between 0.01% and 0.04% by weight of the composition. Even more preferably, the composition comprises alpha-bisabolol in an amount of at least 0.017% by weight of the composition, or most preferably between 0.017% and 0.031% by weight of the composition.

As such, the composition may comprise THC in an amount of between about 2.5% and 10% and alpha-bisabolol in an amount of between about 0.008% and 0.065% by weight of the composition. More preferably, the composition may comprise THC in an amount of between about 4% and 6% by weight of the composition and alpha-bisabolol in an amount of between about 0.01% and 0.04% by weight of the composition. Accordingly, the composition described herein may comprise THC in an amount of between about 2.5% and 10%, of at least 4%, preferably between 4% and 6% by weight of the composition and alpha-bisabolol in an amount of at least 0.008% by weight of the composition, between about 0.008% and 0.065% by weight of the composition, preferably between about 0.01% and 0.04% by weight of the composition.

Most preferably, the composition as described herein comprises THC in an amount of 5% by weight of the composition and alpha-bisabolol in an amount of between about 0.017% and 0.031% by weight of the composition. In the context of the present disclosure, the composition as described herein may comprise THC in an amount of 5% by weight of the composition and alpha-bisabolol in an amount of at least about 0.017% by weight of the composition.

**In** the present disclosure, the composition described herein may comprise THC in an amount of between about 2.5% and 10% THC by weight of the composition and a THC/alpha-bisabolol ratio of between 100:1 and 450:1. Moreover, the composition of the disclosure may comprise alpha-bisabolol in an amount of between about 0.008% and 0.065% by weight of the composition and a THC/alpha-bisabolol ratio of between 100:1 and 450:1.

More preferably, the composition described herein may comprise THC in an amount of between about 4% and 6% THC by weight of the composition and a THC/alpha-bisabolol ratio of between 100:1 and 450:1. Moreover, the composition described herein may comprise alpha-bisabolol in an amount of between about 0.01% and 0.04% by weight of the composition and a THC/alpha-bisabolol ratio of between 100:1 and 450:1.

Most preferably, the composition described herein may comprise **THC** in an amount of 5% **THC** by weight of the composition and a THC/alpha-bisabolol ratio of between 160:1 and 295:1. Further, in the context of the present disclosure, the composition described herein may comprise alpha-bisabolol in an amount of between about 0.014% and 0.031 % by weight of the composition and a THC/alpha-bisabolol ratio of between 160:1 and 295:1.

**In** the present disclosure, the composition described herein may also comprise **THC** in an amount of 5% by weight of the composition and a THC/alpha-bisabolol ratio of at least about 160:1. In the present disclosure, the composition described herein may also comprise alpha-bisabolol in an amount of at least 0.01% by weight of the composition, preferably between about 0.01% and 0.04% by weight of the composition and an alpha-bisabolol/THC ratio of more than 1:100.

In the context of the present disclosure, the composition may also comprise at least about 0.001%, 0.002%, 0.003%, 0.004%, 0.005%, 0.006%, 0.007%, 0.008%, 0.009%, 0.01%, 0.011%, 0.012%, 0.013%, 0.014%, 0.015%, 0.016%, 0.017%, 0.018%, 0.019%, 0.02%, 0.021%, 0.022%, 0.023%, 0.024%, 0.025%, 0.026%, 0.027%, 0.028%, 0.029%, 0.030%, 0.031%, 0.032%, 0.033%, 0.034%, 0.035%, 0.036%, 0.037%, 0.038%, 0.039% or 0.04% guaiol by weight of the composition; for example between about 0.012%, 0.015%, 0.02%, 0.025%, 0.03%, 0.035%, 0.04%, 0.045%, 0.05%, 0.055%, 0.06%, 0.065%, 0.07%, 0.075%, 0.08%, 0.085%, 0.09% and 0.092%; or between 0.001%, 0.002%, 0.0025%, 0.003%, 0.0035%, 0.004%, 0.0045%, 0.005%, 0.0055%, 0.006%, 0.0065%, 0.007%, 0.0075%, 0.008%, 0.0085%, 0.009%, 0.0095%, 0.01%, 0.015%, 0.02%, 0.025%, 0.03%, 0.035%, 0.04%, 0.045%, 0.05%, 0.055%, 0.06%, 0.065%, 0.07%, 0.075% and 0.08%; or between 0.015%, 0.016%, 0.017%, 0.018%, 0.019%, 0.020%, 0.021%, 0.022%, 0.023%, 0.024%, 0.025%, 0.026%, 0.027%, 0.028%, 0.029%, 0.030%, 0.031%, 0.032%, 0.033%, 0.034%, 0.035%, 0.036%, 0.037%, 0.038%, 0.039%, 0.04%, 0.041%, 0.042%, 0.043%, 0.044%, 0.045%, 0.046%, 0.047%, 0.048%, 0.049% and 0.05%; or, alternatively, between 0.025%, 0.026%, 0.027%, 0.028%, 0.029%, 0.030%, 0.031%, 0.032%, 0.033%, 0.034%, 0.035%, 0.036%, 0.037%, 0.038%, 0.039%, 0.040%, 0.041%, 0.042%, 0.043%, 0.044%, 0.045% and 0.046% guaiol by weight of the composition. The composition described herein may comprise guaiol in an amount of between 0.012% and 0.092%, preferably between 0.015% and 0.05% by weight of the composition. Even more preferably, the composition described herein comprises guaiol in an amount of at least 0.025% by weight of the composition, or most preferably between 0.025% and 0.046% by weight of the composition.

As such, the composition described herein may comprise THC in an amount of between about 2.5% and 10% and guaiol in an amount of between about 0.012% and 0.092% by weight of the composition. More preferably, the composition may comprise THC in an amount of between about 4% and 6% by weight of the composition and guaiol in an amount of between about 0.015% and 0.05% by weight of the composition. Accordingly, the composition may comprise THC in an amount of between about 2.5% and 10%, of at least 4%, preferably between 4% and 6% by weight of the composition and guaiol in an amount of at least 0.012% by weight of the composition, between about 0.012% and 0.092% by weight of the composition, preferably between about 0.015% and 0.05% by weight of the composition. Most preferably, the composition as described herein comprises THC in an amount of 5% by weight of the composition and guaiol in an amount of between about 0.025% and 0.046% by weight of the composition. In the context of the present disclosure, the composition as described herein may comprise THC in an amount of 5% by weight of the composition and guaiol in an amount of at least about 0.025% by weight of the composition.

In the present disclosure, the composition described herein may comprise THC in an amount of between about 2.5% and 10% THC by weight of the composition and a THC/guaiol ratio of between 50:1 and 250:1. Moreover, in the context of the present disclosure, the composition described herein may comprise guaiol in an amount of between about 0.012% and 0.092% by weight of the composition and a THC/guaiol ratio of between 50:1 and 250:1. More preferably, in the present disclosure, the composition described herein may comprise THC in an amount of between about 4% and 6% THC by weight of the composition and a THC/guaiol ratio of between 50:1 and 250:1. Moreover, in the context of the present disclosure, the composition described herein may comprise guaiol in an amount of between about 0.015% and 0.05% by weight of the composition and a THC/guaiol ratio of between 50:1 and 250:1.

Most preferably, in the present disclosure, the composition described herein may comprise THC in an amount of 5% THC by weight of the composition and a THC/guaiol ratio of between 105:1 and 200:1. Further, in the context of the present disclosure, the composition may comprise guaiol in an amount of between about 0.025% and 0.046% by weight of the composition and a THC/guaiol ratio of between 105:1 and 200:1.

In the present disclosure, the composition may also comprise THC in an amount of 5% by weight of the composition and a THC/guaiol ratio of at least about 105:1. In the present disclosure, the composition described herein may also comprise guaiol in an amount of at least 0.015% by weight of the composition, preferably between about 0.015% and 0.05% by weight of the composition and an guaiol/THC ratio of more than 1:50.

In the context of the present disclosure, the composition may also comprise at least about 0.001%, 0.002%, 0.003%, 0.004%, 0.005%, 0.006%, 0.007%, 0.008%, 0.009%, 0.01%, 0.011%, 0.012%, 0.013%, 0.014%, 0.015%, 0.016%, 0.017%, 0.018%, 0.019%, 0.02%, 0.021%, 0.022%, 0.023%, 0.024%, 0.025%, 0.026%, 0.027%, 0.028%, 0.029%, 0.030%, 0.031%, 0.032%, 0.033%, 0.034%, 0.035%, 0.036%, 0.037%, 0.038%, 0.039% or 0.04% beta-caryophyllene by weight of the composition; for example between about 0.012%, 0.015%, 0.02%, 0.025%, 0.03%, 0.035%, 0.04%, 0.045%, 0.05%, 0.055%, 0.06%, 0.065%, 0.07%, 0.075%, 0.08%, 0.085%, 0.09% and 0.094%; or between 0.001%, 0.002%, 0.0025%, 0.003%, 0.0035%, 0.004%, 0.0045%, 0.005%, 0.0055%, 0.006%, 0.0065%, 0.007%, 0.0075%, 0.008%, 0.0085%, 0.009%, 0.0095%, 0.01%, 0.015%, 0.02%, 0.025%, 0.03%, 0.035%, 0.04%, 0.045%, 0.05%, 0.055%, 0.06%, 0.065%, 0.07%, 0.075% and 0.08%; or between 0.015%, 0.016%, 0.017%, 0.018%, 0.019%, 0.020%, 0.021%, 0.022%, 0.023%, 0.024%, 0.025%, 0.026%, 0.027%, 0.028%, 0.029%, 0.030%, 0.031%, 0.032%, 0.033%, 0.034%, 0.035%, 0.036%, 0.037%, 0.038%, 0.039%, 0.04%, 0.041%, 0.042%, 0.043%, 0.044%, 0.045%, 0.046%, 0.047%, 0.048%, 0.049% and 0.05%; or between 0.025%, 0.026%, 0.027%, 0.028%, 0.029%, 0.030%, 0.031%, 0.032%, 0.033%, 0.034%, 0.035%, 0.036%, 0.037%, 0.038%, 0.039%, 0.040%, 0.041%, 0.042%, 0.043%, 0.044%, 0.045%, 0.046% and 0.047% beta-caryophyllene by weight of the composition. The composition described herein may comprise beta-caryophyllene in an amount of between 0.0012% and 0.094%, preferably between 0.02% and 0.05% by weight of the composition. Even more preferably, the composition described herein comprises beta-caryophyllene in an amount of at least 0.025% by weight of the composition, or most preferably between 0.025% and 0.047% by weight of the composition.

As such, the composition described herein may comprise THC in an amount of between about 2.5% and 10% and beta-caryophyllene in an amount of between about 0.012% and 0.094% by weight of the composition. More preferably, the composition described herein may comprise THC in an amount of between about 4% and 6% by weight of the composition and beta-caryophyllene in an amount of between about 0.02% and 0.05% by weight of the composition. Accordingly, the composition described herein may comprise THC in an amount of between about 2.5% and 10%, of at least 4%, preferably between 4% and 6% by weight of the composition and beta-caryophyllene in an amount of at least 0.012% by weight of the composition, between about 0.012% and 0.094% by weight of the composition, preferably between about 0.02% and 0.05% by weight of the composition.

Most preferably, the composition as described herein comprises THC in an amount of 5% by weight of the composition and beta-caryophyllene in an amount of between about 0.025% and 0.047% by weight of the composition. In the context of the present disclosure, the composition as described herein may comprise THC in an amount of 5% by weight of the composition and beta-caryophyllene in an amount of at least about 0.025% by weight of the composition.

In the present disclosure, the composition described herein may comprise THC in an amount of between about 2.5% and 10% THC by weight of the composition and a THC/beta-caryophyllene ratio of between 50:1 and 250:1. Moreover, in the context of the present disclosure, the composition described herein may comprise beta-caryophyllene in an amount of between about 0.012% and 0.094% by weight of the composition and a THC/beta-caryophyllene ratio of between 50:1 and 250:1.

More preferably, the composition described herein may comprise THC in an amount of between about 4% and 6% THC by weight of the composition and a THC/beta-caryophyllene ratio of between 50:1 and 250:1. Moreover, in the context of the present disclosure, the composition described herein may comprise beta-caryophyllene in an amount of between about 0.02% and 0.05% by weight of the composition and a THC/beta-caryophyllene ratio of between 50:1 and 250:1.

Most preferably, in the present disclosure, the composition may comprise THC in an amount of 5% THC by weight of the composition and a THC/beta-caryophyllene ratio of between 105:1 and 200:1. Further, in the context of the present disclosure, the composition described herein may comprise beta-caryophyllene in an amount of between about 0.025% and 0.047% by weight of the composition and a THC/beta-caryophyllene ratio of between 105:1 and 200:1.

In the present disclosure, the composition described herein may also comprise THC in an amount of 5% by weight of the composition and a THC/beta-caryophyllene ratio of at least about 105:1. In the present disclosure, the composition may also comprise beta-caryophyllene in an amount of at least 0.025% by weight of the composition, preferably between about 0.02% and 0.05% by weight of the composition and an beta-caryophyllene/THC ratio of more than 1:50.

The composition further comprises at least one terpene selected from the group consisting of linalool, alpha-humulene, nerolidol, caryophyllene oxide, alpha-pinene, camphene, beta-pinene, beta-myrcene, limonene, eucalyptol, ocimene, gamma-terpinene, terpinolene, alpha-terpinene, para-cymene, isopulegol and geraniol. In the present disclosure, the composition may comprise linalool in an amount of between 0.001% and 0.014% by weight of the composition, alpha-humulene in an amount of between 0.003% and 0.028% by weight of the composition, nerolidol in an amount of between 0.004% and 0.035% by weight of the composition, caryophyllene oxide in an amount of between 0.001% and 0.0092% by weight of the composition, alpha-pinene in an amount of between 0.0001% and 0.002% by weight of the composition, camphene in an amount of between 0.00001% and 0.0015% by weight of the composition, beta-pinene in an amount of between 0.0001% and 0.0015% by weight of the composition, beta-myrcene in an amount of between 0.0004% and 0.005% by weight of the composition, limonene in an amount of between 0.0005% and 0.004% by weight of the composition, eucalyptol in an amount of between 0.00006% and 0.002% by weight of the composition, ocimene in an amount of between 0.00005% and 0.004% by weight of the composition, gamma-terpinene in an amount of between 0.00007% and 0.002% by weight of the composition, terpinolene in an amount of between 0.00002% and 0.002% by weight of the composition, alpha-terpinene in an amount of between 0.00004% and 0.002% by weight of the composition, para-cymene in an amount of between 0.00004% and 0.002%, isopulegol in an amount of between 0.0003% and 0.003% by weight of the composition, and/or geraniol in an amount of between 0.0001% and 0.008% by weight of the composition.

Preferably, the composition may comprise linalool in an amount of between 0.003% and 0.0085% by weight of the composition, alpha-humulene in an amount of between 0.006% and 0.017% by weight of the composition, nerolidol in an amount of between 0.007% and 0.021 % by weight of the composition, caryophyllene oxide in an amount of between 0.001% and 0.0055% by weight of the composition, alpha-pinene in an amount of between 0.0002% and 0.001% by weight of the composition, camphene in an amount of between 0.00003% and 0.00082% by weight of the composition, beta-pinene in an amount of between 0.0001% and 0.00092% by weight of the composition, beta-myrcene in an amount of between 0.0004% and 0.005% by weight of the composition, limonene in an amount of between 0.0008% and 0.0022% by weight of the composition, eucalyptol in an amount of between 0.0001% and 0.0012% by weight of the composition, ocimene in an amount of between 0.00009% and 0.0023% by weight of the composition, gamma-terpinene in an amount of between 0.0001% and 0.0013% by weight of the composition, terpinolene in an amount of between 0.00003% and 0.001% by weight of the composition, alpha-terpinene in an amount of between 0.00006% and 0.0012% by weight of the composition, para-cymene in an amount of between 0.00007% and 0.0013% by weight of the composition, isopulegol in an amount of between 0.0005% and 0.0015% by weight of the composition, and/or geraniol in an amount of between 0.0002% and 0.0044% by weight of the composition.

In the present disclosure, the composition described herein may comprise THC in an amount of between about 2.5% and 10% THC by weight of the composition, a THC/alpha-bisabolol ratio of between 100:1 and 450:1, a THC/guaiol ratio of between 50:1 and 250:1, a THC/beta-caryophyllene ratio of between 50:1 and 250:1 and further comprises at least one terpene selected from the group consisting of linalool in a THC/linalool ratio of between 650:1 and 1400:1, alpha-humulene in a THC/alpha-humulene ratio of between 300:1 and 725:1, nerolidol in a THC/nerolidol ratio of between 200:1 and 600:1, caryophyllene oxide in a THC/caryophyllene oxide ratio of between 1000:1 and 2150:1, alpha-pinene in a THC/alpha-pinene ratio of between 5850:1 and 16000:1, camphene in a THC/camphene ratio of between 7300:1 and 132950:1, beta-pinene in a THC/beta-pinene ratio of between 6475:1 and 23100:1, beta-myrcene in a THC/beta-myrcene ratio of between 1950:1 and 6300:1, limonene in a THC/limonene ratio of between 1650:1 and 5050:1, eucalyptol in a THC/eucalyptol ratio of between 5050:1 and 38650:1, ocimene in a THC/ocimene ratio of between 2500:1 and 44700:1, gamma-terpinene a THC/gamma-terpinene ratio of between 4700:1 and 35750:1, terpinolene in a THC/terpinolene ratio of between 5450:1 and 129850:1, alpha-terpinene in a THC/alpha-terpinene ratio of between 5150:1 and 58550:1, para-cymene in a THC/para-cymene ratio of between 4700:1 and 56700:1, isopulegol in a THC/isopulegol ratio of between 4000:1 and 8000:1, and geraniol in a THC/geraniol ratio of between 1300:1 and 18250:1.

Moreover, in the context of the present disclosure, the composition described herein may comprise alpha-bisabolol in an amount of between about 0.008% and 0.065% by weight of the composition, guaiol in an amount of between about 0.012% and 0.092% by weight of the composition, beta-caryophyllene in an amount of between about 0.012% and 0.094% by weight of the composition, and at least one terpene selected from the group consisting of linalool in an amount of between 0.001% and 0.014%, alpha-humulene in an amount of between 0.003% and 0.028%, nerolidol in an amount of between 0.004% and 0.035%, caryophyllene oxide in an amount of between 0.001% and 0.0092%, alpha-pinene in an amount of between 0.0001% and 0.002%, camphene in an amount of between 0.00001% and 0.0015%, beta-pinene in an amount of between 0.0001% and 0.0015%, beta-myrcene in an amount of between 0.0004% and 0.005%, limonene in an amount of between 0.0005% and 0.004%, eucalyptol in an amount of between 0.00006% and 0.002%, ocimene in an amount of between 0.00005% and 0.004%, gamma-terpinene in an amount of between 0.00007% and 0.002%, terpinolene in an amount of between 0.00002% and 0.002%, alpha-terpinene in an amount of between 0.00004% and 0.002%, para-cymene in an amount of between 0.00004% and 0.002%, isopulegol in an amount of between 0.0003% and 0.003%, and geraniol in an amount of between 0.0001% and 0.008% by weight of the composition, a THC/alpha-bisabolol ratio of between 100:1 and 450:1, and further comprises a THC/guaiol ratio of between 50:1 and 250:1, and a THC/beta-caryophyllene ratio of between 50:1 and 250:1, and at least one terpene selected from the group consisting of linalool in a THC/linalool ratio of between 450:1 and 1400:1, alpha-humulene in a THC/alpha-humulene ratio of between 300:1 and 725:1, nerolidol in a THC/nerolidol ratio of between 200:1 and 600:1, caryophyllene oxide in a THC/caryophyllene oxide ratio of between 1000:1 and 2150:1, alpha-pinene in a THC/alpha-pinene ratio of between 5850:1 and 16000:1, camphene in a THC/camphene ratio of between 7300:1 and 132950:1, beta-pinene in a THC/beta-pinene ratio of between 6475:1 and 23100:1, beta-myrcene in a THC/beta-myrcene ratio of between 1950:1 and 6300:1, limonene in a THC/limonene ratio of between 1650:1 and 5050:1, eucalyptol in a THC/eucalyptol ratio of between 5050:1 and 38650:1, ocimene in a THC/ocimene ratio of between 2500:1 and 44700:1, gamma-terpinene a THC/gamma-terpinene ratio of between 4700:1 and 35750:1, terpinolene in a THC/terpinolene ratio of between 5450:1 and 129850:1, alpha-terpinene in a THC/alpha-terpinene ratio of between 5150:1 and 58550:1, para-cymene in a THC/para-cymene ratio of between 4700:1 and 56700:1, isopulegol in a THC/isopulegol ratio of between 4000:1 and 8000:1 and geraniol in a THC/geraniol ratio of between 1300:1 and 18250:1.

More preferably, in the present disclosure, the composition described herein may comprise THC in an amount of between about 4% and 6% THC by weight of the composition, a THC/alpha-bisabolol ratio of between 100:1 and 450:1, a THC/guaiol ratio of between 50:1 and 250:1, and a THC/beta-caryophyllene ratio of between 50:1 and 250:1 and at least one terpene selected from the group consisting of linalool in a THC/linalool ratio of between 450:1 and 1400:1, alpha-humulene in a THC/alpha-humulene ratio of between 300:1 and 725:1, nerolidol in a THC/nerolidol ratio of between 200:1 and 600:1, caryophyllene oxide in a THC/caryophyllene oxide ratio of between 1000:1 and 2150:1, alpha-pinene in a THC/alpha-pinene ratio of between 5850:1 and 16000:1, camphene in a THC/camphene ratio of between 7300:1 and 132950:1, beta-pinene in a THC/beta-pinene ratio of between 6475:1 and 23100:1, beta-myrcene in a THC/beta-myrcene ratio of between 1950:1 and 6300:1, limonene in a THC/limonene ratio of between 1650:1 and 5050:1, eucalyptol in a THC/eucalyptol ratio of between 5050:1 and 38650:1, ocimene in a THC/ocimene ratio of between 2500:1 and 44700:1, gamma-terpinene a THC/gamma-terpinene ratio of between 4700:1 and 35750:1, terpinolene in a THC/terpinolene ratio of between 5450:1 and 129850:1, alpha-terpinene in a THC/alpha-terpinene ratio of between 5150:1 and 58550:1, para-cymene in a THC/para-cymene ratio of between 4700:1 and 56700:1, isopulegol in a THC/isopulegol ratio of between 4000:1 and 8000:1 and geraniol in a THC/geraniol ratio of between 1300:1 and 18250:1.

Moreover, in the context of the present disclosure, the composition described herein may comprise alpha-bisabolol in an amount of between about 0.01% and 0.04% by weight of the composition, guaiol in an amount of between about 0.015% and 0.05% by weight of the composition, beta-caryophyllene in an amount of between about 0.02% and 0.05% by weight of the composition, and at least one terpene selected from the group consisting of linalool in an amount of between 0.003% and 0.0085% by weight of the composition, alpha-humulene in an amount of between 0.006% and 0.017% by weight of the composition, nerolidol in an amount of between 0.007% and 0.021% by weight of the composition, caryophyllene oxide in an amount of between 0.001% and 0.0055% by weight of the composition, alpha-pinene in an amount of between 0.0002% and 0.001% by weight of the composition, camphene in an amount of between 0.00003% and 0.00082% by weight of the composition, beta-pinene in an amount of between 0.0001% and 0.00092% by weight of the composition, beta-myrcene in an amount of between 0.0004% and 0.005% by weight of the composition, limonene in an amount of between 0.0008% and 0.0022% by weight of the composition, eucalyptol in an amount of between 0.0001% and 0.0012% by weight of the composition, ocimene in an amount of between 0.00009% and 0.0023% by weight of the composition, gamma-terpinene in an amount of between 0.0001% and 0.0013% by weight of the composition, terpinolene in an amount of between 0.00003% and 0.001% by weight of the composition, alpha-terpinene in an amount of between 0.00006% and 0.0012% by weight of the composition, para-cymene in an amount of between 0.00007% and 0.0013% by weight of the composition, isopulegol in an amount of between 0.0005% and 0.0015% by weight of the composition, and geraniol in an amount of between 0.0002% and 0.0044% by weight of the composition, and further comprises a THC/alpha-bisabolol ratio of between 100:1 and 450:1, a THC/guaiol ratio of between 50:1 and 250:1, and a THC/beta-caryophyllene ratio of between 50:1 and 250:1 and at least one terpene selected from the group consisting of linalool in a THC/linalool ratio of between 450:1 and 1400:1, alpha-humulene in a THC/alpha-humulene ratio of between 300:1 and 725:1, nerolidol in a THC/nerolidol ratio of between 200:1 and 600:1, caryophyllene oxide in a THC/caryophyllene oxide ratio of between 1000:1 and 2150:1, alpha-pinene in a THC/alpha-pinene ratio of between 5850:1 and 16000:1, camphene in a THC/camphene ratio of between 7300:1 and 132950:1, beta-pinene in a THC/beta-pinene ratio of between 6475:1 and 23100:1, beta-myrcene in a THC/beta-myrcene ratio of between 1950:1 and 6300:1, limonene in a THC/limonene ratio of between 1650:1 and 5050:1, eucalyptol in a THC/eucalyptol ratio of between 5050:1 and 38650:1, ocimene in a THC/ocimene ratio of between 2500:1 and 44700:1, gamma-terpinene a THC/gamma-terpinene ratio of between 4700:1 and 35750:1, terpinolene in a THC/terpinolene ratio of between 5450:1 and 129850:1, alpha-terpinene in a THC/alpha-terpinene ratio of between 5150:1 and 58550:1, para-cymene in a THC/para-cymene ratio of between 4700:1 and 56700:1, isopulegol in a THC/isopulegol ratio of between 4000:1 and 8000:1 and geraniol in a THC/geraniol ratio of between 1300:1 and 18250:1.

The skilled person will appreciate that certain terpenes selected from the group consisting of linalool, alpha-humulene, nerolidol, caryophyllene oxide, alpha-pinene, camphene, beta-pinene, beta-myrcene, limonene, eucalyptol, ocimene, gamma-terpinene, terpinolene, alpha-terpinene, para-cymene, isopulegol and geraniol are present in the composition but may become non-detectable because their concentration may be below the identification threshold or detection limit. Accordingly, in the present disclosure, the composition described herein may comprise THC in an amount of 5% THC and at least one of group of linalool in an amount of 0.003% and 0.0085%, most preferably 0.004% and 0.007% by weight of the composition, alpha-humulene in an amount of between 0.006% and 0.017%, most preferably 0.008 and 0.014 percent by weight of the composition, nerolidol in an amount of between 0.007% and 0.021%, most preferably 0.009% and 0.017% by weight of the composition, caryophyllene oxide in an amount of between 0.001% and 0.0055%, most preferably 0.002% and 0.005% by weight of the composition, and limonene in an amount of between 0.0008% and 0.0022%, most preferably 0.001% and 0.002% by weight of the composition. The composition may also comprise THC in an amount of 5% THC by weight of the composition, a THC/alpha-bisabolol ratio of between 160:1 and 295:1, a THC/guaiol ratio of between 105:1 and 200:1 and a THC/beta-caryophyllene ratio of between 105:1 and 200:1 and further comprises at least one terpene selected from the group consisting of linalool in a THC/linalool ratio of between 500:1 and 1330:1, alpha-humulene in a THC/alpha-humulene ratio of between 355:1 and 665:1, nerolidol in a THC/nerolidol ratio of between 285:1 and 535:1, caryophyllene oxide in a THC/caryophyllene oxide ratio of between 1090:1 and 2080:1 and limonene in a THC/limonene ratio of between 1700:1 and 4970:1.

Further, in the context of the present disclosure, the composition described herein may comprise alpha-bisabolol in an amount of between about 0.017% and 0.031% by weight of the composition, guaiol in an amount of between about 0.025% and 0.046% by weight of the composition, beta-caryophyllene in an amount of between about 0.025% and 0.047% by weight of the composition, at least one terpene selected from the group consisting of linalool in an amount of between 0.004% and 0.007% by weight of the composition, alpha-humulene in an amount of between 0.008% and 0.014% by weight of the composition, nerolidol in an amount of between 0.009% and 0.017% by weight of the composition, caryophyllene oxide in an amount of between 0.002% and 0.005% by weight of the composition, and limonene in an amount of between 0.001% and 0.002% by weight of the composition, and may further comprise a THC/alpha-bisabolol ratio of between 160:1 and 295:1, a THC/guaiol ratio of between 105:1 and 200:1 and a THC/beta-caryophyllene ratio of between 105:1 and 200:1 and further comprises at least one terpene selected from the group consisting of linalool in a THC/linalool ratio of between 500:1 and 1330:1, alpha-humulene in a THC/alpha-humulene ratio of between 355:1 and 665:1, nerolidol in a THC/nerolidol ratio of between 285:1 and 535:1, caryophyllene oxide in a THC/caryophyllene oxide ratio of between 1090:1 and 2080:1, and limonene in a THC/limonene ratio of between 1700:1 and 4970:1.

In the present disclosure, it is most preferred that the composition comprises the components set out in Table 1 below.

**Table 1: Preferred amounts of components comprised in the composition of the disclosure.**

| **Component** | **Amount in composition [% of weight of the extract]** |
|---|---|
| THC | 5 |
| alpha-bisabolol | 0.017 to 0.031 |
| guaiol | 0.025 to 0.046 |
| beta-caryophyllene | 0.025 to 0.047 |
| linalool | 0.004 to 0.007 |
| alpha-humulene | 0.008 to 0.014 |
| nerolidol | 0.009 to 0.017 |
| caryophyllene oxide | 0.002 to 0.005 |
| limonene | 0.001 to 0.002 |

Suitable methods to qualitatively and quantitatively determine, analyze, control or set the content of THC, alpha-bisabolol, guaiol, beta-caryophyllene and/or terpenes selected from the group of linalool, alpha-humulene, nerolidol, caryophyllene oxide, alpha-pinene, camphene, beta-pinene, beta-myrcene, limonene, eucalyptol, ocimene, gamma-terpinene, terpinolene, alpha-terpinene, para-cymene, isopulegol and geraniol are known in the art and include Thin Layer Chromatography (TLC) or High-Performance Liquid Chromatography (HPLC) (Eingestellter Cannabisextrakt (Cannabis extractum normatum), Deutsches Arzneibuch (DAB) 2021, ISBN: 978-3-7692-7804-0) and gas chromatography (GC) with detection by mass spectrometer (Ferrer I. and Thurman M.E., Elsevier 2020. ISBN: 978-0-4446-4341-4; Opie S.R., Springer Nature 2021. ISBN: 978-3-030-62715-7).

The composition described herein may be any suitable composition, provided that it comprises a combination of the substances/components THC, alpha-bisabolol, guaiol, beta-caryophyllene and at least one terpene selected from the group consisting of linalool, alpha-humulene, nerolidol, caryophyllene oxide, alpha-pinene, camphene, beta-pinene, beta-myrcene, limonene, eucalyptol, ocimene, gamma-terpinene, terpinolene, alpha-terpinene, para-cymene, isopulegol and geraniol. The composition is preferably in liquid form. In the context of the present disclosure, the composition may also be provided in solid, e.g. powder, form to be subjected to reconstitution into liquid form, thereby providing the composition described herein. The composition may be an aqueous solution or a non-aqueous solution such as an alcoholic solution, or a mixture of both in a suitable ratio. The composition may be produced from a precursor material naturally comprising THC or its prodrug THCA, alpha-bisabolol, guaiol, beta-caryophyllene or terpene(s) selected from the group consisting of linalool, alpha-humulene, nerolidol, caryophyllene oxide, alpha-pinene, camphene, beta-pinene, beta-myrcene, limonene, eucalyptol, ocimene, gamma-terpinene, terpinolene, alpha-terpinene, para-cymene, isopulegol and geraniol. It is evident for the skilled person that THC content as used herein may also refer to THC equivalents which is the sum of THC and THCA*0.877. Accordingly, in the context of the present invention, the "THC content" may refer to the sum of THC and THCA. THC and THCA may be determined and quantified by methods known in the art, e.g. by using liquid chromatography with addition of respective THC and THCA reference standards (Deutsches Arzneibuch (DAB) 2018, ISBN: 978-3-7692-7217-8), and Danish Medicines Standards 2020.0, BEK Nr. 1231 af.)

In the context of the present invention, it should be understood that THC as used in the present invention may also be initially present in its carboxylated form THCA to be decarboxylated to the active THC comprised in the composition described herein. In the present invention, decarboxylation of THCA to THC (equivalent to other cannabinoids) may be performed by any suitable method known in the art. Preferably, decarboxylation, of e.g. *a Cannabis* plant extract, is conducted via thermally induced decarboxylation under vacuum (in vacuo). Therefore, a rotary evaporator is used by adjusting water bath temperature, pressure, and rotation to 80°C, 185 mbar and 150 rpm, respectively. A suitable decarboxylation time amounts to 72 h. One non-limiting example of a precursor material to produce the composition described herein may be an extract naturally comprising THC(A), alpha-bisabolol, guaiol, beta-caryophyllene and at least one terpene selected from the group consisting of linalool, alpha-humulene, nerolidol, caryophyllene oxide, alpha-pinene, camphene, beta-pinene, beta-myrcene, limonene, eucalyptol, ocimene, gamma-terpinene, terpinolene, alpha-terpinene, para-cymene, isopulegol and geraniol e.g. the extract as described herein. In the context of the present invention, THC, alpha-bisabolol, guaiol, beta-caryophyllene and at least one terpene selected from the group consisting of linalool, alpha-humulene, nerolidol, caryophyllene oxide, alpha-pinene, camphene, beta-pinene, beta-myrcene, limonene, eucalyptol, ocimene, gamma-terpinene, terpinolene, alpha-terpinene, para-cymene, isopulegol and geraniol may have been artificially added/combined to be comprised in the composition described herein. In case THC, alpha-bisabolol, guaiol, beta-caryophyllene and at least one terpene selected from the group consisting of linalool, alpha-humulene, nerolidol, caryophyllene oxide, alpha-pinene, camphene, beta-pinene, beta-myrcene, limonene, eucalyptol, ocimene, gamma-terpinene, terpinolene, alpha-terpinene, para-cymene, isopulegol and geraniol may be artificially added/combined to be comprised in the composition described herein, the pure substances THC (or THCA to be subjected to decarboxylation as described above), alpha-bisabolol, guaiol, beta-caryophyllene and at least one terpene selected from the group consisting of linalool, alpha-humulene, nerolidol, caryophyllene oxide, alpha-pinene, camphene, beta-pinene, beta-myrcene, limonene, eucalyptol, ocimene, gamma-terpinene, terpinolene, alpha-terpinene, para-cymene, isopulegol and geraniol or suitable precursor substances may be commercially obtained. As explained in the Example section, THC (e.g. naturally produced or isolated, or synthetically produced dronabinol) may be obtained from commercial suppliers such as LGC Standards, Smolecule, Benchchem or Cerilliant, and alpha-bisabolol, guaiol, beta-caryophyllene and at least one terpene selected from the group consisting of linalool, alpha-humulene, nerolidol, caryophyllene oxide, alpha-pinene, camphene, beta-pinene, beta-myrcene, limonene, eucalyptol, ocimene, gamma-terpinene, terpinolene, alpha-terpinene, para-cymene, isopulegol and geraniol may be obtained from commercial suppliers such as Sigma-Aldrich, Biomol/Cayman, LGC Standards, Alfa Chemistry, Molport or Restek, respectively. They may also be isolated, chemically synthesized by means known in the art or heterologously produced in a suitable cell or organism. For example, THC as used in the composition described herein or its prodrug THCA may be produced via heterologous cannabinoid production in a biosynthetic process as e.g. reported in the literature (e.g. Lou et al., Nature 2019, 567: 123-126). The skilled person knows suitable methods for recovering and purifying recombinant products. For example, THC may be isolated by chromatographic methods, e.g. continuous simulated moving bed (SMB) chromatography or centrifugal partition chromatography (CPC) (Götz M.R, Dissertation, Julius Maximilians Universität Würzburg 2018), and terpenes such as alpha-bisabolol, guaiol, beta-caryophyllene and terpene(s) selected from the group consisting of linalool, alpha-humulene, nerolidol, caryophyllene oxide, alpha-pinene, camphene, beta-pinene, beta-myrcene, limonene, eucalyptol, ocimene, gamma-terpinene, terpinolene, alpha-terpinene, para-cymene, isopulegol and geraniol can be e.g. isolated by short path distillation and purified by liquid gas chromatography (Breitmaier E., John Wiley & Sons 2012, ISBN: 978-3-527-31498-0; Rostagno, M.A. and Prado J.M., Royal Society of Chemistry, 2013, ISBN: 978-1-84973-606-0; Patil A.S., Studera Press 2020, ISBN: 978-9-38588-319-4, Mandal S.C., Mandal V., and Das A.K., Academic press 2015, ISBN: 9780128023259).

In the present invention, it is preferred that the composition naturally comprises THC, alpha-bisabolol, guaiol, beta-caryophyllene and at least one terpene selected from the group consisting of linalool, alpha-humulene, nerolidol, caryophyllene oxide, alpha-pinene, camphene, beta-pinene, beta-myrcene, limonene, eucalyptol, ocimene, gamma-terpinene, terpinolene, alpha-terpinene, para-cymene, isopulegol and geraniol. That is, the composition of the present invention preferably comprises an extract derived from/obtainable from a plant that does endogenously contain or produce THC(A), alpha-bisabolol, guaiol, beta-caryophyllene and at least one terpene selected from the group consisting of linalool, alpha-humulene, nerolidol, caryophyllene oxide, alpha-pinene, camphene, beta-pinene, beta-myrcene, limonene, eucalyptol, ocimene, gamma-terpinene, terpinolene, alpha-terpinene, para-cymene, isopulegol and geraniol. In the context of the present invention, the term "extract", without further specification, is intended to generally refer to any form of the product of extraction, optionally minus the extracting agent, regardless of the physical form (e.g. viscous, pasty or solid). In case the extract is derived from/obtainable from a plant, such extract is referred to as "plant extract". Accordingly, in the context of the present invention, the term "plant extract" refers to an extract of plant material in a suitable extractant, optionally minus the extractant.

Such plant extract may preferably be derived from/obtainable from a plant that does endogenously contain or produce cannabinoids, cannabinoid-like substances and/or cannabimimetic compounds as well as terpenes and/or terpenoids, provided that such plant is capable of producing THC(A), alpha-bisabolol, guaiol, beta-caryophyllene and at least one terpene selected from the group consisting of linalool, alpha-humulene, nerolidol, caryophyllene oxide, alpha-pinene, camphene, beta-pinene, beta-myrcene, limonene, eucalyptol, ocimene, gamma-terpinene, terpinolene, alpha-terpinene, para-cymene, isopulegol and geraniol. The skilled person will understand that a plant that does endogenously contain or produce cannabinoids, cannabinoid-like substances and/or cannabimimetic compounds as well as terpenes and/or terpenoids may be genetically modified, i.e. it may be a transgenic plant or a transgenic plant cell which differs from naturally occurring ones due to genetic modification. The term "cannabinoid" as used herein relates to any cannabinoid that has been isolated from a plant or has been synthetically or recombinantly created to have activity in the endocannabinoid system, and includes cannabinoid-like substances and/or cannabimimetic compounds. Cannabinoids synthesized by plant sources are considered to be phytocannabinoids, i.e. plant-based cannabinoids. In the context of the present invention, the term "cannabinoid" may be interchangeably used with "phytocannabinoids". The term "cannabinoid fraction" is used to describe the combination of cannabinoid, cannabinoid-like substances or cannabimimetic compounds present in the plant extract. To date, over 100 cannabinoids have been identified in *Cannabis* plants. A comprehensive, non-limiting lists of such cannabinoids in *Cannabis* may be found in ElSohly M. A. and Gul W., in Handbook of Cannabis, Oxford University Press (2014), pp.3-22 and Radwan, M.M. et al. Molecules 2021, 26(9): 2774. One such example is delta-9-tetrahydrocannabinol (THC). The term "terpene(s)" or "terpenoid(s)" as used herein refers to a class of hydrocarbon molecules. Terpenes are derived from units of isoprene, such that the basic molecular formula of terpenes are multiples of the isoprene unit, i.e. (C₅H₈)ₙ, wherein n is the number of linked isoprene units. Examples thereof are alpha-bisabolol, guaiol, beta-caryophyllene, linalool, alpha-humulene, nerolidol, caryophyllene oxide, alpha-pinene, camphene, beta-pinene, beta-myrcene, limonene, eucalyptol, ocimene, gamma-terpinene, terpinolene, alpha-terpinene, para-cymene, isopulegol and geraniol. Exhaustive lists of *Cannabis* terpenes are further well-known in the art and include inene, terpineol, valencene, beta-bisabolol, phellandrene, carene, fenchol, borneol, phytol, sabinene, camphor, isoborneol, menthol, and cedrane (Radwan, M.M. et al. Molecules 2021, 26(9): 2774). Terpenoids are terpene compounds that have been further metabolized in the plant, typically through an oxidative process, and therefore usually contain at least one oxygen atom. The term "terpene fraction" is used to describe the combination of terpene and terpenoid compounds present in the plant extract. Cannabinoids are described to be unique terpenophenolic metabolites found only in *Cannabis* plants (Sirikantaramas and Taura, Springer 2017, 1st edition, Chapter 8, pp. 183-206). However, alternative cannabinoid-like substances or cannabimimetic compounds can not only be found in *Cannabis* but also in other plant species including *Acmella oleracea* (Dallazan et al., Inflammopharmacology 2019, 28, pp.175-186), *Echinaceae angustifolia* (Raduner et al., J Biol Chem. 2006, 281(20), pp. 14192-14206), *Echinaceae purpurea* (Raduner et al., J Biol Chem. 2006, 281(20), pp. 14192-14206), *Helichrysum umbraculigerum* (Pollastro et al., Fitoterapia 2018, 126, pp. 35-39), *Heliopsis helianthoides* (Hajdu et al., J Nat Prod. 2014, 77(7), pp. 1663-1669), *Lepidium meyenii* (Hajdu et al., J Nat Prod. 2014, 77(7), pp. 1663-1669), *Piper methysticum* (Ligresti et al., Pharmacol Res. 2012, 6(2), pp. 163-169), *Piper nigrum* (Reynoso-Moreno et al., J Agric Food Chem. 2017, 65(43), pp. 9435-9442), *Radula marginata* (Hussain et al., Phytochem rev. (2019), 18, pp. 953-965), *Radula perrottetii* (Chicca et al., Neurophysiol. 2018, 4(10)), *Rhododendron anthopogonoides* (Iwata and Kitanaka, Chem Pharm Bull. 2011, 59(11), pp. 1409-1412) and *Tuber melanosporum* (Degenhardt et al., Biology, Pharmacology, Diagnosis, and Treatment 2017, Chapter 2, p. 13-23), (Pacioni et al., Phytochemistry 2015, 110, pp. 104-110).

Accordingly, in the context of the present disclosure, the composition described herein may also comprise an extract derived from/obtainable from a plant that is capable of producing cannabinoids, cannabinoid-like substances or cannabimimetic compounds as well as terpenes and/or terpenoids, but does not endogenously contain or produce them. As such, the skilled person will understand that the plant extract may be obtainable from transgenic plants or plant cells which differ from naturally occurring ones due to genetic modification. Genetically modified plants or plant cells do not naturally occur, i.e., cannot be found in nature, and differ substantially from naturally occurring plants or plant cells due to the introduction of foreign genetic material, for example a foreign nucleic acid molecule. For example, the plant extract used in the present invention may also be derived from a transgenic plant, capable of heterologously producing THC(A), alpha-bisabolol, guaiol beta-caryophyllene and at least one terpene selected from the group consisting of linalool, alpha-humulene, nerolidol, caryophyllene oxide, alpha-pinene, camphene, beta-pinene, beta-myrcene, limonene, eucalyptol, ocimene, gamma-terpinene, terpinolene, alpha-terpinene, para-cymene, isopulegol and geraniol in a biosynthetic process. In case the composition of the invention comprises an extract derived/obtained from a plant as described herein, it is preferred that the extract has substantially the same THC/terpene ratio as the original plant used to obtain the extract and may concentrates it into a more potent form. Further, in the context of the present invention, it may be preferred that the plant extract is also decarboxylated to ensure that THCA as endogenously present in the plant extract is converted into THC.

In the context of the present invention, the term "plant" refers to any various photosynthetic, eukaryotic multicellular organisms of the kingdom *Plantae,* characteristically producing embryos, containing chloroplasts, having cellulose cell walls and lacking locomotion. As used herein, a "plant" includes any plant or part of a plant at any stage of development and progeny thereof containing cannabinoid, terpene and terpenoid compounds. The plant material may be obtained from solid plant material but is not limited thereto. The solid plant material may be obtained from the whole plant or parts thereof, which include plant cells, plant organs, leaves (in the context of the present invention, the term "leaves" includes any plant leave including flower leaves, sugar leaves, fan leaves and the like), flowers, stems, fruits, roots, trichomes, meristems, plant seeds, protoplasts, callus, and any groups of plant cells organized into structural and/or functional units. Moreover, in the context of the present invention, the plant material may be a plant cell culture, specifically a plant suspension cell culture in a liquid medium. Methods to establish an *in vitro* plant cell culture from solid plant material are known to those skilled in the art and include callogenesis (Espinosa-Leal et al., Planta (2018), 248:1-18). In the present invention, when a *Cannabis* plant is used to obtain the extract as described herein which is preferably comprised in the composition of the present invention, the plant material is preferably trichome-rich material of the plant. Accordingly, in the present invention, the plant material is preferably obtained from the flower material of the *Cannabis* plant. In case the flower material is obtained from a *Cannabis* plant, the flower material is preferably trimmed and dried before use and the flower material subjected to extraction is preferably not exceeding the shelf-life.

"Drying" as used herein means that the water/moisture in the plant material is reduced. Several drying methods are known to the skilled person, including freeze drying. In the context of the present invention, it is preferred that drying is performed in a drying chamber by placing the plant material on trays through which air flows. Such drying in a drying chamber normally takes place at a temperature of 18°C to 30°C by continuous flow of dehumidified air through the product. In the context of the present invention, drying preferably takes at least 4 days (96 h) or until the water content of the plant material, e.g. flower material, is below 10 percent. The skilled person is aware how to measure the temperature via standard methods. Furthermore, the skilled person knows how to determine the water content in plant material, for example as described in European Pharmacopoeia 10th Edition, Deutscher Apotheker Verlag 2019, ISBN: 978-3-7692-7453-0. The term "trimming" in the context of the flower material as used herein means that the leaves, particularly the sugar leaves and/or fan leaves, are removed from the flowers. Trimming of the flower material may be performed as described in the following. The flowers of the *Cannabis* plant may be separated from the rest of the plant manually (e.g. by hand with a scissors) or with a destalker (e.g. MB Bucker 500; Master Bucker 500; www.masterproducts.es).

The terms "flower", "flower material" and "blossom" may be used interchangeably herein. The skilled person knows that in *Cannabis* plants the flowers are arranged in so-called colas. Accordingly, flower material as used herein also refers to said colas. The leaves of the *Cannabis* plants may be distinguished in fan leaves and sugar leaves. Fan leaves are the large, primary leaves on the *Cannabis* plant. Sugar leaves develop and grow out of *Cannabis* flowers in the plant's flowering stage. In the present invention, it is preferred that said sugar leaves are removed from the flower material as described herein.

Trichomes (i.e. resin glands) of the *Cannabis* plant material are nearly microscopic, mushroom-like protrusions from the surface of the *Cannabis* plant, mainly of the flower buds. While relatively complex, trichomes are comprised primarily of a stalk and a head. The production of cannabinoids such as THC occurs predominantly in the head of the trichome. Cannabinoids are concentrated in the trichomes of the plant. Accordingly, it is evident for the skilled person that the extract as described herein may be provided by using (isolated) trichomes of *Cannabis* plants.

In the context of the present invention, the plant material used to obtain the extract may be fresh, freeze dried, dried or frozen, but is preferably dried. In addition to trimming the plant material used herein, it may be broken down to produce a plant material of a smaller size. In other words, the plant material may also be mechanically comminuted prior to or during mixing with the extractant. All comminution methods known to the person skilled in the art are suitable including smashing, milling, grinding, and chipping (Salman, A. D., Ghadiri M., and Michael Hounslow M.J., Elsevier Science & Technology 2007, ISBN: 978-0-08055-346-7). The plant extract as described herein can be a crude extract or can optionally be subjected to further customary steps, such that the extract may also be, without limitation, fractionated, sub-fractionated, separated, isolated, purified and/or subjected to centrifugation, purification, and/or concentration. Concentration may be performed by methods known in the art. In the context of the present invention, the extraction solvent is preferably concentrated via evaporation in vacuo by using a rotary evaporator. As a matter of example, vacuum evaporation may be performed in a water bath with 72°C temperature, a pressure of 185 mbar, and a rotation speed of 150 rpm. In the present invention, it is preferred that, in that way, an almost solvent-free extract is established. The skilled person will appreciate that other suitable conditions for vacuum evaporation are known in the art and could be easily established. Thus, in the present invention it is preferred that the composition of the invention, if comprising a plant extract, comprises an extract which has been subjected to evaporation, e.g. as described above. In other words, in the present invention it is preferred that, in case the composition of the present invention comprises an extract, the accompanied extractant/solvent as described herein is completely or almost completely removed prior to incorporation of the extract into the composition of the invention, such that the composition is solvent-free or almost solvent free. Further, it is preferred that such extract has been subjected to decarboxylation to produce THC from THCA.

In the present invention, it is even more preferred that the extract is a plant extract obtained from a *Cannabis* plant or a progeny thereof (hereinafter termed *"Cannabis* plant extract" or *"Cannabis* soft extract"). In the context of the present invention, the term *"Cannabis* plant(s)" is understood as describing a plant of the genus *Cannabis* (Family *Cannabaceae*)*.* Species of the genus *Cannabis* include *Cannabis sativa* and *Cannabis indica,* and *Cannabis* varieties are known in the art, such as *Jack Herer, Chemdawg, Bubba Kush, Trainwreck, Super Silver Haze, Pure Kush, EI Nino, Himalayan Gold, Skunk #1, White Widow, Warlock CBD, Pink Kush, OG Kush, Super Lemon Haze, Jack the Ripper, Lemon Skunk,* and *Hash Plant.* Furthermore, it includes the *Cannabis* plants resulting from genetic crosses, self-crosses, natural selections or hybrids of the above-mentioned plants and *Cannabis* chemovars. Even more preferably, the plant extract, i.e. *Cannabis* plant extract, is obtained from a *Cannabis* plant of the strain "Jack Herer" or a progeny thereof. In the context of the present invention, such progeny may be a *Cannabis* plant as obtained, for example but not limited thereto, by natural selection of seeds of a *Cannabis* plant, e.g. of the strain "Jack Herer". *Cannabis* plants, e.g. of the strain "Jack Herer", may be selected for production of THC(A), alpha-bisabolol, guaiol, beta-caryophyllene and at least one terpene selected from the group consisting of linalool, alpha-humulene, nerolidol, caryophyllene oxide, alpha-pinene, camphene, beta-pinene, beta-myrcene, limonene, eucalyptol, ocimene, gamma-terpinene, terpinolene, alpha-terpinene, para-cymene, isopulegol and geraniol by a process as exemplarily described in the following. *Cannabis* plant seeds, e.g. of the strain "Jack Herer", may be commercially obtained. The seeds may be cultivated by ordinary cultivation methods known to the skilled person, e.g. for between 3 to 14 days until germination. Based on germination and growing behavior, the skilled person can select specific seeds for further cultivation. Such decisions may generally be made based upon analytical results and/or observations by the cultivar during cultivation. Once the selected seeds are germinated and rooted, they may be further cultivated for three to six weeks by ordinary cultivation methods known to the skilled person to establish a stock of seed plants. Fertilization, water supply, pest control, disease monitoring and trimming of the plants may be optimized by a skilled person. Based on growing behaviour, the skilled person can select specific seed plants for further cultivation. From these selected plants (in the following passage referred to as individual plants), cuttings may be taken to secure genetic material of the individual plant. The individual plants may then be brought into flowering phase by reducing daylength from > 18 hours to < 12 hours of daylight. Generally, the environmental conditions for rooting, vegetative and flowering phase need to be fulfilled. Then, the individual plants may finally be selected based on the following criteria: growing behavior, flowering behavior, successful cultivation and good rooting behavior of cuttings taken therefrom, susceptibility to diseases (e.g. grey mould) and analytical results for e.g. THC content, content of other cannabinoids and terpene profile. As part of the selection process, batch-to-batch consistency may be analysed based on cannabinoid and terpene profile. Alternative ways of obtaining such progeny may be, for example, selective breeding or cross breeding, and genetic engineering by using methods such as CRISPR/Cas technology. The skilled person routinely knows methods how to test such progenies for THC(A), alpha-bisabolol, guaiol, beta-caryophyllene, linalool, alpha-humulene, nerolidol, caryophyllene oxide, alpha-pinene, camphene, beta-pinene, beta-myrcene, limonene, eucalyptol, ocimene, gamma-terpinene, terpinolene, alpha-terpinene, para-cymene, isopulegol and/or geraniol production, e.g. by using TLC, HPLC or GC with mass spectrometric detection as described above. Such selected progenies, i.e. chemovars (plants distinguished by the cannabinoids produced, rather than the morphological characteristics of the plant), can be bred by a variety of plant breeding techniques which will be familiar to a person skilled in the art. Propagation of the plants by cuttings for production material ensures that the genotype is fixed and that each crop of plants contains the cannabinoids in substantially the same ratio.

Most preferably, the plant extract, i.e. *Cannabis* plant extract, used in the present invention is obtained by a *Cannabis* variety referred to as DKJ127 and deposited by the Community Plant Variety Office (deposited by Vertanical GmbH with the application number A202104053 (provisional designation of the variety: dk-j127; Botanical taxon: *Cannabis sativa* L.; Breeder's reference: DK-J127; Variety denomination: DKJ127; Application No.: 2021/3223; Electronic Application No: A202104053; Date of receipt by the Community Plant Variety Office: 09/12/2021)) and/or obtained by the process as described in patent application EP22154007.3 (PCT/EP2023/052073). The *Cannabis* plant extract as described herein may comprise a saponifiable fraction comprising e.g. fatty acids and triglycerides, and an unsaponifiable fraction comprising e.g. cannabinoids such as THC(A) and CBG as well as terpenes and/or terpenoids. The saponifiable fraction is understood as the portion of total lipid in the extract that, after treatment with hot alkali, is soluble in water and insoluble in ether. In the present invention, the saponifiable fraction may be between 5% and 20%, 5% and 30%, or 5% and 40% of the *Cannabis* plant extract as disclosed herein. Preferably, the saponifiable fraction may be between 10% and 20% of the *Cannabis* plant extract as disclosed herein. In the present invention, the unsaponifiable fraction may be between 60% and 95%, 70% and 95%, or 80% and 95% of the *Cannabis* plant extract as disclosed herein. THC(A) as comprised in the unsaponifiable fraction may constitute more than 50%, 60%, 70%, 80% or 85% of the *Cannabis* plant extract as disclosed herein. Preferably, THC(A) as comprised in the unsaponifiable fraction constitutes more than 85%, preferably between 80% or 90% of the *Cannabis* plant extract as disclosed herein. Alpha-bisabolol as comprised in the unsaponifiable fraction may constitute more than 0.1%, 0.2%, 0.25%, 0.27%, 0.275%, 0.28%, 0.3% or 0.4% of the *Cannabis* plant extract as disclosed herein. Preferably, alpha-bisabolol as comprised in the unsaponifiable fraction constitutes more than 0.25% of the *Cannabis* plant extract as disclosed herein, preferably between 0.25% and 0.35% of the *Cannabis* plant extract as disclosed herein. Guaiol as comprised in the unsaponifiable fraction may constitute more than 0.3%, 0.4%, 0.49%, 0.5%, 0.6%, or 0.7% of the *Cannabis* plant extract as disclosed herein. Preferably, guaiol as comprised in the unsaponifiable fraction constitutes more than 0.49% of the *Cannabis* plant extract as disclosed herein, preferably between 0.49% and 0.67% of the *Cannabis* plant extract as disclosed herein. Beta-caryophyllene as comprised in the unsaponifiable fraction may constitute more than 0.3%, 0.4%, 0.49%, 0.5%, 0.6%, or 0.7% of the *Cannabis* plant extract as disclosed herein. Preferably, beta-caryophyllene as comprised in the unsaponifiable fraction constitutes more than 0.5% of the *Cannabis* plant extract as disclosed herein, preferably between 0.5% and 0.73% of the *Cannabis* plant extract as disclosed herein. Each one of linalool, alpha-humulene, nerolidol, caryophyllene oxide, alpha-pinene, camphene, beta-pinene, beta-myrcene, limonene, eucalyptol, ocimene, gamma-terpinene, terpinolene, alpha-terpinene, para-cymene, isopulegol and/or geraniol as comprised in the unsaponifiable fraction may constitute more than 0.001%, 0.005%, 0.01%, 0.05%, 0.1%, or 0.25% of the *Cannabis* plant extract as disclosed herein. Linalool as comprised in the unsaponifiable fraction preferably constitutes between 0.09% and 0.17% of the *Cannabis* plant extract as disclosed herein, alpha-humulene as comprised in the unsaponifiable fraction preferably constitutes between 0.15% and 0.29% of the *Cannabis* plant extract as disclosed herein, nerolidol as comprised in the unsaponifiable fraction preferably constitutes between 0.12% and 0.23% of the *Cannabis* plant extract as disclosed herein, caryophyllene oxide as comprised in the unsaponifiable fraction preferably constitutes between 0.02% and 0.06% of the *Cannabis* plant extract as disclosed herein, alpha-pinene as comprised in the unsaponifiable fraction preferably constitutes between 0.006% and 0.02% of the *Cannabis* plant extract as disclosed herein, camphene as comprised in the unsaponifiable fraction preferably constitutes between 0.003% and 0.07% of the *Cannabis* plant extract as disclosed herein, beta-pinene as comprised in the unsaponifiable fraction preferably constitutes between 0.005% and 0.02% of the *Cannabis* plant extract as disclosed herein, beta-myrcene as comprised in the unsaponifiable fraction preferably constitutes between 0.01% and 0.04% of the *Cannabis* plant extract as disclosed herein, limonene as comprised in the unsaponifiable fraction preferably constitutes between 0.02% and 0.05% of the *Cannabis* plant extract as disclosed herein, eucalyptol as comprised in the unsaponifiable fraction preferably constitutes between 0.005% and 0.01% of the *Cannabis* plant extract as disclosed herein, ocimene as comprised in the unsaponifiable fraction preferably constitutes between 0.01% and 0.03% of the *Cannabis* plant extract as disclosed herein, gamma-terpinene as comprised in the unsaponifiable fraction preferably constitutes between 0.005% and 0.015% of the *Cannabis* plant extract as disclosed herein, terpinolene as comprised in the unsaponifiable fraction preferably constitutes between 0.005% and 0.01% of the *Cannabis* plant extract as disclosed herein, alpha-terpinene as comprised in the unsaponifiable fraction preferably constitutes between 0.005% and 0.01% of the *Cannabis* plant extract as disclosed herein, para-cymene as comprised in the unsaponifiable fraction preferably constitutes between 0.006% and 0.02% of the *Cannabis* plant extract as disclosed herein, isopulegol as comprised in the unsaponifiable fraction preferably constitutes between 0.01% and 0.05% of the *Cannabis* plant extract as disclosed herein, and/or geraniol as comprised in the unsaponifiable fraction preferably constitutes between 0.02% and 0.05% of the *Cannabis* plant extract as disclosed herein.

It is preferred that the cannabinoid fraction in the unsaponifiable fraction, excluding THC as main cannabinoid, may be present in an amount of not more than 5% by weight of the *Cannabis* plant extract.

The skilled person knows routine procedures to produce the composition described herein comprising e.g. a fixed content of THC, alpha-bisabolol, guaiol, beta-caryophyllene, linalool, alpha-humulene, nerolidol, caryophyllene oxide, alpha-pinene, camphene, beta-pinene, beta-myrcene, limonene, eucalyptol, ocimene, gamma-terpinene, terpinolene, alpha-terpinene, para-cymene, isopulegol and/or geraniol from a plant extract. An exemplary procedure is described in the following: The plant extract, e.g. *Cannabis* plant extract, is intended to be standardized with a carrier oil, e.g. refined sesame oil of pharmaceutical grade, to a THC content of 5% (by weight of the composition) to provide the composition of described herein. This standardization may be performed in multiple, e.g. three, dilution steps. An analytical sample is taken in each dilution step and the THC content is determined via HPLC analysis to enable a precise determination of the remaining amount of carrier oil necessary to adjust the prior dilution to the desired content of 5% THC. After HPLC analysis, only 80% of the calculated remaining amount of carrier oil is added. In the last dilution step, the correct amount of carrier oil as calculated is added to provide the composition comprising the desired content of 5% THC by weight of the composition.

In the present invention, it is preferred that the plant extract as described herein is preferably substantially free of waxes and other non-specific lipid soluble material but preferably contains substantially all of the cannabinoids naturally present in the plant, most preferably in substantially the same ratios in which they occur in the intact plant, e.g. *Cannabis* plant.

In the present invention, the plant extract as defined herein is obtainable by extraction of plant material. Any extract obtained by solvent extraction, distillation methods, pressing and sublimation, decoction, digestion, percolation, maceration or any other appropriate extraction method known to the person skilled in the art, or a combination thereof may be used such as described in Rostagno, M.A. and Prado J.M., Royal Society of Chemistry, 2013, ISBN: 978-1-84973-606-0, Patil A.S., Studera Press 2020, ISBN: 978-9-38588-319-4, and Mandal S.C., Mandal V., and Das A.K., Academic press 2015, ISBN: 9780128023259. In the present invention, the extract as described herein is preferably obtained by a combination of maceration and percolation, maceration being preferably applied first.

Extracts may be formed by contacting an extractant with the plant material as defined herein. Preferred herein is solvent extraction, i.e. extraction using a solvent. It is envisaged that the plant material, preferably the *Cannabis* plant material is brought into contact with the solvent, which may also be called the extractant. In other words, in the context of the present invention, the term "extractant" refers to a suitable solvent used in the extraction of a substance from a liquid or solid material. As such, the plant material, preferably the *Cannabis* plant material, is treated with a solvent. In the present invention, it is preferred that the plant extract as defined herein is obtainable by solvent extraction. Solvent extraction is a technique whereby soluble organic compounds can be extracted from solids using extracting solvents. As described above, extraction as described herein is preferably performed by percolation. Percolation is understood as process whereby, unless otherwise prescribed, the herbal drug to be extracted is reduced to pieces of suitable size and mixed thoroughly with a portion of the prescribed extraction solvent and allowed to stand for an appropriate time. The mixture is prepared in or transferred to a percolator and more extraction solvent is added until the herbal drug is covered with a layer of extraction solvent. Subsequently, the percolate is allowed to flow slowly from the base of the percolator and collected in a tank. Percolation continues until the percolate is recovered. If the residue is pressed, the two liquids are combined. It is envisaged that the desired (pharmaceutically active) substances from the plant material dissolve in the solvent. Accordingly, it is envisaged that the e.g. cannabinoids (preferably THC) and/or other (pharmaceutically active) substances (preferably alpha-bisabolol, guaiol, beta-caryophyllene, linalool, alpha-humulene, nerolidol, caryophyllene oxide, alpha-pinene, camphene, beta-pinene, beta-myrcene, limonene, eucalyptol, ocimene, gamma-terpinene, terpinolene, alpha-terpinene, para-cymene, isopulegol and/or geraniol) from the *Cannabis* plant material dissolve in the solvent. That means that after the treatment of the *Cannabis* plant material with the solvent the e.g. cannabinoids (e.g. THC and/or CBD) and/or other (pharmaceutically active) substances (e.g. terpenes, preferably alpha-bisabolol, guaiol, beta-caryophyllene, linalool, alpha-humulene, nerolidol, caryophyllene oxide, alpha-pinene, camphene, beta-pinene, beta-myrcene, limonene, eucalyptol, ocimene, gamma-terpinene, terpinolene, alpha-terpinene, para-cymene, isopulegol and/or geraniol) are no longer present in the *Cannabis* plant material but are dissolved in the solvent. In other words, the solvent "removes" the e.g. cannabinoids (e.g. THC and/or CBD) and/or other (pharmaceutically active) substances (e.g. terpenes) from the plant material. In other words, the e.g. cannabinoids (preferably THC) and/or other (pharmaceutically active) substances (e.g. terpenes) are extracted from the plant material. The solvent with the dissolved e.g. cannabinoids (e.g. THC and/or CBD) and/or other (pharmaceutically active) substances (e.g. terpenes) can then be separated (i.e. extracted) from the plant material. The solvent with the dissolved e.g. cannabinoids (e.g. THC and/or CBD) and/or other (pharmaceutically active) substances (e.g. terpenes) separated from the plant material, preferably *Cannabis* plant material, is called the plant extract, preferably *Cannabis* plant extract.

The extraction time and temperature may vary depending on the extraction media and method, and may be readily optimized by a skilled person. An extraction time between 1 h and 10 days may be applied. More particularly, a time between 85 to 120 h, and preferably a time between 93 and 110 h may be applied. The temperature used for extraction may preferably be between 15°C and 25°C. The extraction process is preferably performed by exclusion of light.

Non-limiting examples of solvents that may be suitable are alcohols (e.g. methanol, ethanol, propanol, butanol, propylene glycol etc.), water, hydrocarbons (e.g. butane, hexane, etc.), polar organic solvents (e.g. ethyl acetate, polyethylene glycol, etc.) or a supercritical fluid (e.g. liquid CO₂). Suitable non-polar solvents may be C5-C12 straight chain or branched chain alkanes, C1-C12 alcohols or carbonate esters of C1-C12 alcohols. The more volatile solvents may be particularly useful, as they are more easily removed from the extract if desired. Although completely evident for the skilled person, it is pointed out that also mixtures of the mentioned solvents may be used for the herein described methods. Preferably, the solvent is selected from the group of ethanol, butanol, pentane, heptane, propane, ethyl ether, tert-butyl-methyl-ether, methyl-ethyl-ketone, acetone, ethyl acetate and CO₂. Corresponding solutions may contain the solvent, e.g. the alcoholic solvent, in any volume/volume (v/v) ratio suitable for extraction. In the context of the present invention, extraction is most preferably carried out using ethanol with 96% (v/v) as solvent. In the context of the present invention, solvents of pharmaceutical grade are preferably used.

Suitable conditions conducive for extraction are known to the skilled artisan. Specific parameters such as temperature, ratio of solvent/plant material and extraction time may be optimized. The skilled person can readily determine suitable ratios of plant material to solvent. As a matter of example, in case extraction is carried out using ethanol with 96% (v/v), a cost and time efficient extraction process may be carried out using between 15.9 kg and 17.5 kg (e.g. 16.7 kg) 96% (v/v) ethanol per kg of dried flowers. The solvent/extractant as described herein is preferably of pharmaceutical grade. Accordingly, the plant material, preferably, *Cannabis* plant material, more preferably, flower material of a *Cannabis* plant described herein and the solvent are preferably present in a ratio of about 1:16.8 (w/w). The term "about" as used herein means 10 percent more or 10 percent less than the denoted value.

In the present invention, any method suitable to provide the extract as described herein may be used. Suitable methods to prepare such an extract are routinely known to the skilled person and are further described in the foregoing detailed description. In the present invention, the plant extract is preferably obtainable by solvent extraction of a plant suitable to provide the extract as described herein, a *Cannabis* plant being mentioned as preferred example. Even more preferably, the plant extract is obtainable by solvent extraction of the flower material of a *Cannabis* plant as described herein. As such, the plant extract comprised in the composition of the invention may even more preferably be obtained by solvent extraction of the flower material of a *Cannabis* plant. In the present invention it is most preferred that the plant extract, which may be comprised in the composition of the invention, may be obtained by solvent extraction of the flower material of the *Cannabis* plant as deposited by the Community Plant Variety Office with the application number A202104053 or is the plant extract as obtainable by/produced by the process as described in patent application EP22154007.3 (PCT/EP2023/052073). Flower material used for extraction in the context of the invention, e.g. flower material of the *Cannabis* plant as deposited by the Community Plant Variety Office with the application number A202104053 is preferably trimmed and dried prior to subjection to extraction and/or decarboxylation.

The extract as described herein can be any extract suitable to provide the composition of the present invention and, thus, may be present in liquid, viscous, pasty or solid form. In the context of the present invention, it is preferred that the extract as described herein is present in liquid form, and more specifically, as an aqueous, alcoholic extract after evaporation. As such, the extract used to provide the composition of the present invention is most preferably an aqueous, alcoholic extract obtained by solvent extraction of the flower material of the *Cannabis* plant as deposited by the Community Plant Variety Office with the application number A202104053 after evaporation and/or decarboxylation. In the context of the present invention, the composition may also comprise the plant extract as obtainable by/produced by the process as described in the patent application EP22154007.3 (PCT/EP2023/052073).

In the context of the present invention, it should be noted that the composition described herein may comprise the extract as described herein, but does not consist of the extract as described herein. In other words, in the context of the present invention, the composition comprises the extract as described herein, preferably the plant extract as described herein, and at least one suitable further substance. That is, the composition of the invention may comprise the extract, e.g. plant extract, as described herein or a multitude of different extracts, e.g. plant extracts, as described herein, and at least one suitable further substance. In the present invention, it is preferred that the composition described herein may comprise the extract as described herein and a suitable carrier oil (or a mixture of such oils) in a suitable amount. As an example, the composition comprises/consists of the extract as described herein and a carrier oil as described herein (or a mixture of such oils) such that the THC content of the composition is adjusted to between about 2.5% and 10% or to 5% by weight of the composition. In another example, the composition described herein comprises/consists of the extract as described herein and a carrier oil as described herein (or a mixture of such oils) such that the alpha-bisabolol content of the composition is adjusted to between 0.008% and 0.065%, preferably between 0.01% and 0.04%, or most preferably between 0.017% and 0.031% by weight of the composition, the guaiol content of the composition is adjusted to between 0.012% and 0.092%, preferably between 0.015% and 0.05%, or most preferably between 0.025% and 0.046% by weight of the composition, the beta-caryophyllene content of the composition is adjusted to between 0.012% and 0.094%, preferably between 0.02% and 0.05% or most preferably between 0.025% and 0.047% by weight of the composition and/or the content of one or more of linalool is adjusted to between 0.001% and 0.014%, preferably between 0.003% and 0.0085% by weight of the composition, alpha-humulene is adjusted to between 0.003% and 0.028%, preferably between 0.006% and 0.017% by weight of the composition, nerolidol is adjusted to between 0.004% and 0.035%, preferably between 0.007% and 0.021% by weight of the composition, caryophyllene oxide is adjusted to between 0.001% and 0.0092%, preferably between 0.001% and 0.0055% by weight of the composition, alpha-pinene is adjusted to between 0.0001% and 0.002%, preferably between 0.0002% and 0.001% by weight of the composition, camphene is adjusted to between 0.00001% and 0.0015%, preferably between 0.00003% and 0.00082% by weight of the composition, beta-pinene is adjusted to between 0.0001% and 0.0015%, preferably between 0.0001% and 0.00092% by weight of the composition, beta-myrcene is adjusted to between 0.0004% and 0.005%, preferably between 0.0004% and 0.005% by weight of the composition, limonene is adjusted to between 0.0005% and 0.004%, preferably between 0.0008% and 0.0022% by weight of the composition, eucalyptol is adjusted to between 0.00006% and 0.002%, preferably between 0.0001% and 0.0012% by weight of the composition, ocimene is adjusted to between 0.00005% and 0.004%, preferably between 0.00009% and 0.0023% by weight of the composition, gamma-terpinene is adjusted to between 0.00007% and 0.002%, preferably between 0.0001% and 0.0013% by weight of the composition, terpinolene is adjusted to between 0.00002% and 0.002%, preferably between 0.00003% and 0.001% by weight of the composition, alpha-terpinene is adjusted to between 0.00004% and 0.002%, preferably between 0.00006% and 0.0012% by weight of the composition, para-cymene is adjusted to between 0.00004% and 0.002%, preferably between 0.00007% and 0.0013% by weight of the composition, isopulegol is adjusted to between 0.0003% and 0.003%, preferably between 0.0005% and 0.0015% by weight of the composition, and geraniol in is adjusted to between 0.0001% and 0.008%, preferably between 0.0002% and 0.0044% by weight of the composition.

In a more preferred example, the composition described herein comprises/consists of the extract as described herein and a carrier oil as described herein (or a mixture of such oils) such that the THC content of the composition is adjusted to between about 2.5% and 10%, more preferably to 5% by weight of the composition, the alpha-bisabolol content of the composition is adjusted to between 0.008% and 0.065%, preferably between 0.01% and 0.04%, or most preferably to between 0.017% and 0.031% by weight of the composition, the guaiol content of the composition is adjusted to between 0.012% and 0.092%, preferably between 0.015% and 0.05%, or most preferably to between 0.025% and 0.046% by weight of the composition, the beta-caryophyllene content of the composition is adjusted to between 0.012% and 0.094%, preferably between 0.02% and 0.05%, or most preferably to between 0.025% and 0.047% by weight of the composition, linalool is adjusted to between 0.001% and 0.014%, preferably between 0.003% and 0.0085% by weight of the composition, alpha-humulene is adjusted to between 0.003% and 0.028%, preferably between 0.006% and 0.017% by weight of the composition, nerolidol is adjusted to between 0.004% and 0.035%, preferably between 0.007% and 0.021% by weight of the composition, caryophyllene oxide is adjusted to between 0.001% and 0.0092%, preferably between 0.001% and 0.0055% by weight of the composition, alpha-pinene is adjusted to between 0.0001% and 0.002%, preferably between 0.0002% and 0.001% by weight of the composition, camphene is adjusted to between 0.00001% and 0.0015%, preferably between 0.00003% and 0.00082% by weight of the composition, beta-pinene is adjusted to between 0.0001% and 0.0015%, preferably between 0.0001% and 0.00092% by weight of the composition, beta-myrcene is adjusted to between 0.0004% and 0.005%, preferably between 0.0004% and 0.005% by weight of the composition, limonene is adjusted to between 0.0005% and 0.004%, preferably between 0.0008% and 0.0022% by weight of the composition, eucalyptol is adjusted to between 0.00006% and 0.002%, preferably between 0.0001% and 0.0012% by weight of the composition, ocimene is adjusted to between 0.00005% and 0.004%, preferably between 0.00009% and 0.0023% by weight of the composition, gamma-terpinene is adjusted to between 0.00007% and 0.002%, preferably between 0.0001% and 0.0013% by weight of the composition, terpinolene is adjusted to between 0.00002% and 0.002%, preferably between 0.00003% and 0.001% by weight of the composition, alpha-terpinene is adjusted to between 0.00004% and 0.002%, preferably between 0.00006% and 0.0012% by weight of the composition, para-cymene is adjusted to between 0.00004% and 0.002%, preferably between 0.00007% and 0.0013% by weight of the composition, isopulegol is adjusted to between 0.0003% and 0.003%, preferably between 0.0005% and 0.0015% by weight of the composition, and/or geraniol in is adjusted to between 0.0001% and 0.008%, preferably between 0.0002% and 0.0044% by weight of the composition.

Preferably, the composition described herein comprises/consists of the extract as described herein and a carrier oil as described herein and has a THC/alpha-bisabolol ratio of between 100:1 and 450:1, more preferably of between 160:1 and 295:1, a THC/guaiol ratio of between 50:1 and 250:1, more preferably of between 105:1 and 200:1, a THC/beta-caryophyllene ratio of between 50:1 and 250:1, more preferably of between 105:1 and 200:1, a THC/linalool ratio of between 450:1 and 1400:1, more preferably of between 500:1 and 1330:1, a THC/alpha-humulene ratio of between 300:1 and 725:1, preferably of between 355:1 and 665:1, a THC/nerolidol ratio of between 200:1 and 600:1, preferably of between 1090:1 and 2080:1, a THC/caryophyllene oxide ratio of between 1000:1 and 2150:1, preferably of between 1090:1 and 2080:1, a THC/alpha-pinene ratio of between 5850:1 and 16000:1, preferably of between 6000:1 and 15750:1, a THC/camphene ratio of between 7300:1 and 132950:1, preferably of between 7300:1 and 132950:1, a THC/beta-pinene ratio of between 6475:1 and 23100:1, preferably of between 6545:1 and 23025:1, a THC/beta-myrcene ratio of between 1950:1 and 6300:1, preferably of between 2025:1 and 6210:1, a THC/limonene ratio of between 1650:1 and 5050:1, preferably of between 1700:1 and 4970:1, a THC/eucalyptol ratio of between 5050:1 and 38650:1, preferably of between 5140:1 and 38580:1, a THC/ocimene ratio of between 2500:1 and 44700:1, preferably of between 2570:1 and 44610:1, a THC/gamma-terpinene ratio of between 4700:1 and 35750:1, preferably between 4800:1 and 35685:1, a THC/terpinolene ratio of between 5450:1 and 129850:1, preferably of between 5535:1 and 129965:1, a THC/alpha-terpinene ratio of between 5150:1 and 58550:1, preferably of 5210:1 and 58480:1, a THC/para-cymene ratio of between 4700:1 and 56700:1, preferably of 4800:1 and 56625:1, a THC/isopulegol ratio of between 4000:1 and 8000:1, preferably of between 4060:1 and 7915:1, and/or a THC/geraniol ratio of between 1300:1 and 18250:1, preferably of between 1375:1 and 18150:1.

The carrier oil, which may provide the composition described herein together with the extract as described herein, may constitute any percentage of such composition provided that such percentage is in conformity with the above presented definitions for the content of THC, alpha-bisabolol, guaiol, beta-caryophyllene and the group of linalool, alpha-humulene, nerolidol, caryophyllene oxide, alpha-pinene, camphene, beta-pinene, beta-myrcene, limonene, eucalyptol, ocimene, gamma-terpinene, terpinolene, alpha-terpinene, para-cymene, isopulegol and/or geraniol. In a preferred example, the carrier oil may be present in an amount of between 85% and 97% by weight of the composition. Such carrier oil may be a naturally derived oil (e.g. an essential oil) or one or more waxes or any combination thereof.

An "essential oil" is an oil derived by extraction (e.g. steam extraction, or contacting the plant material with an extractant) or pressing, which contains primarily hydrophobic, and generally fragrant, components of the plant material. Suitable naturally derived oils and waxes include sesame oil, olive oil, arnica oil, lavender oil, frankincense oil, lemongrass essential oil, cinnamon leaf oil, rosemary cineole oil, rosemary oil, bergamot oil, myrrh oil, sage oil, coconut oil, hemp seed oil, castor oil, bees wax or any other edible oil, or a combination thereof. Preferably, the carrier oil may be selected from the following list of sesame oil, rapeseed oil, peanut oil, almond oil, wheat germ oil, sunflower oil, olive oil, cottonseed oil, peanut oil, almond oil, borage oil, safflower oil, soybean oil, fish oil, linseed oil and medium chain triglycerides. In the present invention it is most preferred that the composition of the invention comprises a plant extract, e.g. the *Cannabis* plant extract, as described herein, and sesame oil in accordance with the definitions set out above. As explained herein above, the extract as described herein is preferably a plant extract as defined herein and even more preferably a *Cannabis* plant extract as defined herein. The carrier oil as described herein, which is used to provide the composition described herein, is preferably refined and of pharmaceutical grade.

The composition as described herein may contain a native extract, e.g. the plant extract as described herein, or the extract may be additionally modified by changing its composition, e.g., without limitation, by changing the pH or by adding one or more solvents in a preferred concentration. The extract as described herein may also be reconstituted by mixing with a suitable solvent. In some instances, the extract as described herein may also be filtered to remove particulate material, for example, by passing the extract through filter paper, a particle filter or a fine sieve with pore sizes suitable for filtration. As a matter of example, a suitable deposition rate would be 1.5 µm. The operating pressure and temperature during filtration may be optimized by the skilled person, but should preferably not exceed 5 bar and 50°C. As will be appreciated, one or more additional compounds (e.g. cannabinoid, terpene or terpenoid compounds) may be added to the composition as described herein. The addition of compounds may be to compensate for natural variations in the relative amounts of certain compounds being expressed by the *Cannabis* plant which provides the extract. The added compounds may be natural or synthetic versions of the desired compound(s).

References to "THC" or "delta-9-tetrahydrocannabinol" and "Cannabidiol" or "CBD" or "cannabinoid(s)" as used herein, will be understood to also encompass pharmaceutically acceptable salts of such compounds. The term "pharmaceutically acceptable salts" refers to salts or esters prepared from pharmaceutically acceptable non-toxic bases or acids, including inorganic bases or acids and organic bases or acids, as would be well known to persons skilled in the art. Many suitable inorganic and organic bases are known in the art.

In the context of the present disclosure, it is considered that the composition comprises THC as main cannabinoid, however, the optional presence of other cannabinoids including, without limitation, delta-9-tetrahydrocannabinol (Δ9-THC), delta-8-tetrahydrocannabinol (Δ8-THC), cannabidiol (CBD), cannabidiolic acid (CBDA), cannabichromene (CBC), tetrahydrocannabinolic acid (THCA), tetrahydrocannabivarin (THCV), cannabinol (CBN), cannabigerolic acid (CBGA) and cannabigerol (CBG) is not excluded. Specifically, it will be appreciated that the present composition comprising THC as main cannabinoid may comprise low amounts of CBD, for example, less than 20% CBD by weight of the composition, or less than 15%, 10%, 5% or 2.5% CBD by weight of the composition, or may not comprise any measurable CBD. In the present disclosure, it is preferred that the composition comprises less than 0.5% CBD, or even more preferably less than 0.1% CBD by weight of the composition. The entirety of cannabinoids, i.e. the cannabinoid fraction, typically accounts for the majority of the compounds present in the composition described herein.

In the context of the present disclosed herein, the herein described composition can be employed in a kit as defined herein.

The present invention relates to a pharmaceutical formulation comprising the composition as described herein. In the context of the present invention, the composition is a *Cannabis* plant extract. That is, the formulation of the invention may comprise the composition as described herein, and at least one suitable further substance. In the present invention, it is preferred that the formulation of the invention may comprise the composition as described herein and a suitable carrier oil (or a mixture of such oils) in a suitable amount. Accordingly, the formulation of the invention essentially comprises the same individual components and an identical THC/alpha-bisabolol, THC/guaiol and THC/beta-caryophyllene as well as THC and linalool, alpha-humulene, nerolidol, caryophyllene oxide, alpha-pinene, camphene, beta-pinene, beta-myrcene, limonene, eucalyptol, ocimene, gamma-terpinene, terpinolene, alpha-terpinene, para-cymene, isopulegol or geraniol ratio as the composition of the invention but has been further diluted with a suitable carrier oil (and optionally other additional substances comprised in the formulation). In the context of the present invention, the composition described herein is diluted in a particular ratio such that a specific THC content comprised in the resulting pharmaceutical composition of the invention is set/fixed.

In the present invention, the pharmaceutical formulation of the invention comprises the composition described herein and a suitable carrier oil such that the THC content of the formulation is diluted/adjusted to between about 1%, 1.1%, 1.2%, 1.3%, 1.4%, 1.5%, 1.6%, 1.7%, 1.8%, 1.9%, 2.0%, 2.05%, 2.1%, 2.15%, 2.2%, 2.25%, 2.3%, 2.35%, 2.4%, 2.45% and 2.5% by weight of the formulation. Accordingly, in the context of the present invention, the composition differs from the pharmaceutical formulation by the fact that additional carrier oil is added to the herein described composition. Further, in the context of the present invention, the pharmaceutical formulation of the invention comprises the composition of the invention and a suitable carrier oil such that the THC content of the formulation is diluted/adjusted to below 2.5% by weight of the formulation. In the present invention, it is preferred that the pharmaceutical formulation of the invention comprises the composition of the invention and a suitable carrier oil such that the THC content of the formulation is adjusted to between 1.0% and 2.5%, even more preferably to 2.1% by weight of the formulation. In the present invention, the pharmaceutical formulation of the invention comprises the composition of the invention and the suitable carrier oil such that the alpha-bisabolol content of the composition is adjusted to between about 0.003% and 0.016% by weight of the formulation, the guaiol content of the composition is adjusted to between about 0.01% and 0.023% by weight of the formulation the beta-caryophyllene content of the composition is adjusted to between about 0.01% and 0.024% by weight of the formulation. Further the skilled person will appreciate that certain terpenes from the group of linalool, alpha-humulene, nerolidol, caryophyllene oxide, alpha-pinene, camphene, beta-pinene, beta-myrcene, limonene, eucalyptol, ocimene, gamma-terpinene, terpinolene, alpha-terpinene, para-cymene, isopulegol and geraniol are present in the composition but may become non-detectable because their concentration may be below the identification threshold or detection limit after dilution to provide the pharmaceutical formulation of the invention. Accordingly, the pharmaceutical formulation of the invention comprises the composition of the invention and the suitable carrier oil such that the linalool content of the composition is adjusted to between about 0.0007% and 0.004% by weight of the formulation, the alpha-humulene content of the composition is adjusted to between about 0.001% and 0.007% by weight of the formulation, and/or the nerolidol content of the composition is adjusted to between about 0.001% and 0.009% by weight of the formulation.

In the present invention, it is preferred that the pharmaceutical formulation of the invention comprises the composition described herein and the suitable carrier oil such that the alpha-bisabolol content of the formulation is adjusted to between about 0.006% and 0.014% by weight of the formulation, the guaiol content of the formulation is adjusted to between about 0.01% and 0.019%, the beta-caryophyllene content of the formulation is adjusted to between about 0.01% and 0.02% by weight of the formulation and/or the linalool content of the composition is adjusted to between about 0.001% and 0.003% by weight of the formulation, the alpha-humulene content of the composition is adjusted to between about 0.003% and 0.006% by weight of the formulation, and/or the nerolidol content of the composition is adjusted to between about 0.003% and 0.008% by weight of the formulation.

In a more preferred example, the pharmaceutical formulation of the invention comprises the composition described herein and a carrier oil as described herein (or a mixture of such oils) such that the THC content of the composition is adjusted to between about 1.0% and 2.5% by weight of the composition, the alpha-bisabolol content of the composition is adjusted to between 0.003% and 0.016% by weight of the formulation, the guaiol content of the composition is adjusted to between 0.01% and 0.023% by weight of the formulation, and/or the beta-caryophyllene content of the composition is adjusted to between 0.01% and 0.024% by weight of the formulation, the linalool content of the composition is adjusted to between about 0.0007% and 0.004% by weight of the formulation, the alpha-humulene content of the composition is adjusted to between about 0.001% and 0.007% by weight of the formulation, and/or the nerolidol content of the composition is adjusted to between about 0.001% and 0.009% by weight of the formulation. **In** the present invention, the pharmaceutical composition most preferably comprises the components set out in Table 2 below.

**Table 2: Most preferred amounts of components comprised in the pharmaceutical formulation of the invention.**

| **Component** | **Amount in Formulation [% of weight of the extract]** |
|---|---|
| THC | 2.1 |
| alpha-bisabolol | 0.006 to 0.014 |
| guaiol | 0.010 to 0.019 |
| beta-caryophyllene | 0.010 to 0.020 |
| linalool | 0.001 to 0.003 |
| alpha-humulene | 0.003 to 0.006 |
| nerolidol | 0.003 to 0.008 |

In the present invention, it is preferred that the formulation of the invention comprises/consists of the composition as described herein and a carrier oil as described herein and has a THC/alpha-bisabolol ratio of between 100:1 and 450:1, more preferably of 160:1 and 295:1, a THC/guaiol ratio of between 50:1 and 250:1, more preferably of 105:1 and 200:1, a THC/beta-caryophyllene ratio of between 50:1 and 250:1, more preferably of 105:1 and 200:1, a THC/linalool ratio of between 450:1 and 1400:1, more preferably of 500:1 and 1330:1, a THC/alpha-humulene ratio of between 300:1 and 725:1, more preferably of 355:1 and 665:1, and/or a THC/nerolidol ratio of between 200:1 and 600:1, more preferably of 285:1 and 535:1. Suitable methods to qualitatively and quantitatively determine, analyze, control or set the content of THC, alpha-bisabolol, guaiol, beta-caryophyllene, linalool, alpha-humulene, nerolidol, caryophyllene oxide, alpha-pinene, camphene, beta-pinene, beta-myrcene, limonene, eucalyptol, ocimene, gamma-terpinene, terpinolene, alpha-terpinene, para-cymene, isopulegol and/or geraniol are known in the art and have already been described above and include TLC, HPLC and GC with mass spectrometric detection as described above. The skilled person knows standard procedures how to produce the formulation of the invention by dilution of the composition of the invention, exemplary procedures being described in the following: To produce the pharmaceutical formulation of the present invention comprising 2.1% THC by weight of the formulation, the composition of described herein comprising 5% THC by weight of the composition is further diluted to a content of 2.1% by directly adding the required amount of carrier oil. In another example, 10 g of the composition described herein comprising a THC content of 5% by weight of the composition are weighed, and mixed with 15 g carrier oil, preferably sesame oil (e.g. as comprised in the commercially available Vertanical Production Kit, Vertanical GmbH, catalogue number 15876554), to provide a homogeneous mixture, i.e. the pharmaceutical formulation comprising 2.0% THC by weight of the formulation.

The carrier oil, which provides the formulation of the invention together with the composition as described herein, may constitute any percentage of such formulation provided that such percentage is in conformity with the above presented definitions for the content of THC, alpha-bisabolol, guaiol, beta-caryophyllene and the terpenes selected from linalool, alpha-humulene, nerolidol, caryophyllene oxide, alpha-pinene, camphene, beta-pinene, beta-myrcene, limonene, eucalyptol, ocimene, gamma-terpinene, terpinolene, alpha-terpinene, para-cymene, isopulegol and geraniol in the formulation. Such carrier oil may be a naturally derived oil (e.g. an essential oil) or one or more waxes or any combination thereof as disclosed above in the context of the composition described herein. As such, any carrier oil which may be used or is particularly useful in the context of the composition described herein, is applicable in the context of the formulation of the invention.

In the context of the present invention, the skilled person will understand that, as applicable to the composition described herein, the formulation of the present invention comprises THC as main cannabinoid, but the optional presence of other cannabinoids including, without limitation, cannabidiol (CBD) is not excluded. As such, it is envisaged that other cannabinoids, such as for example cannabidiol (CBD), may be added to the composition of the present invention or the formulation of the present invention in a desired amount.

Both, the composition described herein and the pharmaceutical formulation of the invention may comprise, in addition to the ingredients as described above, further substances such as, without limitation, antioxidants, colorants and flavoring agents in any suitable concentration. Other suitable additives are known to the skilled person and detailed examples are furthermore listed in the continuing detailed description. In case the composition described herein comprises an extract obtained by extraction, the extractant, e.g. solvent as described herein, may be partially or preferably completely removed prior to incorporation of the composition into the pharmaceutical formulation, e.g. by heating the extract under reduced pressure (e.g. under vacuum). The skilled person is aware that some volatile plant metabolites may be removed with the extractant. In the context of the present invention, the extractant may be included in the pharmaceutical formulation.

The terms "composition" and "pharmaceutical formulation" as used herein generally define a composition or formulation suitable for application/administration to the body to treat, care for or improve the appearance of the body. The composition described herein may be applied/administered to a subject which is a patient, preferably a human patient. The pharmaceutical formulation of the invention is specifically intended to be applied/administered to a subject which is a patient, preferably a human patient. As such, the term "pharmaceutical formulation" can be used interchangeably with "medicament". In the present invention, the composition and pharmaceutical formulation as described herein is particularly useful in medicine. Specifically, the composition and pharmaceutical formulation of the present invention may be used in the treatment and/or prevention of conditions/diseases associated with pain, more specifically in the treatment and/or prevention of cancer pain, acute non-cancer pain, chronic non-cancer pain and/or complex pain syndromes. A specific example of cancer pain would be chronic cancer pain. Specific examples of acute non-cancer pain are somatic pain including pain caused by/resulting from tooth extraction, minor cutaneous surgery, skeletal trauma, orthopedic surgery or tension headaches, and visceral pain including pain caused by/resulting from dysmenorrhea, acute pancreatitis or renal/biliary colic. A specific example of chronic non-cancer pain is neuropathic pain such as central neuropathic pain including post-stroke thalamic pain or pain caused by/resulting from spinal cord injury, and peripheral neuropathic pain including pain caused by/resulting from post herpetic neuralgia, diabetic painful neuropathy, trigeminal neuralgia, idiopathic small fiber polyneuropathy or antiretroviral therapy-induced neuropathy. Specific examples of complex pain syndromes include fibromyalgia syndrome, complex regional pain syndrome and migraine.

The references to the methods of treatment by therapy in the following is to be interpreted as references to compounds, pharmaceutical compositions and medicaments of the present invention for use in those methods. The invention also provides a method for treatment and/or prevention of conditions/diseases associated with pain as disclosed herein, the method comprising administering to a patient in need thereof an effective amount of the composition or pharmaceutical formulation described herein. The term "effective amount" it is understood as an amount sufficient that when administered to the patient, the drug is provided to achieve the desired effect. In the case of a therapeutic method, this effect may be the treatment and/or prevention of e.g. cancer pain, acute non-cancer pain, chronic non-cancer pain or complex pain syndromes, more specifically chronic cancer pain, somatic pain, visceral pain, central neuropathic pain, peripheral neuropathic pain and complex pain syndromes, i.e. such that the "effective amount" is considered as a therapeutically effective amount. The "therapeutically effective amount" refers to administration of an amount of a given compound, to a subject in need thereof that achieves the desired therapeutic effect. The term "consecutively" means that each of the composition or pharmaceutical formulation and the other active agent are administered separately and/or at different times. In the present invention it is preferred that the invention provides a method for treatment and/or prevention of cancer pain, acute non-cancer pain, chronic non-cancer pain or complex pain syndromes as disclosed herein, the method comprising administering to a patient in need thereof an effective amount of the composition or pharmaceutical formulation described herein. In the present invention it is even more preferred that the invention provides a method for treatment and/or prevention of chronic cancer pain, somatic pain, visceral pain, central neuropathic pain, peripheral neuropathic pain or complex pain syndromes, the method comprising administering to a patient in need thereof an effective amount of the composition or pharmaceutical formulation described herein. The method disclosed herein may comprise administering more than one composition or formulation of the present invention to the patient in need thereof. The method may also comprise administering an active agent other than the composition or formulation of the present invention. This active agent may be administered simultaneously or consecutively with the composition or formulation. The composition or pharmaceutical formulation as described herein may be administered before or after the other active agent. Further, the route of administration may be the same or different.

As used herein, the terms "treating", "treatment" and the like are understood as affecting a subject, tissue or cell to obtain a desired pharmacological and/or physiological effect in terms of a partial or complete cure of a disease or associated symptoms. The terms "preventing", "prevention" and the like are understood as prophylactic treatment of the subject in terms of completely or partially preventing the occurrence, arresting the development or reducing the severity of a disease or associated symptoms. The term "subject" as used herein refers to a mammal, preferably a human.

The composition and pharmaceutical formulation as disclosed herein, which are particularly useful in medicine as described above, may be administered locally or systematically. They may be administered by any suitable means, including oral, oromucosal (including buccal and sublingual), rectal, intra-nasal, topical (including dermal), vaginal or parenteral (including intramuscular, subcutaneous and intravenous) administration or in a form suitable for administration by inhalation or insufflation. In the present invention, it is preferred that the composition or pharmaceutical formulation as disclosed herein is orally applied. The composition or pharmaceutical formulation as disclosed herein may be in any suitable form including in aerosol, liquid or powder form without limitation, and may be presented in any suitable design such as in liquids, lotions, ampoules, sprays or tablets. Such suitable forms and administration designs are known to the skilled person and are furthermore exemplarily listed in the continuing detailed description without limitation. The skilled person will appreciate that the composition or pharmaceutical formulation as disclosed herein may comprise further ingredients such as acceptable carriers, diluents, adjuvants, excipients and others, or any combination thereof, which depend on the form and route of administration and are exemplarily provided in the continuing detailed description. The skilled person will appreciate that the composition or pharmaceutical formulation of the present invention may be prepared by any means known in the art. It may be employed as pressurised preparations, or as solids or semi-solids such as tablets or filled capsules, or liquids for external or internal use such as solutions, suspensions, emulsions, elixirs, or capsules filled with the same, all for oral use, in the form of suppositories for rectal administration, in the form of aerosols or in the form of sterile injectable solutions for parenteral (including subcutaneous) use. Compositions/formulations for parenteral administration include sterile aqueous or non-aqueous solutions, suspensions and emulsions. Examples of non-aqueous solvents are propylene glycol, polyethylene glycol, vegetable oils such as olive oil, and injectable organic esters such as ethyl oleate. Examples of suitable pharmaceutical carriers are well-known in the art and include both solid and liquid carriers. Solid carriers may be in the form of powders, tablets, pills, capsules, cachets, suppositories, and dispensable granules. A solid carrier can be one or more substances which may also act as diluents, flavouring agents, solubilizes, lubricants, suspending agents, binders, tablet disintegrating agents, or an encapsulating material. Liquid carriers include water, alcoholic/aqueous solutions, emulsions or suspensions, including saline and buffered media, various types of wetting agents, or others. Parenteral vehicles include sodium chloride solution, Ringer's dextrose, dextrose and sodium chloride, lactated Ringer's, or fixed oils. Intravenous vehicles include fluid and nutrient replenishes, electrolyte replenishers (such as those based on Ringer's dextrose) and the like. In addition, the composition or pharmaceutical formulation of the present invention might comprise proteinaceous carriers, like, e.g., serum albumin or immunoglobulins, preferably of human origin and may also comprise, optionally, suitable formulations stabilizers, and/or excipients. Preservatives and other additives may also be present such as, for example, antimicrobials, antioxidants, chelating agents, and inert gases and the like. As described in the foregoing description, the composition as described herein may be mixed with one or more naturally derived oils (e.g. an essential oil) or waxes to provide the pharmaceutical formulation of the present invention.

The composition or pharmaceutical formulation as described herein may be presented in any suitable form including, without limitation, unit dose form in ampoules, pre-filled syringes, small volume infusion, multi-dose containers, droppers, pipettes, sprays, ointments, creams, lotions, or as a transdermal patch. Dosage unit form as used herein refers to physically discrete units suited as unitary dosages for the subjects to be treated. It is also envisaged that the composition or formulation as described herein may be in powder form, obtained by aseptic isolation of sterile solid, for constitution with a suitable vehicle, e.g. sterile, pyrogen-free water, before use. The composition or pharmaceutical formulation as described herein may preferably be orally administered, for example, with an inert diluent or with an assimilable edible carrier, enclosed in hard or soft shell gelatine capsule, compressed into ingestible tablets or buccal tablets, lozenges, troches, capsules, elixirs, suspensions, syrups, wafers, sprays, chewing gums, mouthwashes and the like, or incorporated directly into the food of the diet. Such composition or formulations for oral administration may also contain additional components such as carriers, colorants, stabilizers, buffers, coatings, binders, excipients, disintegrating agents, sweetening agents and/or flavouring agents and the like. Administration to the respiratory tract may also be achieved by means of an aerosol composition or formulation in which the active ingredient is provided in a pressurised pack with a suitable propellant such as a chlorofluorocarbon (CFC), or other suitable gases.

The skilled person will understand that the composition or pharmaceutical formulation as described herein is formulated so as to be suitable for application to a patient, to be compatible with the actives present in the composition/formulation and to not cause any unreasonable safety or toxicity concerns. As such, the composition or pharmaceutical formulation as described herein may be subjected to further treatment, e.g. pH adjustment.

The composition or pharmaceutical formulation described herein can be administered to the subject at any suitable dose. The dosage regimen will be determined by the attending physician and clinical factors. As is well known in the medical arts, dosages for any one patient depend upon many factors, including the patient's size, body surface area, age, the particular compound to be administered, sex, time and route of administration, general health, and other drugs being administered concurrently. As a matter of example, a dosage regimen may be determined based on experience/ sensation of pain and adverse events. The subject, preferably a human patient, may receive 2.5 mg THC by administering a corresponding amount of the composition or pharmaceutical formulation described herein on day 1 in the evening. In case the subject does not experience adverse effects but still experiences pain, the subject may receive the same dose of 2.5 mg THC for further two days in the evening. In case the subject does not experience adverse effects but still experiences pain on day 3, the dose to be administered on day 4 in the evening is increased to a dose of 5 mg THC e.g. by administering a corresponding amount of the composition or pharmaceutical formulation described herein. Further, on day 4, the subject may start to receive an additional dose of 2.5 mg THC in the morning by administering a corresponding amount of the composition or pharmaceutical formulation described herein. The subject may receive the same dose of 5 mg THC in the evening and 2.5 mg in the morning for further two days. In case the subject does not experience adverse effects but still experiences pain on day 6, the dose to be administered on day 7 in the evening is increased to a dose of 7.5 mg THC e.g. by administering a corresponding amount of the composition or pharmaceutical formulation described herein. Further, on day 7, the subject may increase the additional dose in the morning to 5 mg THC by administering a corresponding amount of the composition or pharmaceutical formulation described herein. In case the subject experiences adverse effects, e.g. on day 3, the dose to be administered on day 4 in the evening is decreased to a dose of 2.5 mg THC. Then the subject may receive the same dose of 2.5 mg THC for further two days in the evening. In case the subject does not experience adverse effects and does not experience pain, the dose may be kept constant and is not increased or decreased.

It is envisaged that the composition or formulation of the invention may comprise, in addition to the composition described herein, further biologically active agents depending on the intended use of the composition or pharmaceutical formulation. Any suitable active agent may be used, provided that the activity of the active agents and/or the composition/formulation is not diminished when combined. Exemplarily, such additional active agents may be anti-inflammatory agents such as the commercially available drugs Aspirin^{®}, Ibuprofen, Naproxen, Celecoxib or Diclofenac. Further, the invention envisages the co-administration protocols with other compounds, e.g. commercially available inflammatory agents such as those mentioned above or cotreatment with radiotherapeutic, chemotherapeutic, radionuclide or hormone treatments.

In further embodiments which are present for illustration purposes only but does not form part of the invention, the present disclosure further relates to a kit comprising component A) the composition of the invention and component B) a carrier oil. The carrier oil comprised in the kit as described herein is preferably refined and of pharmaceutical grade and may be provided in a suitable quantity to produce a desired amount of the product when mixed with the composition described herein. Such carrier oil may be a naturally derived oil (e.g. an essential oil) or one or more waxes or any combination thereof. Suitable naturally derived oils and waxes are known to the skilled person and are furthermore described herein. Any carrier oil which may be used or is particularly useful in the context of the composition described herein or the formulation of the invention, is applicable in the context of the kit disclosed herein.

In the context of the disclosure, sesame oil may be used as preferred carrier oil providing component B. In the present disclosure, it may be preferred that component A as described herein comprises THC in an amount of 5% by weight of component A, alpha-bisabolol in an amount of at least 0.017% by weight of component A, preferably of between 0.017% and 0.031% by weight of component A, guaiol in an amount of at least 0.025% by weight of component A, preferably of between 0.025% and 0.046% by weight of component A, beta-caryophyllene in an amount of at least 0.025% by weight of component A, preferably of between 0.025% and 0.047% by weight of component A, and at least one terpene selected from the group consisting of linalool in an amount of at least 0.004% by weight of component A, preferably of between 0.004% and 0.007% by weight of component A, alpha-humulene in an amount of at least 0.008% by weight of component A, preferably of between 0.008% and 0.014% by weight of component A, nerolidol in an amount of at least 0.004% by weight of component A, preferably of between 0.009% and 0.017%, caryophyllene oxide in an amount of at least 0.002% by weight of component A, preferably of between 0.003% and 0.005% by weight of component A, and limonene in an amount of at least 0.001% by weight of component A, preferably of between 0.001% and 0.002% by weight of component A.

In the context of the present disclosure, component A as described herein preferably comprises THC in an amount of 5% by weight of component A, alpha-bisabolol in a THC/alpha-bisabolol ratio of more than 160:1, preferably between 160:1 and 295:1, guaiol in a THC/guaiol ratio of more than 105:1, preferably between 105:1 and 200:1, beta-caryophyllene in a THC/beta-caryophyllene ratio of more than 105:1, preferably between 105:1 and 200:1 and at least one terpene selected from the group consisting of linalool in a THC/linalool ratio of between 450:1 and 1400:1, more preferably of 500:1 and 1330:1, alpha-humulene in a THC/alpha-humulene ratio of between 300:1 and 725:1, more preferably of 355:1 and 665:1, nerolidol in a THC/nerolidol ratio of between 200:1 and 600:1, more preferably of 285:1 and 535:1, caryophyllene oxide in a THC/caryophyllene oxide ratio of between 1000:1 and 2150:1, preferably between 1090:1 and 2080:1, and limonene in a THC/limonene ratio of between 1650:1 and 5050:1, preferably between 1700:1 and 4970:1.

The kit as described herein further comprises instructions for use of the kit. Specifically, the kit instructs the user to combine component A and component B in a suitable ratio. In the context of the present disclosure, a suitable dilution ratio would be a component A/component B ratio of between 1:1 and 1:1.5. More preferably, the kit instructs the user to combine component A and component B in a component A/component B ratio of 1:1.38. In the context of the present disclosure, the kit instructs the user to combine component A and component B in such way, that the product resulting from combining component A and component B comprises between 1.0% and 2.5% THC. Preferably, the kit instructs the user to combine component A and component B in such way, that the product resulting from combining component A and component B comprises between 1.0% and 2.5% THC, alpha-bisabolol in a THC/alpha-bisabolol ratio of between 100:1 and 450:1, preferably 160:1 and 295:1, guaiol in a THC/guaiol ratio of between 50:1 and 250:1, preferably 105:1 and 200:1 and/or beta-caryophyllene in a THC/beta-caryophyllene ratio of between 50:1 and 250:1, preferably 105:1 and 200:1 and/or at least one terpene selected from the group consisting of linalool, alpha-humulene and nerolidol in a THC/linalool ratio of between 450:1 and 1400:1, more preferably of 500:1 and 1330:1, a THC/alpha-humulene ratio of between 300:1 and 725:1, more preferably of 355:1 and 665:1, and a THC/nerolidol ratio of between 200:1 and 600:1, more preferably of 285:1 and 535:1. More preferably, the kit instructs the user to combine component A and component B in such way, that the product resulting from combining component A and component B comprises 2.1% THC, alpha-bisabolol in a THC/alpha-bisabolol ratio of more than 160:1, guaiol in a THC/guaiol ratio of more than 105:1, beta-caryophyllene in a THC/beta-caryophyllene ratio of more than 105:1 and/or at least one terpene selected from the group consisting of linalool, alpha-humulene and nerolidol in a THC/linalool ratio of more than 715:1, a THC/alpha-humulene ratio of more than 355:1 and a THC/nerolidol ratio of more than 285:1.

Most preferably, in the context of the present disclosure, the kit instructs the user to combine component A and component B in such way, that the product resulting from combining component A and component B comprises 2.1% THC, alpha-bisabolol in a THC/alpha-bisabolol ratio of between 160:1 and 295:1, guaiol in a THC/guaiol ratio of between 105:1 and 200:1, beta-caryophyllene in a THC/beta-caryophyllene ratio of between 105:1 and 200:1 and/or at least one terpene selected from the group consisting of linalool, alpha-humulene and nerolidol in a THC/linalool ratio of 500:1 and 1330:1, a THC/alpha-humulene ratio of between 355:1 and 665:1 and a THC/nerolidol ratio of between 285:1 and 535:1.

Correctly following the instructions for use of the kit, the resulting product provides the pharmaceutical composition of the invention. As such, the kit of the described herein can be employed in a variety of applications referred to in the context of the composition and/or pharmaceutical formulation as disclosed herein. Specifically, the kit as described herein is particular useful in medicine. Furthermore, the kit as described herein is particular useful for the treatment and/or prevention of conditions/diseases associated with pain as described herein above. Furthermore, the kit as described herein may be advantageously used, inter alia, for carrying out the method for treatment and/or prevention of conditions/diseases associated with pain as disclosed herein.

The kit may further comprise additional components for convenience of the user, such as a suitable container, preferably an amber glass flask, suitable dropper inserts/dropper inserts which allow more exact dosing and/or closing caps/sealing caps which may be childproof for safety reasons. Such additional components are preferably of pharmaceutical grade and/or sterile. Furthermore, parts of the kit of the invention can be packaged individually in vials or bottles or in combination in containers or multicontainer units.

In the context of the present disclosure, the skilled person will understand that, as applicable to the composition described herein and the pharmaceutical formulation of the present invention, THC is comprised as main cannabinoid, but the optional presence of other cannabinoids including, without limitation, cannabidiol (CBD) is not excluded. As such, it is envisaged that the kit may further comprise a further cannabinoid preparation, e.g. an alcoholic solution comprising a cannabinoid other than THC as main cannabinoid, CBD being mentioned as preferred example (e.g. as commercially provided by 1x Cannabis Vollextrakt VERTANICAL CBD 50 solution, Vertanical GmbH, catalogue number: 16234579). The kit may then instruct the user to combine component A, component B and the additional cannabinoid preparation into one product.

In the foregoing detailed description, a number of individual elements, characterizing features, techniques and/or steps are disclosed. It is readily recognized that each of these has benefit not only individually when considered or used alone, but also when considered and used in combination with one another. Accordingly, to avoid exceedingly repetitious and redundant passages, this description has refrained from reiterating every possible combination and permutation. Nevertheless, whether expressly recited or not, it is understood that such combinations are entirely within the scope of the presently disclosed subject matter.

All technical and scientific terms used herein, unless otherwise defined, are intended to have the same meaning as commonly understood by one of ordinary skill in the art. Reference to techniques employed herein are intended to refer to the techniques as commonly understood in the art, including variations on those techniques or substitutions of equivalent techniques that would be apparent to one of skill in the art.

The present invention is further described by reference to the following non-limiting figures and examples.

The Figures show:
**Figure 1****: Exemplary IC50 titration curve obtained with an HCT-116 cell line exposed to *Cannabis* plant extract** as described in Example 1. The concentration on the x-axis was normalized to THC content in the *Cannabis* plant extract as a reference for the entire mixture. Measured average values are shown as circles.
**Figure 2****: IC50 values in MCF7 cell line of single substances when compared to a mixture of those.** Figure 2 shows the comparison of single IC50 concentrations measured in a MCF7 cell line in µg/ml for THC and ng/ml for alpha-bisabolol and guaiol as single substances and the measured and derived individual IC50 concentrations in respective combination of THC, alpha-bisabolol and guaiol.

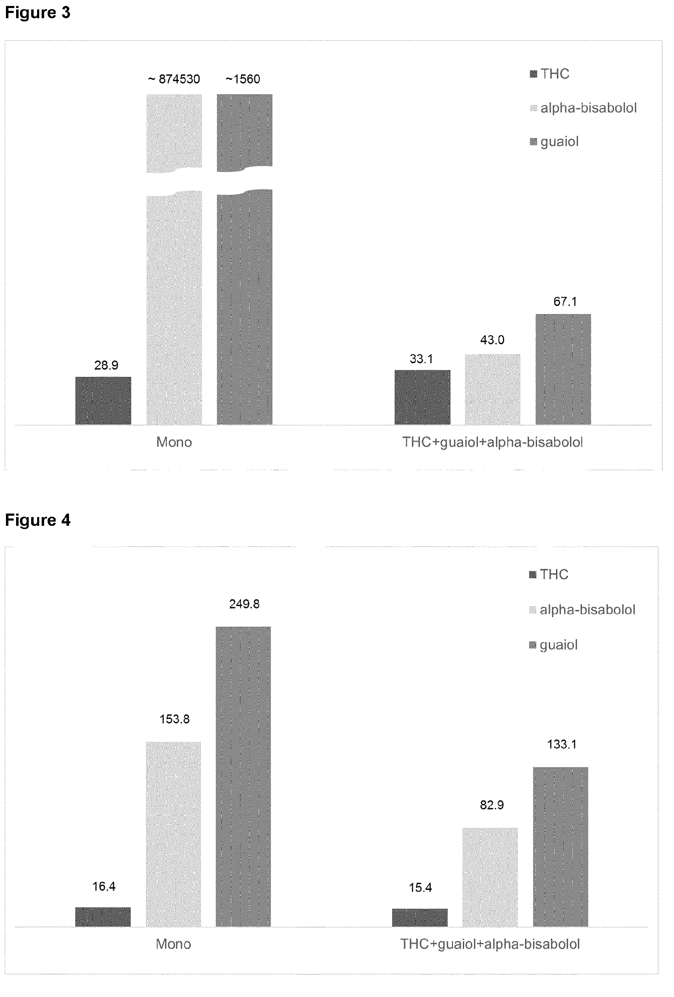

**Figure 3****: IC50 values in HCT-116 cell line of single substances when compared to a mixture of those.** Figure 3 shows the comparison of single IC50 concentrations measured in HTC 116 cell line in µg/ml for THC and ng/ml for alpha-bisabolol and guaiol as single substances and the measured and derived individual IC50 concentrations in respective combination of THC, alpha-bisabolol and guaiol.
**Figure 4****: IC50 values in CaCO2 cell line of single substances when compared to a mixture of those.** Figure 4 shows the comparison of single IC50 concentrations measured in CaCO2 cell line in µg/ml for THC and ng/ml for alpha-bisabolol and guaiol as single substances and the measured and derived individual IC50 concentrations in respective combination of THC, alpha-bisabolol and guaiol.

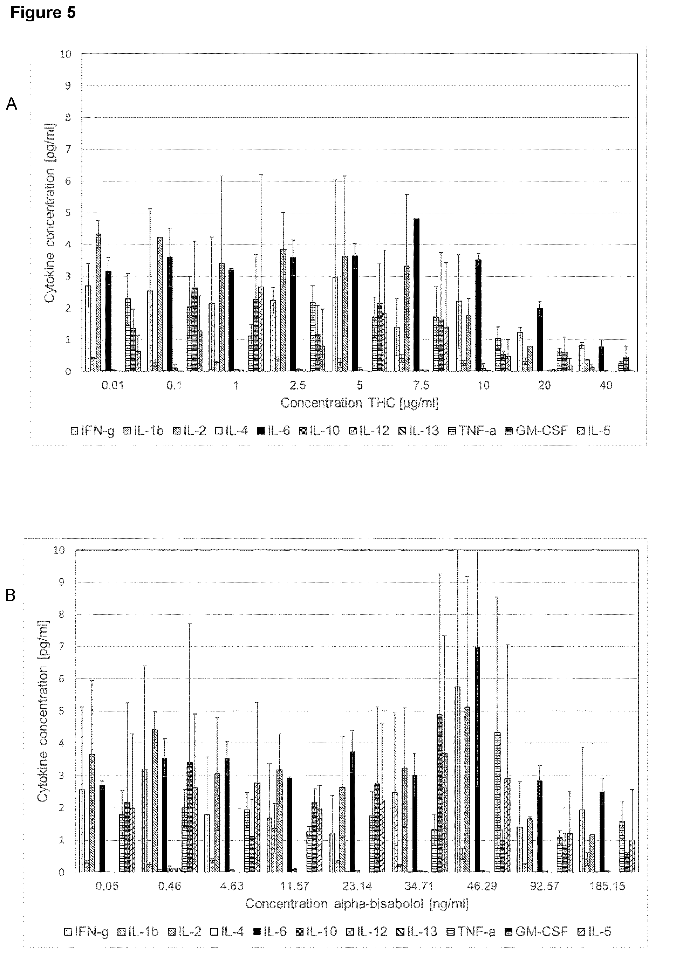

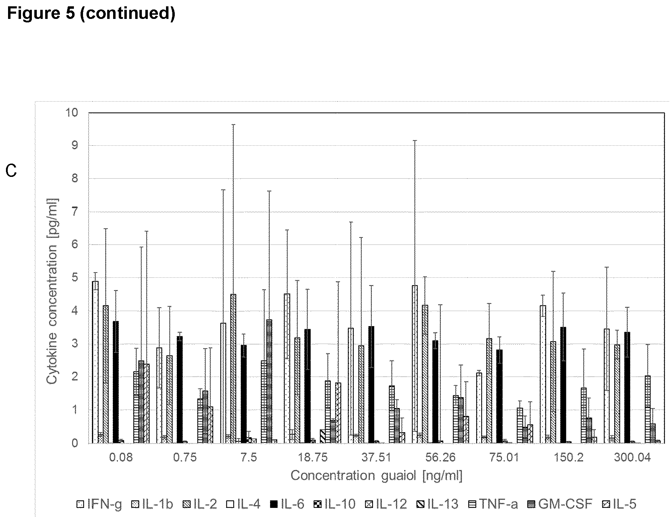

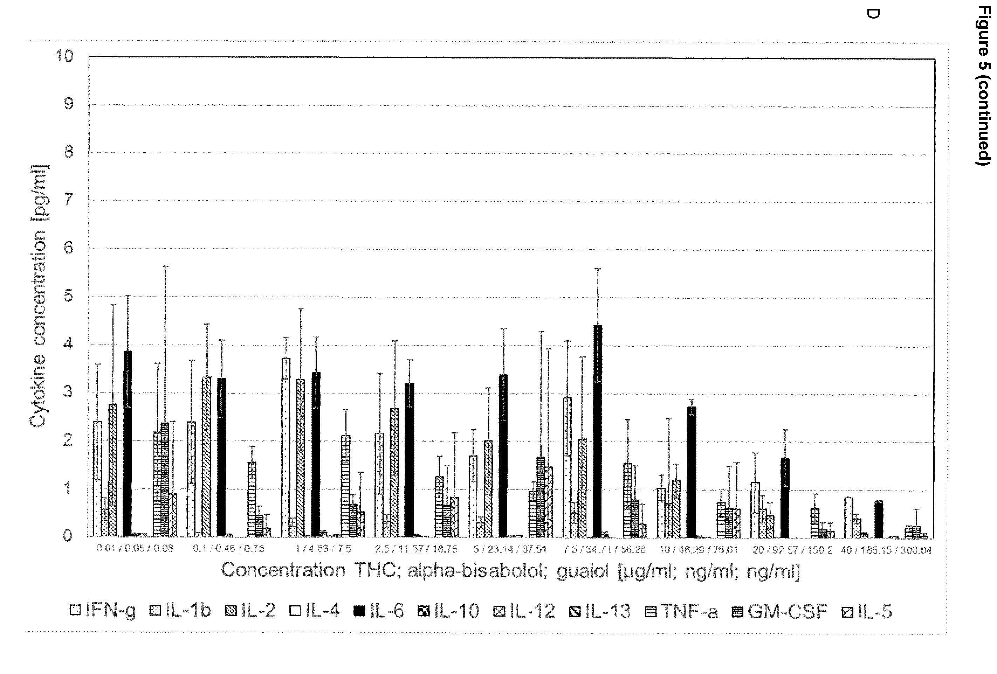

**Figure 5****: Inflammatory cytokine secretion of PBMC in dependence on single substances when compared to a mixture of those without PHA-stimulation.** Figure 5 shows the measured respective concentrations of a variety of cytokines depicted in the Figure at different concentrations of THC (A), alpha-bisabolol (B), guaiol (C) and a mixture of THC, alpha-bisabolol and guaiol (D) in non-PHA-stimulated control PBMCs.

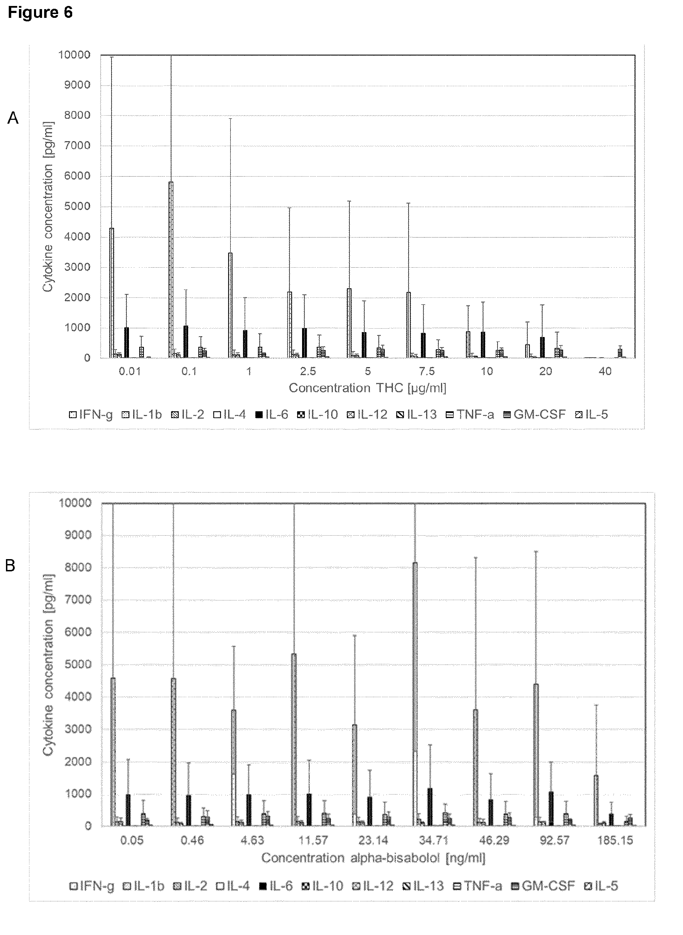

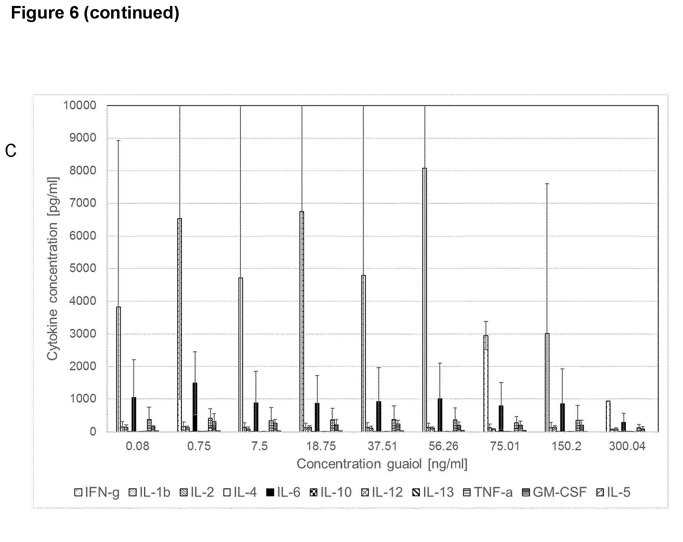

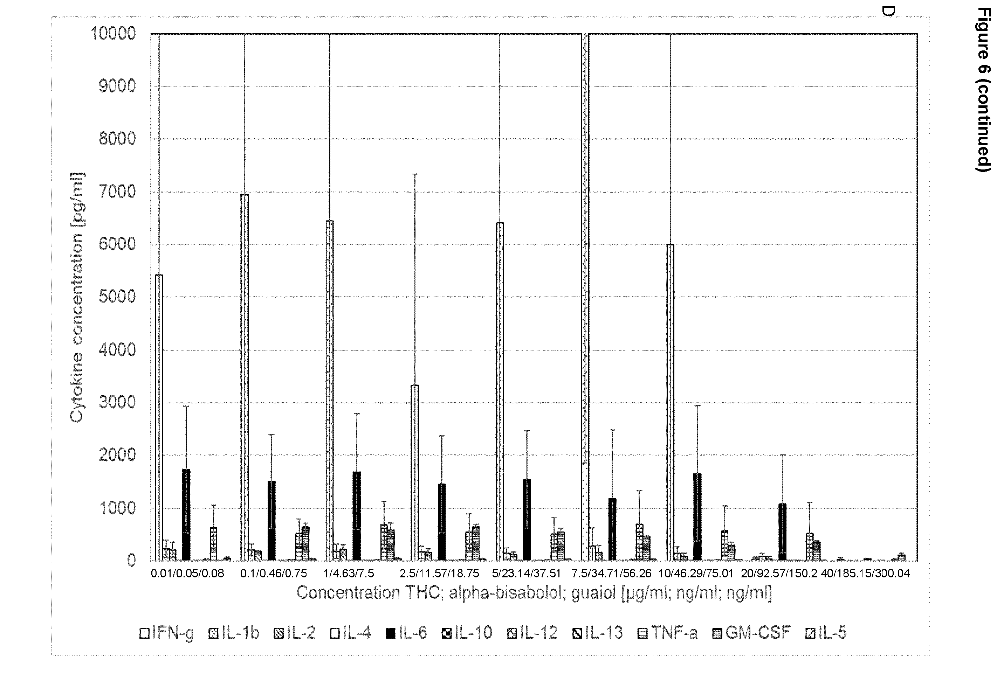

**Figure 6****: PHA-induced inflammatory cytokine secretion in dependence on single substances when compared to a mixture of those.** Figure 6 shows the measured respective concentrations of a variety of cytokines depicted in the Figure at different concentrations of THC (A), alpha-bisabolol (B), guaiol (C) and a mixture of THC, alpha-bisabolol and guaiol (D) in PHA-stimulated PBMCs.

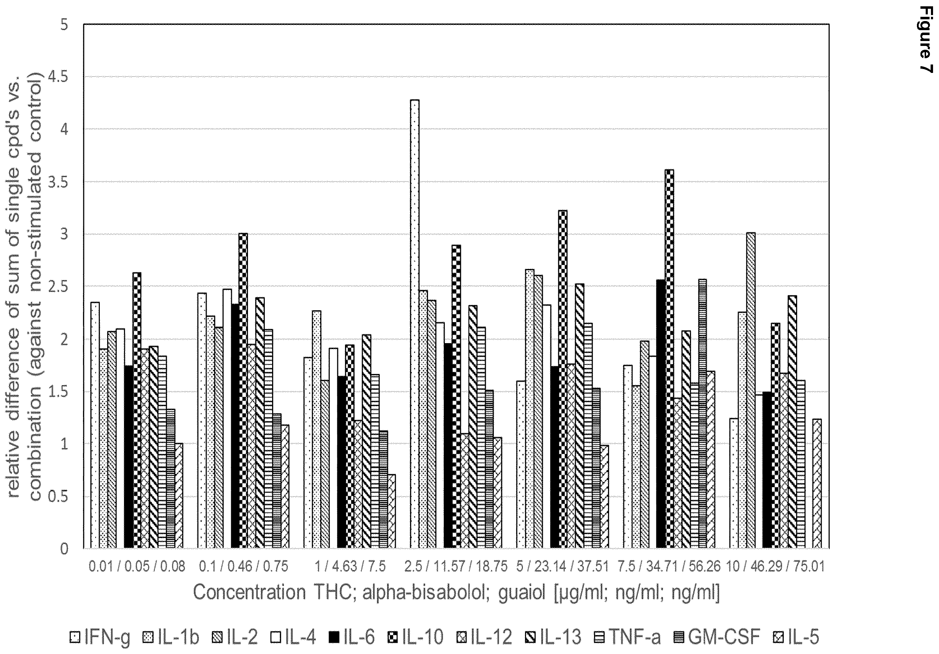

**Figure 7****: Cytokine secretion when THC, alpha-bisabolol and guaiol are administered as single substances relative to a mixture of those.** Figure 7 shows the calculated concentration-dependent relative differences in secretion of a variety of cytokines depicted in the Figure by PBMCs between the administration of THC, alpha-bisabolol and guaiol as single substances relative to a mixture of THC, alpha-bisabolol and guaiol. Calculation of the relative values is based on the experimental data shown in Figures 5 and 6, whereby PHA-induced inflammatory cytokine secretion has been normalized to non-PHA-stimulated control values.

The following Examples illustrate the invention:

### Example 1 Cell viability of stimulated cancer cell lines

The following example demonstrates that cytotoxic activity of the exemplary terpenes alpha-bisabolol and guaiol is enhanced in a combinatory treatment with THC when compared to a treatment with alpha-bisabolol or guaiol alone.

### 1.1 Cell line culture

The following cell lines were used for the experiment: CaCO2 (Pharmacelsus), HCT-116 (ATCC, Cat# CCL-247), and MCF-7 (ECACC, Cat# 86012803). All cell lines were maintained at 37°C, 5% CO₂ and 95% humidity. The medium was replaced three times per week and the monolayer was sub-cultured in a ratio of 1:5 when the culture reaches a confluence of 80%. The specific cultivation medium for each cell line was as follows: CaCO2 cell line was cultured in DMEM (PAN-Biotech, Cat# P04-03500) supplemented with 10% FBS Good (PAN-Biotech, Cat# P40-37500) and 1% NEAA (PAN-Biotech, Cat# P08-32100), HCT-116 cell line was cultured in McCoy's A5 medium (PAN-Biotech, Cat# P04-05500) supplemented with 10% FBS Good, and MCF-7 cell line was cultured in RPMI 1640 (PAN-Biotech, Cat# P04-16500) supplemented with 10% FBS and 2 mM GlutaMAX (Gibco, Cat# 35050). All cell culture media were routinely supplemented with 1% Penicillin/Streptomycin (PAN-Biotech, Cat# P06-07100).

For the cytotoxicity assay, cells were seeded into 96-well flat bottom microtiter plates at a density of 10,000 cells/well and allowed to attach to the plate for 24 hours before the start of the experiment.

### 1.2 Cytotoxicity assay

### 1.2.1 Preparation of test substances

Dronabinol (produced by Pharma Wernigerode GmbH; THC content: 95.37%), *Cannabis* plant extract as specified in Table 3 (produced by Pharma Wernigerode GmbH; obtainable by the process as described in patent application EP22154007.3 (PCT/EP2023/052073)), alpha-bisabolol (Sigma, Cat# 14462) and guaiol (Cayman Chemicals, Cat# 23172) prepared in dimethylsulfoxide (DMSO, Carl Roth, Cat# 4720.2) were diluted in cell culture medium to obtain the final concentrations for the different cell lines as shown in Tables 4 to 6. Each concentration for assayed single substances and the indicated combination of substances was tested in technical triplicates.

As negative control, cell culture medium was used containing the same amount of the respective solvent that is also present in the samples (0.5% DMSO) to make sure that toxic observations resulted from the test items and not from the solvent. As positive control, 1% Triton X-100 (Alfa Aesar, Cat# A16046) was added to cell culture medium. Medium only was used as blank.

**Table 3: Characteristics of the Cannabis plant extract/Cannabis soft extract as obtainable by a Cannabis plant as deposited by the Community Plant Variety Office with the application number A202104053 after evaporation and decarboxylation or as obtainable by the process as described in patent application EP22154007.3 (PCT/EP2023/052073).**

| **Component** | **Amount in plant extract [% of weight of the extract]** |
|---|---|
| THC | 70.8% to 73.8% |
| CBD | 0.133% to 0.247% |
| alpha-bisabolol | 0.21% to 0.39% |
| guaiol | 0.32% to 0.59% |
| beta-caryophyllene | 0.35% to 0.65% |
| nerolidol | 0.104% to 0.179% |
| alpha-humulen | 0.128% to 0.221% |
| linalool | 0.081% to 0.140% |
| caryophyllene oxid | 0.025% to 0.044% |
| limonene | 0.020% to 0.034% |
| beta-myrcen | 0.014% to 0.024% |
| ocimen | 0.011% to 0.019% |
| alpha-pinen | 0.005% to 0.009% |
| beta-pinen | 0.004% to 0.008% |
| eucalyptol | 0.004% to 0.008% |
| gamma-terpinen | 0.005% to 0.008% |
| terpinolen | 0.004% to 0.008% |
| alpha-terpinen | 0.004% to 0.008% |
| camphen | 0.003% to 0.005% |
| p-cymen | 0.006% to 0.010% |
| isopulegol | 0.010% to 0.017% |
| geraniol | 0.022% to 0.038% |

### 1.2.2 Assayed concentrations of test substances

**Table 4: Assayed concentrations of test substances for MCF-7 cell line.**

| **Description** | **Concentrations (3 replicates per stimulus)** | | | | | | | | |
|---|---|---|---|---|---|---|---|---|---|
| Cannabis Soft Extract [µg/ml THC] | 0.01 | 0.1 | 1 | 2.5 | 5 | 7.5 | 10 | 20 | 40 |
| Dronabinol [µg/ml THC] | 0.01 | 0.1 | 1 | 2.5 | 5 | 7.5 | 10 | 20 | 40 |
| alpha-bisabolol [ng/ml] | 0.05 | 0.46 | 4.63 | 11.57 | 23.14 | 34.71 | 46.29 | 92.57 | 185.15 |
| Guaiol [ng/ml] | 0.08 | 0.75 | 7.50 | 18.75 | 37.51 | 56.26 | 75.01 | 150.02 | 300.04 |
| THC [µg/ml] | 0.01 | 0.1 | 1 | 2.5 | 5 | 7.5 | 10 | 20 | 40 |
| + alpha-bisabolol [ng/ml] | 0.05 | 0.46 | 4.63 | 11.57 | 23.14 | 34.71 | 46.29 | 92.57 | 185.15 |
| + guaiol [ng/ml] | 0.08 | 0.75 | 7.50 | 18.75 | 37.51 | 56.26 | 75.01 | 150.02 | 300.04 |

**Table 5: Assayed concentrations of test substances for HCT-116 cell line.**

| **Description** | **Concentrations (3 replicates per stimulus)** | | | | | | | | |
|---|---|---|---|---|---|---|---|---|---|
| Cannabis Soft Extract [µg/ml THC] | 5 | 10 | 15 | 20 | 25 | 30 | 35 | 40 | 50 |
| Dronabinol [µg/ml THC] | 5 | 10 | 15 | 20 | 25 | 30 | 35 | 40 | 50 |
| alpha-bisabolol [ng/ml] | 23.1 | 46.3 | 69.4 | 92.6 | 115.7 | 138.9 | 162.0 | 185.1 | 231.4 |
| Guaiol [ng/ml] | 37.5 | 75.0 | 112.5 | 150.0 | 187.5 | 225.0 | 262.5 | 300.0 | 375.1 |
| THC [µg/ml] | 5 | 10 | 15 | 20 | 25 | 30 | 35 | 40 | 50 |
| + alpha-bisabolol [ng/ml] | 23.1 | 46.3 | 69.4 | 92.6 | 115.7 | 138.9 | 162.0 | 185.1 | 231.4 |
| + guaiol [ng/ml] | 37.5 | 75.0 | 112.5 | 150.0 | 187.5 | 225.0 | 262.5 | 300.0 | 375.1 |

**Table 6: Assayed concentrations of test substances for CaCO2 cell line.**

| **Description** | **Concentrations (3 replicates per stimulus)** | | | | | | | | |
|---|---|---|---|---|---|---|---|---|---|
| Cannabis Soft Extract [µg/ml THC] | 1 | 2 | 3 | 4 | 6 | 8 | 12 | 16 | 32 |
| Dronabinol [µg/ml THC] | 1 | 2 | 3 | 4 | 6 | 8 | 12 | 16 | 32 |
| alpha-bisabolol [ng/ml] | 4.6 | 9.3 | 13.9 | 18.5 | 27.8 | 37.0 | 55.5 | 74.1 | 148.1 |
| Guaiol [ng/ml] | 7.5 | 15.0 | 22.5 | 30.0 | 45.0 | 60.0 | 90.0 | 120.0 | 240.0 |
| THC [µg/ml] | 1 | 2 | 3 | 4 | 6 | 8 | 12 | 16 | 32 |
| + alpha-bisabolol [ng/ml] | 4.6 | 9.3 | 13.9 | 18.5 | 27.8 | 37.0 | 55.5 | 74.1 | 148.1 |
| + guaiol [ng/ml] | 7.5 | 15.0 | 22.5 | 30.0 | 45.0 | 60.0 | 90.0 | 120.0 | 240.0 |

### 1.2.3 Assay procedure

Quantification of viable cells was performed by using commercial MTT assay kit (Cell Proliferation Kit I (MTT), Sigma, Cat# 11465007001). The MTT assay is based on the cleavage of the yellow tetrazolium salt MTT to purple formazan crystals by metabolic active cells. The formazan crystals formed are solubilized and the resulting coloured solution is quantified.

Cells were prepared as described in Example 1.1. Cell culture medium was completely removed from the cells by vacuum aspiration and 200 µl of test item solutions was added to the cells. Cells were exposed to test substances for 48 h at 37°C, 5% CO₂ and 95% humidity. After the incubation time the supernatant was discarded and cells were washed with PBS (PAN, Cat# P04-36500). Fresh cell culture medium with 10% MTT reagent was added to each well. The cells will be further incubated for 4 h at 37°C, 5% CO₂ and 95% humidity. After the incubation time 200 µl of solubilization solution was added to each well and cells were further incubated over night at 37°C, 5% CO₂ and 95% humidity. Conversion of MTT to formazan was recorded by measuring the absorbance at 570 nm with reference wavelength 650 nm by using a spectrophotometer (Spectramax Plus, Molecular Devices).

### 1.3 Results

The results shown in Figures 2 to 4 are derived from primary data that were obtained pursuant to Example 1. To arrive at the specific data shown, each measurement taken in triplicate was averaged and the average relative viabilities are set into relation to the (for the single substances) concentration of respective compound applied.

As a matter of Example, Figure 1 shows the average relative viability of an HCT-116 cell line exposed to a *Cannabis* plant extract, whereas - due to the extract being a mixture of a broad variety of compounds - only the concentration of THC is shown on the x-axis, as the relative fraction of the other compounds varies along with that concentration. Measured average values are shown as circles and the dashed line represents the theoretically ideal curve of an IC50 titration. Said curve follows the equation: $relative viability = {e}^{∧} \left(B-A⋅c\right) / \left(1+{e}^{∧}\left(B-A⋅c\right)\right) .$

As can be readily seen, it depends on the values of "A" and "B" in the above equation to have a specific relative viability at any given concentration "c" of a compound. To retrieve such values "A" and "B", the measured relative viabilities at known concentrations were used in a numerical approximation to fit the equation to the measured values. To achieve this, the Microsoft^{®}Excel Solver algorithm was used, seeking to minimize the standard deviation between each numerically (by the above equation) calculated relative viability and the respective experimentally determined relative viability at known concentration. The Solver algorithm was allowed to modulate the values A and B using the GRG nonlinear algorithm module in seeking a minimum for the sum of all of the above referred to standard deviations at known concentrations.

With "A" and "B" determined the above equation provides with a continuous way of setting relative viability and concentration in correlation. By then it was numerically determined which value of "c" must be placed into the above equation to achieve a relative viability of 50%. The result thereof, which is the value determined for "c," is IC50. As a matter of example, the IC50 value read from Figure 1 is approximately 29 µg/ml THC.

As evident from Figures 4 to 6 presenting IC50 values for different cancer cell lines, IC50 values of alpha-bisabolol and guaiol are remarkably reduced in combinatory treatment with THC when compared to a treatment with alpha-bisabolol or guaiol alone. IC50 values of THC remain unchanged in single treatment and combinatory treatment with alpha-bisabolol and guaiol. Thus, the cytotoxic activity of the terpenes alpha-bisabolol and guaiol is enhanced by the presence of THC when the components are present in a THC/alpha-bisabolol ratio of between 100:1 and 450:1, specifically between 160:1 and 295:1 and/or a THC/guaiol ratio of between 50:1 and 250:1, specifically between 105:1 and 200:1. The *Cannabis* plant extract of the present invention comprises THC and the terpenes alpha-bisabolol and guaiol in the ratio as tested individually in the Examples (see Table 3). Thus, it is believed that a composition and pharmaceutical formulation of the invention which has the same individual effective components and THC/terpene ratio as the *Cannabis* extract disclosed in the present Example are plausible to be particularly effective in providing cytotoxic activity. Accordingly, the cytotoxic activity of alpha-bisabolol, guaiol, beta-caryophyllene and at least one terpene selected from the group consisting of linalool, alpha-humulene, nerolidol, caryophyllene oxide, alpha-pinene, camphene, beta-pinene, beta-myrcene, limonene, eucalyptol, ocimene, gamma-terpinene, terpinolene, alpha-terpinene, para-cymene, isopulegol and geraniol is plausible to be enhanced by the presence of THC. These results render plausible that the composition and the formulation of the invention have the superior effect of having increased terpene-associated cytotoxic activity.

### Example 2 Cytokine secretion of stimulated PBMCs

The following example demonstrates that anti-inflammatory activity is enhanced when THC, and the exemplary terpenes alpha-bisabolol and guaiol are administered in a combinatory treatment when compared to a treatment with either THC, alpha-bisabolol or guaiol alone.

### 2.1 PBMC culture

PBMCs of four different healthy donors of Caucasian ethnicity without any known diagnosis of autoimmune diseases were commercially obtained (Immunospot) and used in the experiment: PBMCs No. 1 (male, 24 years old, CTL, # HHU20210720, PBMCs No. 2 (female, 24 years old, CTL, # HHU20210722), PBMCs No. 3 (female, 44 years old, CTL, # HHU20210727) and PBMCs No. 4 (male, 20 years old, CTL, # HHU20210729). Cells of all four donors were pooled for the experiment.

PBMCs were cultured in RPMI 1640 (PAN-Biotech, Cat# P04-16500) supplemented with 10% FBS. For the assays, pooled PBMCs were seeded at a concentration of 200.000 cells/well on 96 well plates and were immediately used for the assay. The test substances (as prepared in 2.2) were added, and after 30 min pre-incubation, 10 µg/ml (final) phytohemagglutinin L (PHA) (Roche, Cat# 11249738001) as pro-inflammatory stimulus or medium as control were added. After 48 h the plates were centrifugated and the supernatant was collected for the cytokine measurement, the remaining cells were used for the cell viability test.

### 2.2 Preparation of test substances

Dronabinol (described in Example 1.2.1), *Cannabis* plant extract as defined in Example 1 (Table 3), alpha-bisabolol (Sigma, Cat# 14462) and guaiol (Cayman Chemicals, Cat# 23172) prepared in dimethylsulfoxide (DMSO, Carl Roth, Cat# 4720.2) were diluted in cell culture medium to obtain the final concentrations as shown in Tables 7 and 8. Each concentration for assayed single substances and the indicated combination of substances was tested in technical triplicates.

As negative control, PBMCs were cultured in culture medium containing the same amount of the respective solvent that is also present in the samples (0.4% DMSO) to make sure that toxic observations resulted from the test items and not from the solvent. As positive control, 10 µg/ml PHA was added to PBMCs in cell culture medium. Medium only was used as blank.

### 2.2.1 Assayed concentrations of test substances

**Table 7: Assayed concentrations of test substances with PHA as pro-inflammatory stimulus.**

| **Description** | **Concentrations (3 replicates per stimulus)** | | | | | | | | |
|---|---|---|---|---|---|---|---|---|---|
| Cannabis Soft Extract [µg/ml THC] | 0.01 | 0.1 | 1 | 2.5 | 5 | 7.5 | 10 | 20 | 40 |
| + PHA [µg/ml] | 10 | 10 | 10 | 10 | 10 | 10 | 10 | 10 | 10 |
| Dronabinol [µg/ml THC] | 0.01 | 0.1 | 1 | 2.5 | 5 | 7.5 | 10 | 20 | 40 |
| + PHA [µg/ml] | 10 | 10 | 10 | 10 | 10 | 10 | 10 | 10 | 10 |
| alpha-bisabolol [ng/ml] | 0.05 | 0.46 | 4.63 | 11.57 | 23.14 | 34.71 | 46.29 | 92.57 | 185.15 |
| + PHA [µg/ml] | 10 | 10 | 10 | 10 | 10 | 10 | 10 | 10 | 10 |
| Guaiol [ng/ml] | 0.08 | 0.75 | 7.50 | 18.75 | 37.51 | 56.26 | 75.01 | 150.02 | 300.04 |
| + PHA [µg/ml] | 10 | 10 | 10 | 10 | 10 | 10 | 10 | 10 | 10 |
| THC [µg/ml] | 0.01 | 0.1 | 1 | 2.5 | 5 | 7.5 | 10 | 20 | 40 |
| + alpha-bisabolol [ng/ml] | 0.05 | 0.46 | 4.63 | 11.57 | 23.14 | 34.71 | 46.29 | 92.57 | 185.15 |
| + guaiol [ng/ml] | 0.08 | 0.75 | 7.50 | 18.75 | 37.51 | 56.26 | 75.01 | 150.02 | 300.04 |
| + PHA [µg/ml] | 10 | 10 | 10 | 10 | 10 | 10 | 10 | 10 | 10 |

**Table 8: Assayed concentrations of test substances without PHA as stimulus.**

| **Description** | **Concentrations (3 replicates per stimulus)** | | | | | | | | |
|---|---|---|---|---|---|---|---|---|---|
| Cannabis Soft Extract [µg/ml THC] | 0.01 | 0.1 | 1 | 2.5 | 5 | 7.5 | 10 | 20 | 40 |
| Dronabinol [µg/ml THC] | 0.01 | 0.1 | 1 | 2.5 | 5 | 7.5 | 10 | 20 | 40 |
| alpha-bisabolol [ng/ml] | 0.05 | 0.46 | 4.63 | 11.57 | 23.14 | 34.71 | 46.29 | 92.57 | 185.15 |
| Guaiol [ng/ml] | 0.08 | 0.75 | 7.50 | 18.75 | 37.51 | 56.26 | 75.01 | 150.02 | 300.04 |
| THC [µg/ml] | 0.01 | 0.1 | 1 | 2.5 | 5 | 7.5 | 10 | 20 | 40 |
| + alpha-bisabolol [ng/ml] | 0.05 | 0.46 | 4.63 | 11.57 | 23.14 | 34.71 | 46.29 | 92.57 | 185.15 |
| + guaiol [ng/ml] | 0.08 | 0.75 | 7.50 | 18.75 | 37.51 | 56.26 | 75.01 | 150.02 | 300.04 |

### 2.3 Assay procedures

### 2.3.1 Quantification of cytokines

Quantification of the cytokines IFN-γ, IL-1β, IL-2, IL-4, IL-6, IL-10, IL-13, IL-12p70, TNF-α, IL-5 and GM-CSF was performed by using V-Plex MSD kit (MSD, Cat# K151A9H-2 ) and MESO QuickPlex SQ 120MM system (Meso Scale Discovery). The human PBMC cells (200.000 cells) were mixed with the test items (except PHA) in the concentrations and combinations as indicated in Tables 5 and 6, and were incubated for 30 min at 37°C. Then, PHA or the respective volume of medium as control was added and incubated for 48 h. Afterwards, 200 µl of supernatant was collected and frozen. 30 µl or less (depending on the dilution) of the supernatant was used for the cytokine quantification. Dilution of the collected supernatant might be required for correct cytokine measurement. The correct dilution will be established in the two test measurements of the cell supernatants with and without PHA stimulation.

For the measurements, samples, calibrators (part of the V-Plex MSD kit) and controls were transferred to a special assay plate covered with capture antibodies that are able to bind the cytokines of interest from the samples. After incubating the plate for 2 h at room temperature, the detection antibodies were added and the plate was incubated for another 2 h at room temperature. Afterwards, the test plate was washed and reading buffer was added. The detection antibodies contain a special ruthenium tag (SULFO-TAG^{™}) and the reading buffer contains Tripropylamin (TPrA). Tripropylamine (TPrA) is oxidized at the electrode and generates the radical cation (TprA°⁺), which is quickly (half-life ~ 200 µs) deprotonated and forms the radical (TprA°). The radical and the radical cation react with the electrochemiluminescence phosphor (Ru(bpy)₃²⁺). The luminescent material in the excited state Ru^{2+ *} relaxes to the ground state and emits photons at 620 nm. The QuickPlex SQ 120MM (Meso Scale Discovery) uses a CCD camera to detect the light emission and to obtain images of the plate during reading. The obtained results were quantified using special software (MSD Discovery workbench) and calibration standards.

### 2.3.2 Viability testing

CellTiter-Glo^{®} Luminescent Cell Viability Assay was used to quantify viable cells (Cell Viability Kit I, Promega, Cat# G7570). The test is based on quantification of the ATP present, which signals the amount of the metabolically active cells. The single reagent containing luciferin, which is converted into oxyluciferin in the presence of ATP and Mg²⁺, was be added to the cells, mixed for 2 min, stabilised for additional 10 min and afterwards the luminescence was measured by using Wallac 1420 Multilabel Counter (Perkin Elmer GmbH).

### 2.3.3 Data analysis

For the dose-response relationship, absolute absorption (OD treated wells - background) were related to the negative (medium) control and relative viability values were plotted against the test item concentrations.

### 2.4 Results

Figure 7 shows the beneficial interaction of THC with the terpenes alpha-bisabolol and guaiol in terms of anti-inflammatory activity. To arrive at the depicted values presented in Figure 7, for each data point of the concentration of THC, alpha-bisabolol or guaiol, the respective cytokine secretion measured in the PHA-stimulated group was corrected by the cytokine secretion of the non-PHA-stimulated control. The respective experimental data of cytokine secretion in non-PHA-stimulated control PBMCs and PHA-stimulated PBMCs are shown in Figures 5 and 6. Those corrected values of the individual compounds THC, alpha-bisabolol or guaiol were set into relation to the corrected values of the respective mixture of THC, alpha-bisabolol and guaiol.

As a matter of example, the calculation for the exemplary cytokine IL-2 shown in Figure 7 was accomplished by using the following equation: $\begin{matrix}Relative difference \\ = \frac{\begin{matrix}{\left[{c}_{IL - 2 , stimulated}-{c}_{IL - 2 , non - stimulated}\right]}_{THC} + {\left[{c}_{IL - 2 , stimulated}-{c}_{IL - 2 , non - stimulated}\right]}_{α - bisabolol} + \\ {\left[{c}_{IL - 2 , stimulated}-{c}_{IL - 2 , non - stimulated}\right]}_{guaiol}\end{matrix}}{{\left[{c}_{IL - 2 , stimulated}-{c}_{IL - 2 , non - stimulated}\right]}_{THC + α - bisabolol + guaiol}}\end{matrix}$

As all tested cytokines are indicatory for an inflammatory reaction of the PBMC's in response to PHA-induced stimulation, any decrease of cytokine secretion after administration of either THC, alpha-bisabolol or guaiol alone or of all three components in combination, when compared to the non-stimulated control, is indicative for an anti-inflammatory activity. By virtue of the above-described equation, a direct comparison can be drawn between the anti-inflammatory activity of the single substances THC, alpha-bisabolol and guaiol and the respective mixture thereof. Any value higher than 1 implies that the cytokine secretion of the mixture (in the denominator) is less than the sum of cytokine secretion of the respective individual substances. In such case the anti-inflammatory activity of the mixture therefore outperforms the combined anti-inflammatory activity of the individual compounds.

As evident from Figure 7, cytokine secretion is reduced when THC, alpha-bisabolol and guaiol are administered in combination compared to THC, alpha-bisabolol or guaiol alone. These results indicate that anti-inflammatory activity is enhanced when THC, alpha-bisabolol and guaiol are administered in a combinatory treatment in a THC/alpha-bisabolol ratio of between 100:1 and 450:1, specifically between 160:1 and 295:1, and/or a THC/guaiol ratio of between 50:1 and 250:1, specifically between 105:1 and 200:1. The *Cannabis* plant extract of the present invention comprises THC and the terpenes alpha-bisabolol and guaiol in the ratio as tested individually in the Examples (see Table 3). Thus, it is believed that a composition and pharmaceutical formulation of the invention which has the same individual effective components and THC/terpene ratio as the *Cannabis* extract disclosed in the present Examples (Table 3), are particularly effective in providing anti-inflammatory activity. Accordingly, the anti-inflammatory activity of alpha-bisabolol, guaiol, beta-caryophyllene and at least one terpene selected from the group consisting of linalool, alpha-humulene, nerolidol, caryophyllene oxide, alpha-pinene, camphene, beta-pinene, beta-myrcene, limonene, eucalyptol, ocimene, gamma-terpinene, terpinolene, alpha-terpinene, para-cymene, isopulegol and geraniol is plausible to be enhanced by the presence of THC.These results render plausible that the composition and the formulation of the invention have the superior effect of having an increased anti-inflammatory activity.

### Example 3 Stability of the composition

The following example demonstrates that stability of THC in a plant extract can be increased by addition of a carrier oil such as sesame oil.

### 3.1 Test substances

*Cannabis* plant *extract*/*Cannabis* soft extract as obtainable by a *Cannabis* plant deposited by Vertanical GmbH by the Community Plant Variety Office with the application number A202104053 or as obtainable by the process as described in patent application EP22154007.3 (PCT/EP2023/052073) has been used for the experiment (Table 1). The aforementioned *Cannabis* plant extract was optionally further processed by distillation and/or by further dilution. The distillate was produced from the *Cannabis* plant extract by short path distillation. Cannabinoids and terpenes present in the extract are evaporated and collected as a distillate. Heavier material like chlorophyll, sugars, salts and fats are unable to evaporate and remained as residue. Dilution of the *Cannabis* plant extract was performed by adjusting the extract to a THC content of either 10% or 5% by weight by adding the respective volume of either ethanol or sesame oil.

### 3.2 Results

**Table 7: Stability of Cannabis plant extract, and Cannabis plant extract which has been further processed as described in the Table, after 14 days storage at the indicated temperature.**

| **Test Product** | **0 weeks [wt. % THC]** | **2 weeks [wt. % THC]** | **Change** |
|---|---|---|---|
| *Cannabis* plant extract stored at 50°C | 72.75 | 61.65 | - 15% |
| *Cannabis* plant extract standardized to 10% THC by adding ethanol and stored at 50°C | 10.54 | 9.76 | - 7% |
| *Cannabis* plant extract standardized to 5% THC by adding ethanol and stored at 50°C | 5.10 | 4.82 | - 5% |
| Distillate 5% standardized to 5% THC by adding ethanol and stored at 50°C | 4.84 | 4.41 | - 9% |
| *Cannabis* plant extract standardized to 5% THC by adding sesame oil and stored at 70°C | 4.88 | 4.82 | - 1% |

As evident from Table 7, THC in *Cannabis* plant extract is relatively unstable at elevated temperatures of 50°C, showing a degradation of 15% of THC over 14 days of storage. *Cannabis* plant extract which has been diluted with ethanol to 10% or 5% THC is more stable at elevated temperatures compared to *Cannabis* plant extract not further processed, but still relatively unstable. *Cannabis* plant extract standardized to 5% THC by adding sesame oil remains stable over 2 weeks, even when stored at a very high temperature of 70°C.

As shown in Tables 8 and 9, further studies proof the long-term stability of *Cannabis* plant extract standardized to 5% THC by adding sesame oil.

**Table 8: Stability of Cannabis plant extract in sesame oil after 0, 4, 8 or 12 weeks of storage at 40°C.**

| **Test Product** | **0 weeks [wt. % THC]** | **4 weeks [wt. % THC]** | **8 weeks [wt. % THC]** | **12 weeks [wt. % THC]** |
|---|---|---|---|---|
| *Cannabis* plant extract standardized to 5% THC by adding sesame oil and stored at 40°C | 4.92 | 4.91 | 4.82 | 4.89 |

**Table 9: Stability of Cannabis plant extract in sesame oil after 2 years of storage at 25°C.**

| **Test Product** | **0 weeks [wt. % THC]** | **12 months [wt. % THC]** | **18 months [wt. % THC]** | **24 months [wt. % THC]** |
|---|---|---|---|---|
| *Cannabis* plant extract standardized to 5% THC by adding sesame oil and stored at 25°C | 5.03 | 5.00 | 4.99 | 5.08 |

These results show that the preferred composition of the invention comprising a *Cannabis* plant extract and sesame oil, e.g. preferably a *Cannabis* plant extract as defined in Table 3 diluted with sesame oil, has a superior technical effect of providing improved short- and long-term stability of cannabinoids such as THC.

## Claims

1. A pharmaceutical formulation comprising a *Cannabis* plant extract, wherein the formulation comprises delta-9-tetrahydrocannabinol (THC) in an amount of between about 1.0 and 2.5 percent and wherein THC and alpha-bisabolol are present in a ratio of between about 100:1 and 450:1 in the formulation, THC and guaiol are present in a ratio of between about 50:1 and 250:1 in the formulation, THC and beta-caryophyllene are present in a ratio of between about 50:1 and 250:1 in the formulation, THC and linalool are present in a ratio of between about 450:1 and 1400:1 in the formulation, THC and alpha-humulene are present in a ratio of between about 300:1 and 725:1 in the formulation, and THC and nerolidol are present in a ratio of between about 200:1 and 600:1 in the formulation.

2. The pharmaceutical formulation of claim 1 further comprising at least one terpene selected from the group consisting of caryophyllene oxide, alpha-pinene, camphene, beta-pinene, beta-myrcene, limonene, eucalyptol, ocimene, gamma-terpinene, terpinolene, alpha-terpinene, para-cymene, isopulegol and geraniol.

3. The pharmaceutical formulation of claim 1 or 2, wherein
(a) delta-9-tetrahydrocannabinol (THC) and linalool are present in a ratio of between about 500:1 and 1330:1 in the formulation;
(b) THC and alpha-humulene are present in a ratio of between about 355:1 and 665:1 in the formulation;
(c) THC and nerolidol are present in a ratio of between about 285:1 and 535:1 in the formulation,
(d) THC and caryophyllene oxide are present in a ratio of between about 1000:1 and 2150:1 in the formulation;
(e) THC and alpha-pinene are present in a ratio of between about 5850:1 and 16000:1 in the formulation;
(f) THC and camphene are present in a ratio of between about 7300:1 and 132950:1 in the formulation;
(g) THC and beta-pinene are present in a ratio of between about 6475:1 and 23100:1 in the formulation;
(h) THC and beta-myrcene are present in a ratio of between about 1950:1 and 6300:1 in the formulation;
(i) THC and limonene are present in a ratio of between about 1650:1 and 5050:1 in the formulation;
(j) THC and eucalyptol are present in a ratio of between about 5050:1 and 38650:1 in the formulation;
(k) THC and ocimene are present in a ratio of between about 2500:1 and 44700:1 in the formulation;
(l) THC and gamma-terpinene are present in a ratio of between about 4700:1 and 35750:1 in the formulation;
(m) THC and terpinolene are present in a ratio of between about 5450:1 and 129850:1 in the formulation,
(n) THC and alpha-terpinene are present in a ratio of between about 5150:1 and 58550:1 in the formulation;
(o) THC and para-cymene are present in a ratio of between about 4700:1 and 56700:1 in the formulation;
(p) THC isopulegol are present in a ratio of between about 4000:1 and 8000:1 in the formulation; and/or
(q) THC and geraniol are present in a ratio of between about 1300:1 and 18250:1 in the formulation.

4. The pharmaceutical formulation of any one of claims 1 to 3, wherein the formulation comprises alpha-bisabolol in an amount of between about 0.003 and 0.016 percent by weight of the formulation.

5. The pharmaceutical formulation of any one of claims 1 to 4, wherein the formulation comprises guaiol in an amount of between about 0.01 and 0.023 percent by weight of the formulation.

6. The pharmaceutical formulation of any one of claims 1 to 5, wherein the formulation comprises beta-caryophyllene in an amount of between about 0.01 and 0.024 percent by weight of the formulation.

7. The pharmaceutical formulation of any one of claims 1 to 6, wherein the formulation comprises
(a) linalool in an amount of between about 0.0007 and 0.004 percent by weight of the formulation;
(b) alpha-humulene in an amount of between about 0.001 and 0.007 percent by weight of the formulation; and/or
(c) nerolidol in an amount of between about 0.001 and 0.009 percent by weight of the formulation.

8. The pharmaceutical formulation of any one of claims 1 to 7, wherein the *Cannabis* plant extract is obtainable by solvent extraction of a *Cannabis* plant.

9. The pharmaceutical formulation of claim 8, wherein the *Cannabis* plant extract is obtainable by solvent extraction of flower material of the *Cannabis* plant.

10. The pharmaceutical formulation of claim 8 or claim 9, wherein the *Cannabis* plant extract is an alcoholic extract.

11. The pharmaceutical formulation of any one of claims 1 to 9, wherein the formulation is in liquid form.

12. The pharmaceutical formulation of any one of claims 1 to 10, wherein the formulation further comprises a carrier oil.

13. The pharmaceutical formulation of any one of claims 1 to 12 for use in medicine.

14. The pharmaceutical formulation of any one of claims 1 to 13 for use in the treatment and/or prevention of chronic cancer pain, somatic pain, visceral pain, central neuropathic pain, peripheral neuropathic pain or complex pain syndromes.

15. The pharmaceutical formulation of claim 13 or 14 for use of claim 13 or 14, wherein the formulation is administered via oral-, oromucosal-, intra-nasal-, topical-, rectal- or vaginal administration.

## Patentansprüche

1. Pharmazeutische Formulierung, die einen Cannabis-Pflanzen-Extrakt umfasst, wobei die Formulierung Delta-9-Tetrahydrocannabinol (THC) in einer Menge von zwischen etwa 1,0 und 2,5 % umfasst, und wobei THC und Alpha-Bisabolol in der Formulierung in einem Verhältnis von zwischen etwa 100:1 und 450:1 vorliegen, THC und Guaiol in der Formulierung in einem Verhältnis zwischen etwa 50:1 und 250:1 vorliegen, THC und Beta-Caryophyllen in der Formulierung in einem Verhältnis zwischen etwa 50:1 und 250:1 vorliegen, THC und Linalool in der Formulierung in einem Verhältnis zwischen etwa 450:1 und 1400:1 vorliegen, THC und Alpha-Humulen in der Formulierung in einem Verhältnis zwischen etwa 300:1 und 725:1 vorliegen und THC und Nerolidol in der Formulierung in einem Verhältnis zwischen etwa 200:1 und 600:1 vorliegen.

2. Pharmazeutische Formulierung nach Anspruch 1, die des Weiteren mindestens ein Terpen, ausgewählt aus der Gruppe bestehend aus Caryophyllenoxid, Alpha-Pinen, Camphen, Beta-Pinen, Beta-Myrcen, Limonen, Eukalyptol, Ocimen, Gamma-Terpinen, Terpinolen, Alpha-Terpinen, Para-Cymol, Isopulegol und Geraniol, umfasst.

3. Pharmazeutische Formulierung nach Anspruch 1 oder 2, wobei
(a) Delta-9-Tetrahydrocannabinol (THC) und Linalool in der Formulierung in einem Verhältnis zwischen etwa 500:1 und 1330:1 vorliegen;
(b) THC und Alpha-Humulen in der Formulierung in einem Verhältnis zwischen etwa 355:1 und 665:1 vorliegen;
(c) THC und Nerolidol in der Formulierung in einem Verhältnis zwischen etwa 285:1 und 535:1 vorliegen;
(d) THC und Caryophyllenoxid in der Formulierung in einem Verhältnis zwischen etwa 1000:1 und 2150:1 vorliegen;
(e) THC und Alpha-Pinen in der Formulierung in einem Verhältnis zwischen etwa 5850:1 und 16000:1 vorliegen;
(f) THC und Camphen in der Formulierung in einem Verhältnis zwischen etwa 7300:1 und 132950:1 vorliegen;
(g) THC und Beta-Pinen in der Formulierung in einem Verhältnis zwischen etwa 6475:1 und 23100:1 vorliegen;
(h) THC und Beta-Myrcen in der Formulierung in einem Verhältnis zwischen etwa 1950:1 und 6300:1 vorliegen;
(i) THC und Limonen in der Formulierung in einem Verhältnis zwischen etwa 1650:1 und 5050:1 vorliegen;
(j) THC und Eukalyptol in der Formulierung in einem Verhältnis zwischen etwa 5050:1 und 38650:1 vorliegen;
(k) THC und Ocimen in der Formulierung in einem Verhältnis zwischen etwa 2500:1 und 44700:1 vorliegen;
(l) THC und Gamma-Terpinen in der Formulierung in einem Verhältnis zwischen etwa 4700:1 und 35750:1 vorliegen;
(m) THC und Terpinolen in der Formulierung in einem Verhältnis zwischen etwa 5450:1 und 129850:1 vorliegen;
(n) THC und Alpha-Terpinen in der Formulierung in einem Verhältnis zwischen etwa 5150:1 und 58550:1 vorliegen;
(o) THC und Para-Cymol in der Formulierung in einem Verhältnis zwischen etwa 4700:1 und 56700:1 vorliegen;
(p) THC und Isopulegol in der Formulierung in einem Verhältnis zwischen etwa 4000:1 und 8000:1 vorliegen; und/oder
(q) THC und Geraniol in der Formulierung in einem Verhältnis zwischen etwa 1300:1 und 18250:1 vorliegen.

4. Pharmazeutische Formulierung nach einem der Ansprüche 1 bis 3, wobei die Formulierung Alpha-Bisabolol in einer Menge von zwischen etwa 0,003 und 0,016 Gew.-% der Formulierung umfasst.

5. Pharmazeutische Formulierung nach einem der Ansprüche 1 bis 4, wobei die Formulierung Guaiol in einer Menge von zwischen etwa 0,01 und 0,023 Gew.-% der Formulierung umfasst.

6. Pharmazeutische Formulierung nach einem der Ansprüche 1 bis 5, wobei die Formulierung Beta-Caryophyllen in einer Menge von zwischen etwa 0,01 und 0,024 Gew.-% der Formulierung umfasst.

7. Pharmazeutische Formulierung nach einem der Ansprüche 1 bis 6, wobei die Formulierung
(a) Linalool in einer Menge von zwischen etwa 0,0007 und 0,004 Gew.-% der Formulierung umfasst;
(b) Alpha-Humulen in einer Menge von zwischen etwa 0,001 und 0,007 Gew.-% der Formulierung umfasst; und/oder
(c) Nerolidol in einer Menge von zwischen etwa 0,001 und 0,009 Gew.-% der Formulierung umfasst.

8. Pharmazeutische Formulierung nach einem der Ansprüche 1 bis 7, wobei der Cannabis-Pflanzen-Extrakt durch Lösungsmittelextraktion einer *Cannabis*-Pflanze erhältlich ist.

9. Pharmazeutische Formulierung nach Anspruch 8, wobei der Cannabis-Pflanzen-Extrakt durch Lösungsmittelextraktion von Blütenmaterial der *Cannabis-*Pflanze erhältlich ist.

10. Pharmazeutische Formulierung nach Anspruch 8 oder Anspruch 9, wobei der Cannabis-Pflanzen-Extrakt ein alkoholischer Extrakt ist.

11. Pharmazeutische Formulierung nach einem der Ansprüche 1 bis 9, wobei die Formulierung in flüssiger Form ist.

12. Pharmazeutische Formulierung nach einem der Ansprüche 1 bis 10, wobei die Formulierung des Weiteren ein Trägeröl umfasst.

13. Pharmazeutische Formulierung nach einem der Ansprüche 1 bis 12 zur Verwendung für medizinische Zwecke.

14. Pharmazeutische Formulierung nach einem der Ansprüche 1 bis 13 zur Verwendung bei der Behandlung und/oder Vorbeugung von chronischen Krebsschmerzen, somatischen Schmerzen, viszeralen Schmerzen, zentralen neuropathischen Schmerzen, peripheren neuropathischen Schmerzen oder komplexen Schmerzsyndromen.

15. Pharmazeutische Formulierung nach Anspruch 13 oder 14 zur Verwendung nach Anspruch 13 oder 14, wobei die Formulierung oral, oromukosal, intranasal, topisch, rektal oder vaginal verabreicht wird.

## Revendications

1. Formulation pharmaceutique comprenant un extrait de plant de *Cannabis,* laquelle formulation comprend du delta-9-tétrahydrocannabinol (THC) en une quantité comprise entre environ 1,0 et 2,5 %, dans laquelle le THC et l'alpha-bisabolol sont présents en un rapport compris entre environ 100/1 et 450/1 dans la formulation, le THC et le guaiol sont présents en un rapport compris entre environ 50/1 et 250/1 dans la formulation, le THC et le bêta-caryophyllène sont présents en un rapport compris entre environ 50/1 et 250/1 dans la formulation, le THC et le linalool sont présents en un rapport compris entre environ 450/1 et 1400/1 dans la formulation, le THC et l'alpha-humulène sont présents en un rapport compris entre environ 300/1 et 725/1 dans la formulation, et le THC et le nérolidol sont présents en un rapport compris entre environ 200/1 et 600/1 dans la formulation.

2. Formulation pharmaceutique selon la revendication 1, comprenant en outre au moins un terpène choisi dans le groupe constitué par l'oxyde de caryophyllène, l'alpha-pinène, le camphène, le bêta-pinène, le bêta-myrcène, le limonène, l'eucalyptol, l'ocimène, le gamma-terpinène, le terpinolène, l'alpha-terpinène, le para-cymène, l'isopulégol et le géraniol.

3. Composition pharmaceutique selon la revendication 1 ou 2, dans laquelle
(a) le delta-9-tétrahydrocannabinol (THC) et le linalool sont présents en un rapport compris entre environ 500/1 et 1330/1 dans la formulation ;
(b) le THC et l'alpha-humulène sont présents en un rapport compris entre environ 355/1 et 665/1 dans la formulation ;
(c) le THC et le nérolidol sont présents en un rapport compris entre environ 285/1 et 535/1 dans la formulation ;
(d) le THC et l'oxyde de caryophyllène sont présents en un rapport compris entre environ 1000/1 et 2150/1 dans la formulation ;
(e) le THC et l'alpha-pinène sont présents en un rapport compris entre environ 5850/1 et 16000/1 dans la formulation ;
(f) le THC et le camphène sont présents en un rapport compris entre environ 7300/1 et 132950/1 dans la formulation ;
(g) le THC et le bêta-pinène sont présents en un rapport compris entre environ 6475/1 et 23100/1 dans la formulation ;
(h) le THC et le bêta-myrcène sont présents en un rapport compris entre environ 1950/1 et 6300/1 dans la formulation ;
(i) le THC et le limonène sont présents en un rapport compris entre environ 1650/1 et 5050/1 dans la formulation ;
(j) le THC et l'eucalyptol sont présents en un rapport compris entre environ 5050/1 et 38650/1 dans la formulation ;
(k) le THC et l'ocimène sont présents en un rapport compris entre environ 2500/1 et 44700/1 dans la formulation ;
(l) le THC et le gamma-terpinène sont présents en un rapport compris entre environ 4700/1 et 35750/1 dans la formulation ;
(m) le THC et le terpinolène sont présents en un rapport compris entre environ 5450/1 et 129850/1 dans la formulation ;
(n) le THC et l'alpha-terpinène sont présents en un rapport compris entre environ 5150/1 et 58550/1 dans la formulation ;
(o) le THC et le para-cymène sont présents en un rapport compris entre environ 4700/1 et 56700/1 dans la formulation ;
(p) le THC et l'isopulégol sont présents en un rapport compris entre environ 4000/1 et 8000/1 dans la formulation ; et/ou
(q) le THC et le géraniol sont présents en un rapport compris entre environ 1300/1 et 18250/1 dans la formulation.

4. Formulation pharmaceutique selon l'une quelconque des revendications 1 à 3, laquelle formulation comprend de l'alpha-bisabolol en une quantité comprise entre environ 0,003 et 0,016 % en poids de la formulation.

5. Formulation pharmaceutique selon l'une quelconque des revendications 1 à 4, laquelle formulation comprend du guaiol en une quantité comprise entre environ 0,01 et 0,023 % en poids de la formulation.

6. Formulation pharmaceutique selon l'une quelconque des revendications 1 à 5, laquelle formulation comprend du bêta-caryophyllène en une quantité comprise entre environ 0,01 et 0,024 % en poids de la formulation.

7. Formulation pharmaceutique selon l'une quelconque des revendications 1 à 6, laquelle formulation comprend
(a) du linalool en une quantité comprise entre environ 0,0007 et 0,004 % en poids de la formulation ;
(b) de l'alpha-humulène en une quantité comprise entre environ 0,001 et 0,007 % en poids de la formulation ; et/ou
(c) du nérolidol en une quantité comprise entre environ 0,001 et 0,009 % en poids de la formulation.

8. Formulation pharmaceutique selon l'une quelconque des revendications 1 à 7, dans laquelle l'extrait de plant de *Cannabis* peut être obtenu par extraction au solvant d'un plant de *Cannabis.*

9. Formulation pharmaceutique selon la revendication 8, dans laquelle l'extrait de plant de *Cannabis* peut être obtenu par extraction au solvant du matériau floral du plant de *Cannabis.*

10. Formulation pharmaceutique selon la revendication 8 ou la revendication 9, dans laquelle l'extrait de plant de *Cannabis* est un extrait alcoolique.

11. Formulation pharmaceutique selon l'une quelconque des revendications 1 à 9, dans laquelle la formulation est sous forme liquide.

12. Formulation pharmaceutique selon l'une quelconque des revendications 1 à 10, dans laquelle la formulation comprend en outre un véhicule huileux.

13. Formulation pharmaceutique selon l'une quelconque des revendications 1 à 12 pour une utilisation en médecine.

14. Formulation pharmaceutique selon l'une quelconque des revendications 1 à 13 pour une utilisation dans le traitement et/ou la prévention d'une douleur de cancer, d'une douleur somatique, d'une douleur viscérale, d'une douleur neuropathique centrale, d'une douleur neuropathique périphérique ou de syndromes douloureux complexes, chroniques.

15. Formulation pharmaceutique selon la revendication 13 ou 14 pour une utilisation selon la revendication 13 ou 14, dans laquelle la formulation est administrée par voie orale, oromuqueuse, intranasale, topique, rectale ou vaginale.
